Europäisches Patentamt
European Patent Office
Office européen des brevets

(19)

(11) Publication number: **0 368 410 A2**

(12) **EUROPEAN PATENT APPLICATION**

(21) Application number: **89202803.6**

(22) Date of filing: **06.11.89**

(51) Int. Cl.5: **C07D 265/36, C07D 279/16, C07D 498/06, C07D 513/06, A61K 31/535, A61K 31/54, //(C07D498/06,265:00,221:00), (C07D513/06,279:00,221:00)**

(30) Priority: **07.11.88 EP 88202484**
**14.02.89 EP 89200368**

(43) Date of publication of application:
**16.05.90 Bulletin 90/20**

(84) Designated Contracting States:
**AT BE CH DE ES FR GB GR IT LI LU NL SE**

(71) Applicant: **GIST-BROCADES N.V.**
**Wateringseweg 1 P.O. Box 1**
**NL-2600 MA Delft(NL)**

(72) Inventor: **Van Zoest, Willem Johan**
**Koekoekslaan 11**
**NL-3121 XJ Schiedam(NL)**

Inventor: **Marx, Arthur Friedrich**
**Fl. Nightingalelaan 12**
**NL-2614 GM Delft(NL)**
Inventor: **Koger, Hein Simon**
**G. Vermootenstraat 32**
**NL-2064 XR Spaarndam(NL)**
Inventor: **Booy, Johannes**
**Bos en Duinplein 10**
**NL-2061 VS Bloemendaal(NL)**

(74) Representative: **Huygens, Arthur Victor, Dr. et al**
**c/o Gist-Brocades N.V. Patent & Trademarks Department Wateringseweg 1 PO Box 1**
**NL-2600 MA Delft(NL)**

(54) **Optically active benzoxazines and bezothiazines and a process for their stereospecific preparation.**

(57) Optically active 7,8-difluoro-3,4-dihydro-3-methyl-2H-[1,4]benzoxazines and 7,8-difluoro-3,4-dihydro-2-methyl-2H-[1,4]benzoxazines and the corresponding benzothiazines of formula III,

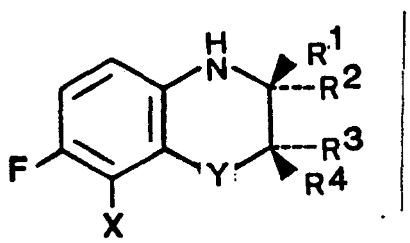

III

where, one of the substituents $R^1$, $R^2$, $R^3$ and $R^4$ is $CH_2OH$ or $-CH_2Z$, the remaining being hydrogen, X denotes fluoro, chloro, methyl or hydrogen, Y is oxygen or sulfur and Z is hydrogen, fluoro or protected hydroxyl are obtained as the stereochemically pure products of a stereospecific synthesis using a 3,4-difluoronitrobenzene substituted with a stereochemically pure 2-(1-hydroxyisopropoxy), 2-(2-hydroxypropoxy), 2-(1-hydroxyisopropylthio) or 2-(2-hydroxypropylthio) substituent.

The obtained compounds are suited for the production of optically active pyridobenzoxazines and pyridobenzothiazines, among which are useful antibacterial optically active quinolones, particularly (S)-(-)-ofloxacin.

EP 0 368 410 A2

# OPTICALLY ACTIVE BENZOXAZINES AND BENZOTHIAZINES AND A PROCESS FOR THEIR STEREOSPECIFIC PREPARATION

The present invention relates to optically active benzoxazines and optically active benzothiazines, to a process for their preparation, to their use for the preparation of optically active pyridobenzoxazines and pyridobenzothiazines and to pharmaceutical compositions containing these pyridobenzoxazines and pyridobenzothiazines.

The quinolones are a group of recently developed potent antibacterial agents against both gram-negative and gram-positive organisms. Hardly any of the quinolones synthesized sofar has an asymmetric centre. Of a specific group of quinolones, characterized by a pyridobenzoxazine ring system, some compounds have a sole methyl group at position 3 in the oxazine ring. These compounds therefore have an asymmetric center and occur as a couple of (R)- and (S)-stereoisomers. Ofloxacin, being an example of this group, has been used until recently only as a racemic mixture of both stereoisomers.

It is known that many biologically active compounds exist as a mixture of stereoisomers. Up to now these mixtures are often used as such in pharmaceutical applications. Usually the desired biological activity resides mainly in one stereoisomer so that the potency of the mixture may be reduced to as low as half. However, a major reason for the continued use of mixtures of stereoisomers is that the advantage of a possible increase in activity does not outweigh the costs of separation of the stereoisomers.

For example, it has recently been disclosed that the (S)-(-)-isomer of 6,7-dihydro-5,8-dimethyl-9-fluoro-1-oxo-1H,5H-benzo[ij]quinolizine-2-carboxylic acid has potent antibacterial activity in vitro while the (R)-(+)-isomer was much less active (J.F. Gerster c.s, Abstract of Papers, 25th Intersc. Conf. Antimicrob. Agents and Chemotherapy, Minneapolis, U.S.A., 1985, 114). (S)-(-)-ofloxacin is reported to be 8 to 128 times more active than (R)-(+)-ofloxacin (1. Hayakawa c.s., Antimicrob. Agents Chemother. 1986, 29, 163).

Racemic mixtures can be resolved by known methods e.g. by fractional crystallisation of appropriate salts or by enzymatic hydrolysis of appropriate esters or amides. To resolve racemic ofloxacin the following methods have been applied:

1. Resolution by high performance liquid chromatography of an intermediate, e.g. the 3,5-dinitrobenzoyl ester of racemic ethyl 9,10-difluoro-2,3-dihydro-3-hydroxymethyl-7-oxo-7H-pyrido[1,2,3-de][1,4]-benzoxazine-6-carboxylate (I. Hayakawa c.s., Antimicrob. Agents Chemother. 1986, 29, 163) and conversion of the resulting (S)-isomer into (S)-ofloxacin by known methods.

This method, however, was inadequate for the separation of larger amounts of the optically active isomers. More suitable methods are the following ones:

2. Enzymatic resolution of the intermediate racemic 3-acetoxymethyl-7,8-difluoro-3,4-dihydro-2H-[1,4]benzoxazine (K. Sakano c.s., Agric. Biol. Chem. 1987, 51, 1265) and conversion of the resulting (S)-isomer into (S)-ofloxacin by known methods.

3. Preparation of the N-p-toluenesulfonyl-(L)-prolinylamide of the intermediate racemic 7,8-difluoro-3,4-dihydro-3-methyl-2H-[1,4]benzoxazine, separating the (R)-(+)- and (S)-(-)-isomers by crystallisation and column chromatography (S. Atarashi c.s., Chem. Pharm. Bull. 1987, 55, 1896) and conversion of the resulting (S)-isomer into 5-ofloxacin by known methods.

4. In DE-A-3639465 a method is described for resolving racemic chinolon structures, including ofloxacin comprising introduction of a hydrazinium group, converting the molecule into a betaine form and preparation of a mixture of diastereomeric salts with an optically active organic acid. Both salts are separated by crystallisation, subsequently freed of the acid and finally reduced to the original compound but in optically pure form.

5. EP-A-0304684 discloses a further method for the optical resolution of racemic benzoxazines with an asymmetric carbon atom at position 3. To that end an optically active salt is made with (R)-(-)-camphor-10-sulphonic acid. The undesired isomer is racemized and introduced into a new resolution cycle.

The disadvantage of said methods is that not more than 50% of the starting material is initially recovered as the desired product. Furthermore additional steps are needed to make and to splice these optically active derivatives which are amenable for the usual separation methods.

According to EP-A-0273399 benzoxazines are prepared by a stereoselective reduction of the corresponding 3,4-dehydrobenzoxazines. A rather expensive reducing agent is needed. A further disadvantage is that extra steps are needed for introducing and removing a protecting group.

In US 4777253 a method is described for the preparation of optically active ofloxacin using one enantiomer of a 4-oxoquinoline-3-carboxylate having an asymmetrical N-1-(hydroxymethyl)ethyl substituent. After ring closure by heating with potassium hydroxide a pyridobenzoxazine results having a chiral methyl group on C2.

According to the present invention an advantageous chirality preserving process is found for the preparation at reduced costs of an important group of optically active 7-fluoro-8-X-3,4-dihydro-2H-[1,4]-benzoxazines and -benzothiazines according to formula III,

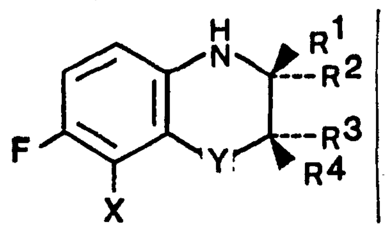

III

where, one of the substituents $R^1$, $R^2$, $R^3$ and $R^4$ is $CH_2OH$ or $-CH_2Z$, the remaining being hydrogen, where Z is hydrogen, fluoro or protected hydroxy, Y is oxygen or sulfur and X denotes fluoro, chloro, methyl or hydrogen. Hydroxy protecting groups suited for the reactions of this invention are e.g. trityl, benzyl, methoxymethyl, 1-ethoxyethyl and tetrahydropyranyl.

The invented process is characterised by the use of certain hydroxypropoxy or hydroxyisopropoxy substituted fluoronitrobenzenes as starting compounds, particularly the 4-fluoro-3-$X'$-2-(hydroxy(3C)alkoxy)-nitrobenzenes of the formulas I and II

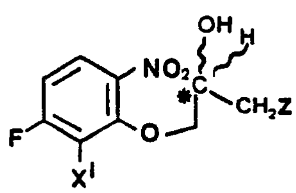

I

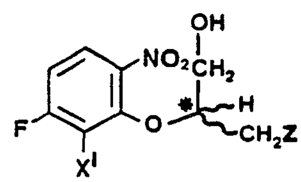

II

where $X'$ denotes fluoro or chloro and Z is as defined before.

With hydroxy(3C)alkoxy is meant the 2-hydroxypropoxy or the 1-hydroxyisopropoxy group. It should be noted that the asterisk in formulas I and II points to an asymmetric centre. Therefore both compounds exist as enantiomeric (R)-and (S)-isomers.

To obtain the benzothiazines analogous to formula III one departs from the corresponding thia-analogues of formulas I and II which contain a 2-hydroxypropylthio or a 1-hydroxyisopropylthio substituent.

According to the invented multi-step process first the nitro group of the compounds I and II is reduced into an amino group by conventional methods, followed, but preferably preceded by the activation of the free hydroxyl group e.g. by replacing it by a leaving group such as methylsulfonyloxy, p-toluenesulfonyloxy, bromo or a group with the formula $(phenyl)_3P^+$-O-, which are apt for ring closure. The reaction may be carried e.g. with methylsulfonylchloride at -10° C to room temperature, which takes only several minutes.

Suitable amino reducing agents are hydrogen in the presence of Raney nickel, stannous chloride in ethanol or tin metal with hydrogen chloride. Provided no sulfide group containing compounds are present, also hydrogenation with a palladium or platina catalyst could be applied. Ethanol and toluene are suitable solvents. Hydrogen gas may be substituted by ammonium formate. The reduction is carried out at room temperature and is finalized within 15 min. - 5 hrs. The resulting aniline having an activated hydroxyl group is subsequently treated with a base, e.g. potassium tertbutoxide or the hydroxide of an alkali metal, but preferably potassium hydroxide and more preferably potassium hydroxide with potassium carbonate in the presence of a phase transfer catalyst such as $^+N(C_4H_9)_4$ $^{-H}SO_4$, which results into ring closure, giving the desired benzoxazine according to formula III,

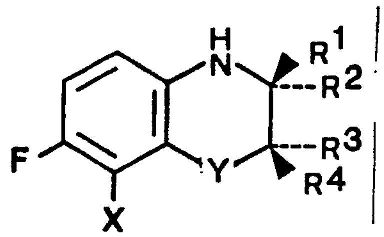

III

where X, Y and $R^1$-$R^4$ are as defined before. The use of potassium hydroxide as a base, preferably in combination with potassium carbonate, is particularly advantageous because in high yield the benzoxazine is obtained containing a relatively small amount of by-products. Suitable solvents for the ring closure reaction are found in the group of apolar organic solvents, such as methylene chloride. Preferably toluene is used. The reaction takes 0.5 - 5 hrs when carried out at -10° C to room temperature.

The above-mentioned hydroxyl activation of the hydroxy(3C)alkoxyaniline and the subsequent ring closure may be carried out as a one-pot-process, preferably using toluene as the solvent.

The corresponding benzothiazines are obtained using the same reaction sequence but starting with the corresponding thiaanalogues as said before.

Compounds III with X = hydrogen or methyl are novel substances and they may be prepared from the corresponding compounds with X = fluor by methods known in the art, for example by reduction with sodium hydride in dimethylformamide at a temperature which ranges from room temperature to reflux temperature resp. by methylation under the same reaction conditions with a metal organic compound such as lithium dimethyl cuprate in a suitable solvent, e.g. diethyl ether or tetrahydrofuran.

Starting compounds I with a propoxy substituent yield a 3-$CH_2Z$-benzoxazine, whereas compounds II with an isopropoxy substituent yield a 2-$CH_2Z$-benzoxazine. The 3-$CH_2Z$-benzoxazines and the corresponding 2-$CH_2Z$-benzoxazines are said to be regioisomers of each other. Both may be formed in the same reaction from a mixture of I and II obtained in a previous step.

The compounds I and II both contain an asymmetric centre indicated with an asterisk. The production of the benzoxazines III can be carried out with a racemic mixture, but preferably stereochemically pure starting compounds I or II are used. In that case, because the reaction proceeds without substantial or even without any racemisation, a stereochemically pure or substantially pure benzoxazine of formula III is obtained.

Therefore the reaction according to this invention offers a method to synthesize $CH_2OH$- or $CH_2Z$-substituted benzoxazines III (and the corresponding benzothiazines) with the desired stereochemical configuration.

For the preparation of the above mentioned starting compounds I and II, if desired in an optically active form, the invention provides several more or less chirality preserving reactions, departing from commercially available and, if desired, optically active compounds. Table I provides a survey of the reactants of methods a - j. For compounds I and II exemplified hereafter Z is hydrogen, but for compounds with Z is fluoro or protected hydroxy the method is valid as well:

a. 2,4-difluoro-3-halonitrobenzene is reacted with (R)-1,2-propanediol, under basic conditions to give (R)-4-fluoro-3-halo-2-(2-hydroxypropoxy)nitrobenzene (I) together with its regioisomer (R)-4-fluoro-3-halo-2-(1-hydroxyisopropoxy)nitrobenzene (II), where halo is fluoro or chloro and in a molar ratio of about 60/40 to about 70/30.

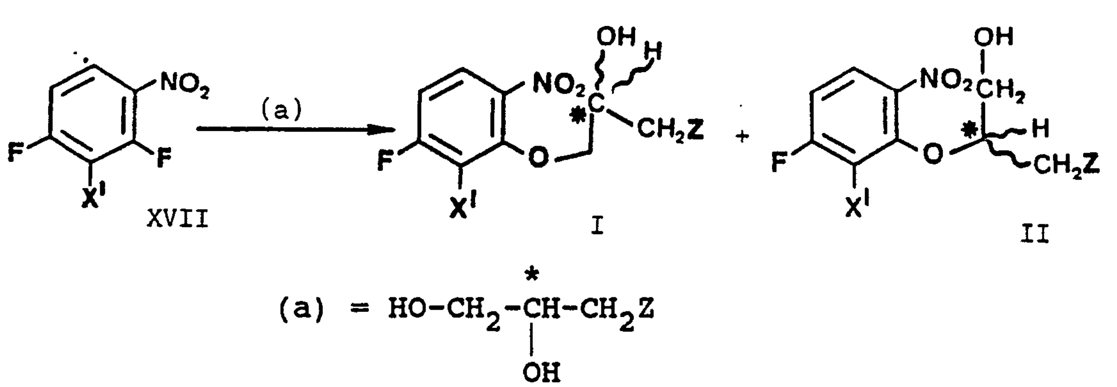


XVII I II

(a) = HO-CH₂-CH-CH₂Z

Starting with the corresponding (S)-1,2-propanediol both corresponding (S)-products are formed, in the same molar ratio. When using (R)- or (S)-2-hydroxypropanethiol the respective (R)- and (S)-4-fluoro-3-halo-2-(2-hydroxypropylthio)nitrobenzenes are formed but without their regioisomer.
Suitable basic conditions are created with a (1-4C)alkoxide of lithium, sodium or potassium, a (1-4C)alkyl lithium compound such as butyl lithium, an alkali hydride such as sodium hydride or tertiary amine such as triethylamine. Suitable solvents are tetrahydrofuran or toluene. The reaction is carried out preferably between room temperature and reflux temperature.
It should be remarked that during this preparation the chirality of the compounds is preserved, so that when in this case a pure optically active starting compound is used, a stereochemically pure product is obtained.

b. 3-Fluoro-2-halo-6-nitrophenol is reacted in the presence of a base with either
(R)- or (S)-2-methyloxirane,
where halo is fluoro or chloro to give
(R)- or (S)-4-fluoro-3-halo-2-(2-hydroxypropoxy)nitrobenzene (I), together with their regioisomer (R)- or (S)-4-fluoro-3-halo-2-(1-hydroxyisopropoxy)nitrobenzene (II) in a molar ratio of about 60/40 to about 70/30.
As indicated by the chirality marks (R) resp. (S) the reaction has proceeded by retention of the chirality.

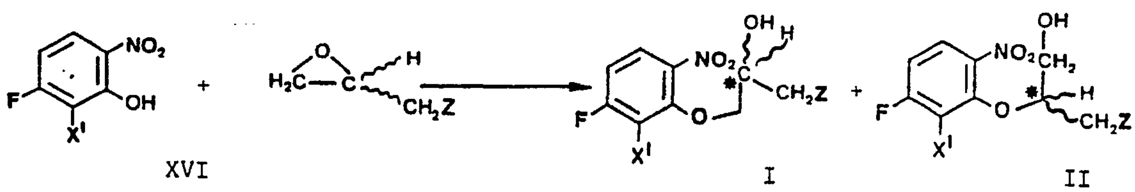

XVI I II

(b)

When using 3-fluoro-2-halo-6-nitrothiophenol with said (R)-or (S)-oxiranes the (R)- or (S)-4-fluoro-3-halo-2-(2-hydroxypropylthio)nitrobenzenes are formed but without their regioisomer. Several bases, such as ethyldiisopropylamine, are suited, but preferably an inorganic base is used, because lesser racemisation occurs. Suitable inorganic bases are the hydroxides of lithium, sodium and potassium. The reaction should be carried out in a sufficient polar sovent e.g. in ethanol, but also tetrahydrofuran is a suitable solvent. Preferably the reaction temperature is between room temperature and 150° C, more preferably at the boiling point of the solvent. The sulfur analogues react already at room temperature.

c. 3-Fluoro-2-halo-6-nitrophenol is reacted in the presence of an inorganic or organic base with either (R)- or (S)-1-L-2-propanol where L signifies an easily leaving atom or group such as bromo, iodo or tosylate and halo is as defined before, to give
(R)- or (S)-4-fluoro-3-halo-2-(2-hydroxypropoxy)nitrobenzene (I) together with its regioisomer
(R)- or (S)-4-fluoro-3-halo-2-(1-hydroxyisopropoxy)nitrobenzene (II) in a molar ratio of about 60/40 to about 70/30.
As indicated by the chirality marks (R) resp. (S) the reaction has proceeded by retention of the chirality.

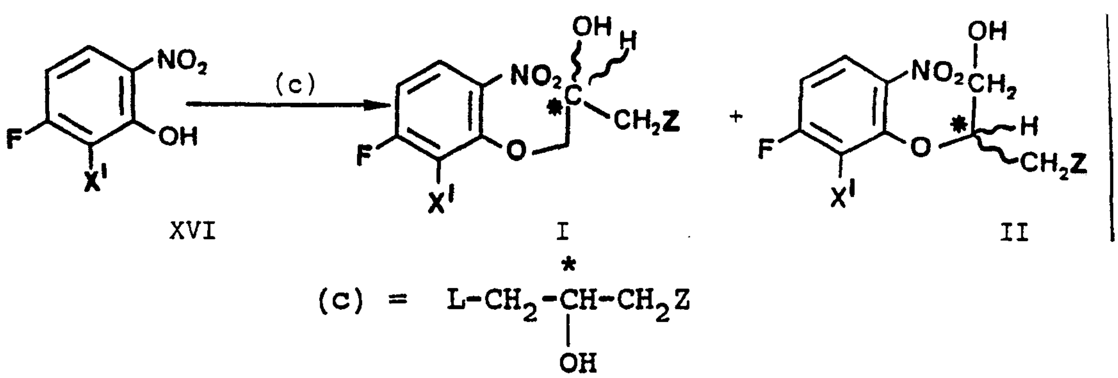


XVI I II

When using 3-fluoro-2-halo-6-nitrothiophenol with either the (R)- or (S)-1-L-2-propanol the (R)- or (S)-4-fluoro-3-halo-2-(2-hydroxypropylthio)nitrobenzene is formed but without its regioisomer. A suitable anorganic base is e.g. potassium carbonate, but also other bases of potassium, sodium or lithium, such as the hydroxides may be used. The reaction is carried out in a polar solvent e.g. in dimethylformamide. Good results are obtained also with organic bases such as ethyldiisopropylamine or triethylamine

in combination with solvents such as tetrahydrofuran or dioxan. Reaction temperatures are between room temperature and reflux temperature, preferably below 100 °C.

d. A mixture of 3-fluoro-2-halo-6-nitrophenol and (R)- or (S)-1,2-propanediol is reacted at room temperature, in the presence of an activator such as triphenylphosphine with a dialkyl azodicarboxylate e.g. diethyl azodicarboxylate (so-called Mitsunobu reaction) to give (R)- or
(S)-4-fluoro-3-halo-2-(2-hydroxypropoxy)nitrobenzene (I) together with its regioisomer respectively
(S)- or (R)-4-fluoro-3-halo-2-(1-hydroxyisopropoxy)nitrobenzene (II).

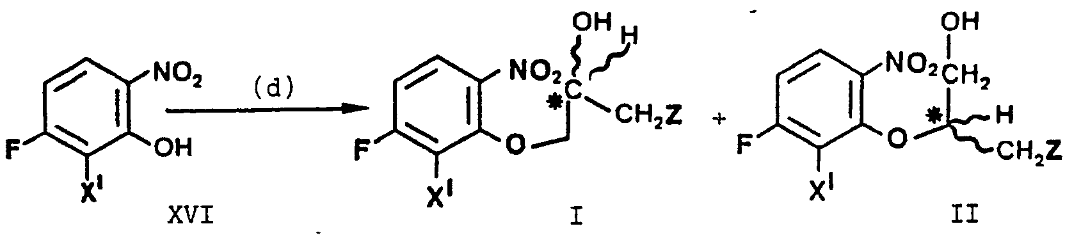


XVI I II

$$(d) = HO-CH_2-\overset{*}{C}H(OH)-CH_2Z$$

When using 3-fluoro-2-halo-6-nitrothiophenol with either the (R)- or (S)-1,2-propanediol the respective (R)- or (S)-4-fluoro-3-halo-2-(2-hydroxypropylthio)nitrobenzene is formed together with their regioisomer respectively
(S)- or (R)-4-fluoro-3-halo-2-(1-hydroxyisopropylthio)nitrobenzene.
Several sufficiently polar solvents may be used, but tetrahydrofuran is a preferred one.
It should be remarked that during this preparation the chirality of the compounds is preserved, so that when in this case a pure optically active starting compound is used, a stereochemically pure product is obtained.
The described reactions result in a mixture of regioisomers, each in a stereochemically pure form, with the proviso that the grade of optical purity may vary with the nature of reactant 2 as specified in table I. The separation of the intermediate regioisomers may be postponed, however. The mixture is then used in the subsequent benzoxazine synthesis, giving rise to a mixture of a 3-methylbenzoxazine and the corresponding 2-methylbenzoxazine which could be easily separated by usual physical methods such as column chromatography.
The following preparations (e. - j.) of 4-fluoro-3-halo-2-(hydroxy(3C)alkoxy)nitrobenzenes of formula I or II are preferred, however, because no regioisomer is formed at all, which contributes considerably to the economy of the process.

e. A mixture of 3-fluoro-2-halo-6-nitrophenol and either (R)- or (S)-2-OR″-propanol, where R″ is a hydroxyl protecting group as exemplified before, is reacted at room temperature, in the presence of an activator such as triphenylphosphine with a dialkyl azodicarboxylate, e.g. diethyl azodicarboxylate (Mitsunobu reaction), to give after removing the protecting group under mild acidic conditions by using e.g. an acidic ion-exchange resin, such as DOWEX 50W-X8-400 $H^+$ (RTM), resp. (R)- or
(S)-4-fluoro-3-halo-2-(2-hydroxypropoxy)nitrobenzene (I) without its regioisomer. In a more acidic environment the regioisomer does occur.

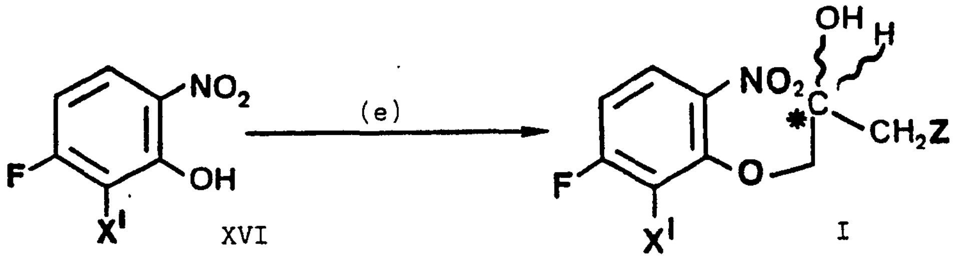


XVI I

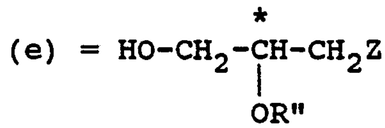

When using 3-fluoro-2-halo-6-nitrothiophenol with either (R)- or (S)-2-OR″-propanol, respectively (R)- or (S)-4-fluoro-3-halo-2-(2-hydroxypropylthio)nitrobenzene is formed, also without any regioisomer. Several sufficiently polar solvents may be used, but tetrahydrofuran is a preferred one.

It should be remarked that during this preparation the chirality of the compounds is preserved, so that when in this case a pure optically active starting compound is used, a stereochemically pure product is obtained.

f. To obtain the regioisomers of the products according to e. a mixture of 3-fluoro-2-halo-6-nitrophenol and either (R)- or (S)-1-(OR″)-2-propanol, where R″ is a hydroxyl protecting group as exemplified before, is reacted at room temperature, in the presence of an activator, such as triphenylphosphine with a dialkyl azodicarboxylate, e.g. diethyl azodicarboxylate, to give after removing the protecting group under appropriate, known conditions resp. (S)- or (R)-4-fluoro-3-halo-2-(1-hydroxyisopropoxy)nitrobenzene (I) without its regioisomer.

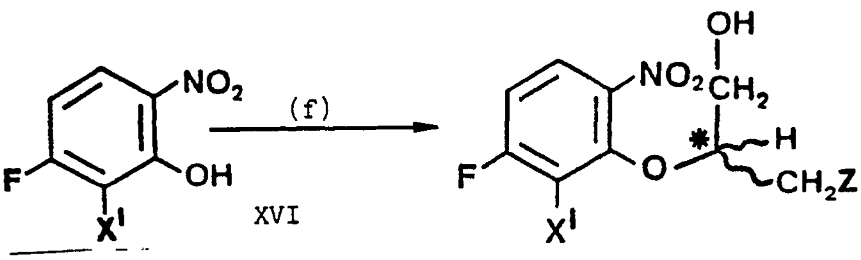


XVI

II

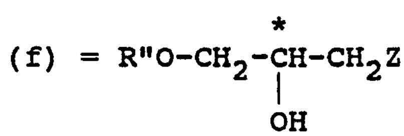

When using 3-fluoro-2-halo-6-nitrothiophenol with either (R)- or (S)-1-OR″-2-propanol, respectively (S)- or (R)-4-fluoro-3-halo-2-(1-hydroxyisopropylthio)nitrobenzene is formed, also without any regioisomer.

Several polar solvents may be used, but tetrahydrofuran is a preferred one.

It should be remarked that during this preparation the chirality of the compounds is preserved, so that when in this case a pure optically active starting compound is used, a stereochemically pure product is obtained.

g. 3-Fluoro-2-halo-6-nitrophenol is reacted in the presence of an anorganic or organic base with either (R)- or (S)-1-L-2-OR″-propane, where L and halo are as defined under c. and R″, as exemplified before, is a hydroxyl protecting group which should be stable in the environment of the phenol, to give after removing the protecting group under appropriate known conditions (R)- or (S)-4-fluoro-3-halo-2-(2-hydroxypropoxy)-nitrobenzene (I) without its regioisomer.

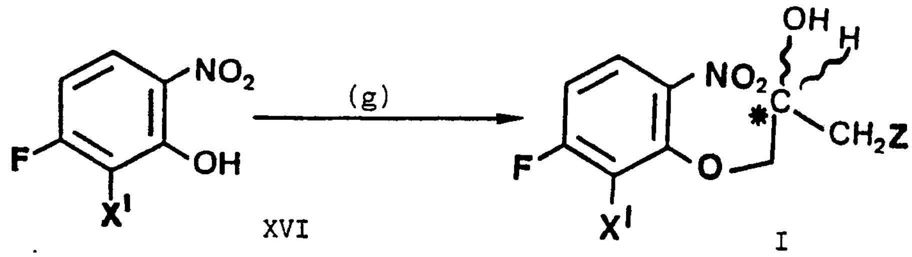


XVI I

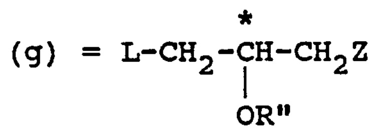

When using 3-fluoro-2-halo-6-nitrothiophenol with either (R)- or (S)-1-L-2-OR″-propane, respectively (R)- or (S)-4-fluoro-3-halo-2-(2-hydroxypropylthio)nitrobenzene is formed, also without any regioisomer.
A preferred base is ethyldiisopropylamine, which is used in dimethylformamide, preferably at 80-100 °C, but other tertiary amines and anorganic bases of sodium, potassium and lithium may be used as well.
Several polar solvents may be used, but dimethylformamide is a preferred one.

h. To obtain the regioisomers of the products according to g. 3-fluoro-2-halo-6-nitrophenol is reacted in the presence of an anorganic or organic base with either (R)- or (S)-2-L-1-OR″-propane, where L and halo are as defined under c. and R″ is a hydroxyl protecting group as exemplified before, to give after removing the protecting group under appropriate known conditions resp. (S)- or (R)-4-fluoro-3-halo-2-(1-hydroxyisopropoxy)nitrobenzene (II) without its regioisomer.

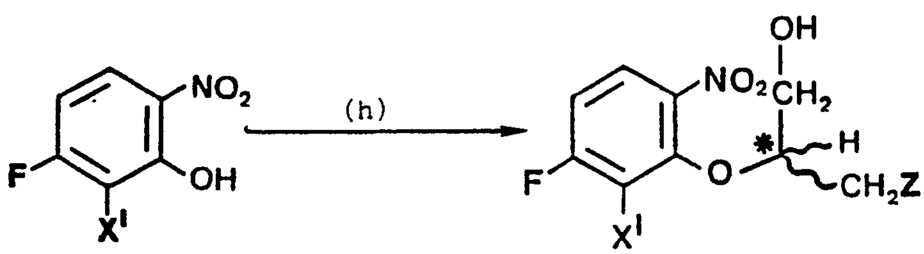


XVI II

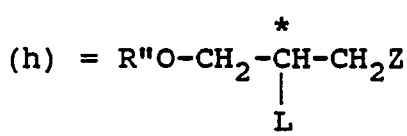

When using 3-fluoro-2-halo-6-nitrothiophenol with either (R)- or (S)-2-L-1-OR″-propane, respectively (S)- or (R)-4-fluoro-3-halo-2-(1-hydroxyisopropylthio)nitrobenzene is formed, also without any regioisomer.
The preferred reaction conditions with respect to base and solvent are as mentioned under g.

i. The most preferred method, however, starts with a 2,4-difluoro-3-halonitrobenzene as mentioned under a., which is reacted between room temperature and reflux temperature with either (R)- or (S)-2-OR″-propanol, where R″ is a hydroxyl protecting group as exemplified before and halo is fluoro or chloro, under basic conditions to give after removing the protecting group under appropriate known conditions resp. (R)- or (S)-4-fluoro-3-halo-2-(2-hydroxypropoxy)nitrobenzene (I) without any regioisomer.

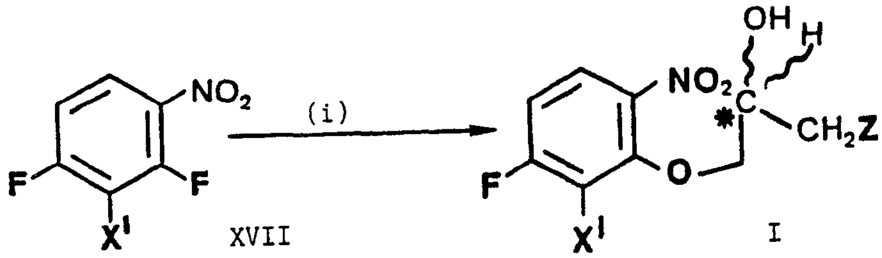

XVII I

(i) = 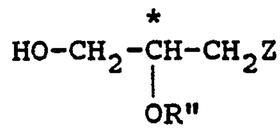

When using (R)- or (S)-2-OR″-propanethiol then (R)- or (S)-4-fluoro-3-halo-2-(2-hydroxypropylthio)-nitrobenzene is obtained.

Suitable basic conditions are as exemplified under a. With cheap anorganic bases such as potassium hydroxide or potassium carbonate and preferably with a mixture of both excellent results are obtained, with yields amounting over 90%. A phase transfer catalyst, e.g. $^{+}N(C_4H_9)_4{}^{-H}SO_4$ may be useful. Suitable solvents are tetrahydrofuran and preferably toluene, but other solvents may be used as well. The removing of the hydroxyl protecting group can be carried out e.g. by contacting a solution of the protected compound in methanol with a strongly acidic ion-exchange resin, such as DOWEX 50W-X8-400 $H^+$ (RTM).

It should be remarked that during this preparation the chirality of the compounds is preserved, so that the process is most advantageous for the preparation of a stereochemically pure product departing from a pure optically active propanol derivative.

j. Analogous to the method under i. the regioisomer of the compounds under i. namely (R)- or (S)-4-fluoro-3-halo-2-(1-hydroxyisopropoxy)nitrobenzene (II) is obtained exclusively, using (R)- resp. (S)-1-OR″-2-propanol.

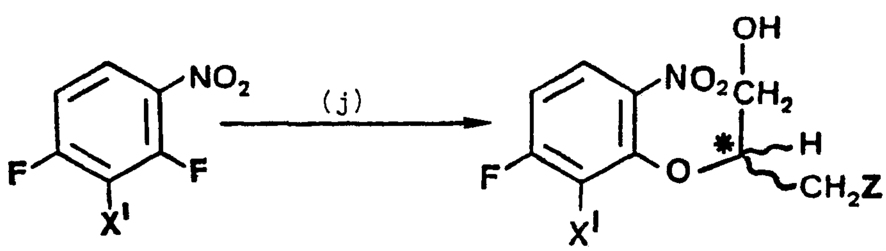

XVII II

(j) = 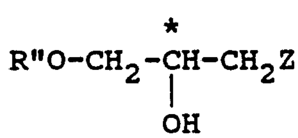

When using the corresponding thiol (analogous to method i.) then (R)- or (S)-4-fluoro-3-halo-2-(1-hydroxyisopropylthio)nitrobenzene is obtained.

It should be noted that with the methods under i. and j., using a 2,4-difluoro-3-halonitrobenzene as starting compound, remarkably little, if any, substitution of 4-fluoro occurs. This selectivity further contributes to the economy of the process.

The methods described under a. - j. are also suited to prepare the corresponding optically active 3-Z-substituted propoxy and 3-Z-substituted isopropoxy compounds where Z is fluoro or protected hydroxy (and their thio-analogues) by using as starting compounds respectively the compounds mentioned in Table I as Reactant 2, where Z is fluoro or protected hydroxy, R″ is a hydroxy protecting group and where L is a leaving group as exemplified above. In the event that the reaction affords a mixture of regioisomers, their ratio is dependent on the electronegativity of Z, especially when oxiranes are used as in method b. The

results of the various starting compound preparations with regard to the preservation of chirality and the occurrence of regioisomers are summarized in Table I.

Table I Preparation of starting compounds I and II (corresp. to IX and X)

| Method | Reactant 1 | Reactant 2 (R)- or (S)- | Preservation of chirality compounds (2) O | Preservation of chirality compounds (2) S | Absence of regioisomers in product (3) O | Absence of regioisomers in product (3) S |
|---|---|---|---|---|---|---|
| a. | 2,4-difluoro-3-halonitrobenzene | 3-Z-1,2-propanediol * | ++ | ++ | - | +(1) |
| b. | 3-fluoro-2-halo-6-nitrophenol * | 2-$CH_2$Z-oxirane | +/- | ++ | - | + |
| c. | 3-fluoro-2-halo-6-nitrophenol * | 1-L-3-Z-2-propanol | + | ++ | - | + |
| d. | 3-fluoro-2-halo-6-nitrophenol * | 3-Z-1,2-propanediol | ++ (i) | ++ (i) | - | - |
| e. | 3-fluoro-2-halo-6-nitrophenol * | 2-OR"-3-Z-propanol | ++ | ++ | + | + |
| f. | 3-fluoro-2-halo-6-nitrophenol * | 1-OR"-3-Z-2-propanol | ++ (i) | ++ (i) | + | + |
| g. | 3-fluoro-2-halo-6-nitrophenol * | 1-L-2-OR"-3-Z-propane | + | ++ | + | + |
| h. | 3-fluoro-2-halo-6-nitrophenol * | 2-L-1-OR"-3-Z-propane | + (i) | ++ (i) | + | + |
| i. | 2,4-difluoro-3-halonitrobenzene | 2-OR"-3-Z-propanol * | ++ | ++ | + | + |
| j. | 2,4-difluoro-3-halonitrobenzene | 1-OR"-3-Z-2-propanol * | ++ | ++ | + | + |

* inclusive of the corresponding thio compound

Z hydrogen, fluoro, protected hydroxy

L leaving group

(i) inversion with one of the regioisomers

(1) low yields only

(2) ++ : optically pure products (< 2% stereoisomer)

+ : substantially pure products (< 6% stereoisomer)

+/-: more or less pure depending on the nature of Z

(3) + : regioisomers are not formed; - : regioisomers are formed

compounds O: characterised by an (iso)propoxy substituent

compounds S: characterised by an (iso)propylthio subst.

With the obtained Z-substituted compounds, using the process of the invention as described before, optically active 2-$CH_2Z$- and 3-$CH_2Z$-benzoxazines and the corresponding benzothiazines are obtained.

The (R)- and (S)-7-fluoro-8-X-3,4-dihydro-2H-[1,4]benzoxazines and -benzothiazines with the general formula III, where one of the substituents $R^1$-$R^4$ is $CH_2OH$ or $CH_2Z$, the remaining being hydrogen, where Z is hydrogen, fluoro or protected hydroxy and X is fluoro, chloro, methyl or hydrogen with the proviso that for benzoxazines where $R^3$ and $R^4$ both are hydrogen, X is methyl or hydrogen, and the corresponding benzothiazines are novel compounds.

The optically active, substituted benzoxazines and benzothiazines prepared according to the present invention are useful compounds. Representatives from this group are starting compounds in known processes for the preparation of pyridobenzoxazines and pyridobenzothiazines, among which are important antibacterial quinolones such as (S)-ofloxacin (formula IV)

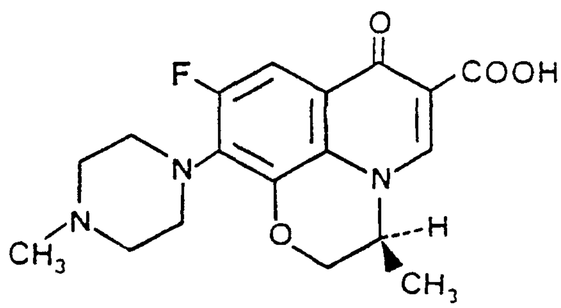

IV

Whereas prior art methods suited for the production of pyridobenzoxazines and pyridobenzothiazines often use starting compounds which are racemic, so that a resolution step is unavoidable when an optically active end product is desired, the present invention offers a method without an expensive resolution step, because the present process affords relatively cheap starting compounds which are themselves already optically pure, or at least substantially pure and remain so in the subsequent steps of the known process during which chirality is preserved. In the most preferred embodiments not more than 2% of the other stereoisomer could be detected in the resulting product.

Therefore according to another aspect of this invention optically active pyridobenzoxazines and pyridobenzothiazines are prepared by methods known per se from the benzoxazines and benzothiazines obtained according to this invention and characterised by the stereochemical configuration necessary to obtain the specifically desired, optically active end product.

These products belong to a group of (R)- and (S)-10-A-9-fluoro-2,3-dihydro-2(or 3)-$CH_2Z$-7-oxo-7H-pyrido[1,2,3-de][1,4]benzoxazine-6-carboxylic acids, and the corresponding thia-analogues, which group is represented by formula V

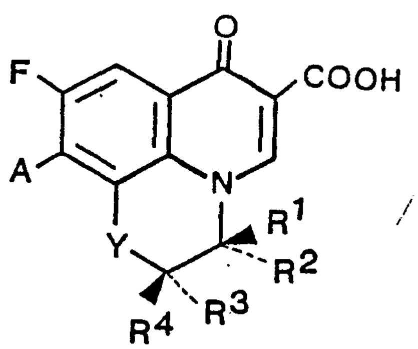

V

where one of $R^1$, $R^2$, $R^3$ or $R^4$ is $CH_2Z$ or $CH_2OH$ and the remaining R's are hydrogen, A is X or $NQ^1Q^2$, where X, Y and Z are as defined before and where $Q^1$ and $Q^2$ are the same or different and are either hydrogen or a (1-6C)alkyl group, with the proviso that $Q^1$ and $Q^2$ are not both hydrogen, or $Q^1$ and $Q^2$ together form a

(3-7C)heterocyclic ring which contains one or two nitrogen ring atoms and/or an oxygen ring atom and which ring may be substituted by a (1-4C)alkyl group. Suitable heterorings are: piperazine, 1-methylpiperazine, pyrrolidine, 2- or 3-methylpyrrolidine and imidazole.

Compounds of formula V, where A and $R^1$-$R^4$ are as defined above with the proviso that for pyridobenzoxazines where $R^3$ and $R^4$ both are hydrogen A is methyl or hydrogen, are novel compounds.

A suitable method known in the art to prepare these compounds from the benzoxazines and benzothiazines of formula III obtained according to this invention comprises

a. reacting the benzoxazine or benzothiazine with a di(1-6C)alkyl (preferably diethyl) ethoxymethylenemalonate,

b. subsequent ring closure in the presence of an acid,

c. hydrolysing the obtained ethyl ester according to formula VI

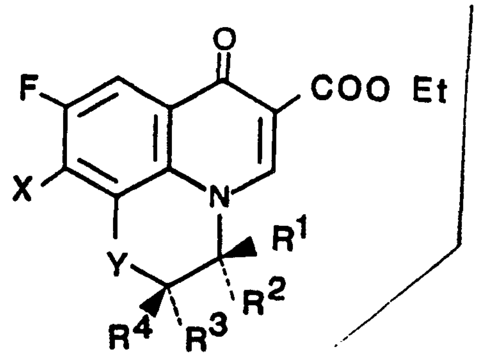

VI

where $R^1$, $R^2$, $R^3$, $R^4$, X and Y are as defined before to the corresponding carboxylic acid,

d. optionally substituting the 10-fluoro atom of the obtained carboxylic acid by an amine $NHQ^1Q^2$, where $Q^1$ and $Q^2$ are as defined before.

In the event that a 10-chloro-pyridobenzothiazine is to be substituted, the sulfur should first be oxidized to sulfoxide or sulfon and reduced again after the substitution reaction.

To the novel pyridobenzothiazines prepared according to the invention belong compounds which exhibit, as shown in Table II, remarkable antibacterial activity, which is equal to or slightly less than the antibacterial activity of ofloxacin. They have the additional advantage that they cannot pass the blood/brain barrier.

Table II

| Bacterium | (S)-ofloxacin | | GBR 23790 | |
|---|---|---|---|---|
| | MIC | MBC | MIC | MBC |
| 1. Staph. aureus | 0.04 | 0.04 | 0.08 | 0.08 |
| | 0.16 | 0.32 | 0.16 | 0.32 |
| 2. E. coli | 0.04 | 0.04 | 0.08 | 0.08 |
| | 0.04 | 0.04 | 0.08 | 0.08 |
| 3. Strep. faecalis | 1.25 | 1.25 | 1.25 | 2.5 |
| | 1.25 | 1.25 | 1.25 | 2.5 |
| 4. Ps. aeruginosa | 0.63 | 1.25 | 1.25 | 2.5 |
| | 0.63 | 0.63 | 1.25 | 2.5 |

GBR 23790 is (S)-9-fluoro-3-methyl-10-(4-methyl-1-piperazinyl)-2,3-dihydro-7-oxo-7H-pyrido[1,2,3-de][1,4]-benzothiazine-6-carboxylic acid, which is S-ofloxacin, but with the ring oxygen atom substituted by a ring sulfur atom.

MIC = Minimum Inhibitory Concentration (mg/l)

MBC = Minimum Bactericidal Concentration (mg/l)

Each assay has been carried out in duplicate.

GBR 23790 in racemic form is known from EP 0165375 (compound (p) in Table I). Its MIC-values, are for bacteria 1, 2 and 4 in the above Table II, resp. 0.78, 0.78 and 50. The racemic substance, apparently, is far less potent.

According to a further aspect of the invention therapeutically useful representatives of the pyridobenzoxazines of formula V obtained according to the invention or the corresponding pyridobenzothiazines as such, particularly optically active (S)-ofloxacin and their pharmaceutically acceptable salts are used in an antibacterially effective amount for the preparation of pharmaceutical compositions.

These compounds can be administered alone, but will generally be administered in admixture with a pharmaceutically acceptable diluent or carrier selected with regard to the intended route of administration and standard pharmaceutical practice. For example, they can be administered orally either in the form of tablets containing such excipients as starch or lactose, or in capsules either alone or in admixture with excipients, or in the form of elixirs or suspensions containing flavouring or colouring agents. The compounds can be contained in compositions suited for parenteral, intramuscular, intravenous or subcutaneous use. For parenteral administration they are best used in the form of a sterile aqueous solution which can contain other solutes, for example, enough salt or glucose to make the solution isotonic.

The following examples are meant to be illustrative and should not be construed as a limitation of the invention.

## EXAMPLES

### General remarks

In general, NMR spectra were measured on a Brucker AM 360 MHz spectrophotometer. In some cases Brucker's AM 600 MHz spectrophotometer was used.

(R)-2,2,2-Trifluoro-1-(9-anthryl)ethanol was introduced for the NMR spectral determinations of optical purity, preferably at 253° K.

Optically active (3C)-starting compounds were prepared from isobutyl (R)-lactate, ethyl (S)-lactate and (R)-2,3-isopropylidenedioxy-1-propanol (R-solketal) using well-known methods. (R)- and (S)-propylene oxide and also (R)-2,3-isopropylidenedioxy-1-propanol were used as such.

All these compounds were commercially available.

Usually, the reactions were carried out under a stream of dry $N_2$.

As regards the nomenclature, a substituted isopropoxy group was introduced which is synonymous with a substituted 1-methylethoxy group. For example, the name 3,4-difluoro-2-(1-hydroxyisopropoxy)nitrobenzene is used for 3,4-difluoro-2-(2-hydroxy-1-methylethoxy)nitrobenzene.

### Example 1

(R)-3,4-Difluoro-2-(2-hydroxypropoxy)nitrobenzene and (R)-3,4-difluoro-2-(1-hydroxyisopropoxy)nitrobenzene

A solution of 11.4 g of (R)-1,2-propanediol in 50 ml of tetrahydrofuran was added to a suspension of 1.8 g of sodium hydride in 75 ml of tetrahydrofuran while stirring at 0° C. An oily precipitate of the sodium salt was formed. After stirring for 20 minutes the reaction mixture was cooled to -40° C and a solution of 13.28 g of 2,3,4-trifluoronitrobenzene in 75 ml of tetrahydrofuran was added. After reacting for 1 hour at -40° C and 1 hour at 0° C the reaction mixture was evaporated to dryness, the residue was dissolved in diethyl ether and the solution was washed with water. The ethereal layer was dried over anhydrous sodium sulfate, filtered and evaporated under reduced pressure. The residue was chromatographed on a silica gel column (eluent: diethyl ether/hexane: 1/1). The product, 10.3 g of an oil, proved to be a 3:2 mixture of the title compounds. Yield: 59%. The mixture had a $[\alpha]_D^{22}$ = + 28.8° (c=2, $CHCl_3$) and was used as such in example 2.

$^1H$ NMR ($CDCl_3$, ppm) major compound: delta = 1.26 (d, 3H), 2.66 (br.s, 1H), 4.11 and 4.39 (2x m, 2H), 4.23 (m, 1H), 7.02 (m, 1H), 7.74 (m, 1H).

Minor compound: delta = 1.40 (d, 3H), 2.66 (br.s, 1H), 3.80 (m, 2H), 4.71 (m, 1H), 7.02 (m, 1H), 7.72 (m,

1H).

## Example 2

(R)-3,4-Difluoro-2-(2-mesyloxypropoxy)nitrobenzene and (R)-3,4-difluoro-2-(1-mesyloxyisopropoxy)-nitrobenzene

To 14.67 g of the mixture described in example 1 and dissolved in 250 ml of anhydrous diethyl ether were added 12.0 ml of triethylamine. To this mixture a solution of 7.44 g methanesulfonyl chloride in 75 ml of anhydrous diethyl ether was added dropwise with stirring while keeping the temp. between 15 and 20°C. After stirring for 0.5 hour conversion was completed, as evidenced by TLC on silica gel (hexane/diethyl ether: 2/3), and water was added, the organic phase separated, washed five times with water and dried over anhydrous sodium sulfate.
The organic solution was then filtered and evaporated under reduced pressure to afford 19.3 g of a 3:2 mixture of the title compounds. Yield: 98.5%. This mixture of mesylates was used as such in example 3.

## Example 3

(R)-3,4-Difluoro-2-(2-mesyloxypropoxy)aniline and (R)-3,4-difluoro-2-(1-mesyloxyisopropoxy)aniline

To 18.7 g of the mixture prepared in example 2 and dissolved in 60 ml of abs. ethanol were added 3 g of 10% Pd/C and the mixture was saturated with $H_2$ at 20-25°C while stirring. After the required amount of $H_2$ had been absorbed the reaction mixture was filtered over Hyflo (RTM) and the filtrate concentrated. The residue was dissolved in diethyl ether, the ethereal solution washed with water, dried over anhydrous sodium sulfate, filtered and evaporated under reduced pressure. The yield was 16.3 g of a 3:2 mixture of the title compounds (96%). This mixture was used as such in example 4.

## Example 4

(S)-(-)-7,8-Difluoro-3,4-dihydro-3-methyl-2H-[1,4]benzoxazine

14.2 G of potassium tert-butoxide were added in small amounts while stirring to a solution of 16.02 g of the mixture prepared in example 3 and dissolved in 500 ml of anhydrous tetrahydrofuran. The temperature was kept at 20-25°C and the reaction mixture was stirred for 30 minutes. Next, the mixture was evaporated to dryness and the residue was dissolved in a 1:1 mixture of water and diethyl ether. The organic layer was separated, washed with water, dried over anhydrous sodium sulfate, filtered and evaporated. The oily residue (10 g) contained two products, which were separated by column chromatography on silica gel (eluent: diethyl ether/ hexane: 1/1). The fractions containing the main product were combined and evaporated to afford 3.5 g of (S)-7,8-difluoro-3,4-dihydro-3-methyl-2H-[1,4]benzoxazine. Yield: 33%. $[\alpha]_D^{22}$ = -6.3° (c=2.1, $CHCl_3$). The product was contaminated with less than 2% of the (R)-isomer.
$^1H$ NMR ($CDCl_3$, ppm) delta = 1.19 (d, 3H), 3.50 (m, 1H), 3.79 and 4.28 (2x dd, 1H), 6.25 (m, 1H), 6.54 (m, 1H).

## Example 5

(R)-(+)-7,8-Difluoro-3,4-dihydro-2-methyl-2H-[1,4]benzoxazine

The fractions containing the by-product of example 4 were combined and evaporated to dryness to afford 2.6 g of (R)-7,8-difluoro-3,4-dihydro-2-methyl-2H-[1,4]benzoxazine. Yield: 25%.
$[\alpha]_D^{22}$ = +37.7° (c=2.33, $CHCl_3$). The optical purity was higher than 99.0%, as shown by NMR spectroscopy.
The NMR spectrum was identical to that of (R)-7,8-difluoro- 3,4-dihydro-2-methyl-2H-[1,4]benzoxazine, prepared previously by converting 2,3-difluor-6-nitrophenol with (R)-propylene oxide and ethyldiisopropylamine at 140° C. After the usual handling a 3:2 mixture of (R)-3,4-difluoro-2-(2-hydroxypropoxy)-nitrobenzene and (R)-3,4-difluoro-2-(1-hydroxyisopropoxy)nitrobenzene was obtained. The mixture as such was mesylated, reduced, cyclized and finally separated as described in the examples 2, 3 and 4.
$^1$H NMR ($CDCl_3$, ppm) delta = 1.42 (d, 3H), 3.10 and 3.36 (2x dd, 2H), 3.26 (br.s, 1H), 4.28 (m, 1H), 6.28 (m, 1H), 6.54 (m, 1H).

## Example 6

### Ethyl (S)-(-)-9,10-difluoro-2,3-dihydro-3-methyl-7-oxo-7H-pyrido[1,2,3-de][1,4]benzoxazine-6-carboxylate

A mixture of 2.66 g of (S)-(-)-7,8-difluoro-3,4-dihydro-3-methyl-2H-[1,4]benzoxazine, as such produced in example 4, and 3.11 g of diethyl ethoxymethylenemalonate was stirred under a nitrogen atmosphere and heated at 140-145° C for 2 hours. After cooling 20 g of ethyl polyphosphate were added and the mixture was again heated at 140-145° C for 1 hour. After cooling the reaction mixture was poured into ice water and extracted three times with chloroform. The collected organic layers were washed with 5% sodium carbonate solution and water. The organic solution was dried over anhydrous sodium sulfate, filtered and evaporated to dryness. Diethyl ether was added to the residue and the crystalline material was filtered, washed and dried to afford 3.1g of title product (70%). M.p. 254-255° C.$[\alpha]_D^{22}$ = -64.8° (c=0.25, acetic acid).
$^1$H NMR ($CF_3COOD$, ppm) delta = 1.19 (tr, 3H), 1.44 (d, 3H), 4.38 (m, 4H), 4.84 (m, 1H), 7.75 (dd, 1H), 8.97 (s, 1H).

## Example 7

### (S)-(-)-9,10-Difluoro-2,3-dihydro-3-methyl-7-oxo-7H-pyrido[1,2,3-de][1,4]benzoxazine-6-carboxylic acid

A mixture of 3.2 g of ethyl (S)-(-)-9,10-difluoro-2,3-dihydro-3-methyl-7-oxo-7H-pyrido[1,2,3-de][1,4]-benzoxazine-6-carboxylate, 32 ml of acetic acid and 8 ml of conc. hydrochloric acid was heated at reflux temperature for 3 hours. After cooling to 5° C the precipitated crystals were collected by filtration and washed successively with water, ethanol and diethyl ether.
After drying 2.64 g of title product were obtained. Yield: 91%. M.p. >300° C. $[\alpha]_D^{22}$ = -64.9° (c=1.0, DMSO).
$^1$H NMR ($CF_3COOD$, ppm) delta = 1.46 (d, 3H), 4.38 (ABq, 2H), 4.85 (m, 1H), 7.75 (dd, 1H), 9.04 (s, 1H).

## Example 8

### (S)-(-)-9-Fluoro-2,3-dihydro-3-methyl-10-(4-methyl-1-piperazinyl)-7-oxo-7H-pyrido[1,2,3-de][1,4]benzoxazine-6-carboxylic acid

7 Ml of $BF_3$ etherate were added dropwise to a suspension of 1.41 g of (S)-(-)-9,10-difluoro-2,3-dihydro-3-methyl-7-oxo-7H-pyrido[1,2,3-de][1,4]benzoxazine-6-carboxylic acid in 150 ml of diethyl ether while stirring at room temperature. The reaction mixture was stirred for 5 hours, then the precipitate was collected by suction, washed with diethyl ether and dried under reduced pressure.
The resulting chelate was dissolved in 15 ml of dry dimethylsulfoxide, and 1.42 ml of triethylamine and 0.8

g of N-methylpiperazine were added to the solution. The mixture was stirred at room temperature for 18 hours, followed by concentration to dryness under reduced pressure. The residue was treated with diethyl ether and dissolved in 40 ml of methanol and 1.42 ml of triethylamine and the solution was heated at reflux temperature for 24 hours. After cooling the reaction mixture was concentrated to dryness, the residue was dissolved in 50 ml of 10% hydrochloric acid and the solution was extracted three times with chloroform. The aqueous layer was separated and adjusted to pH = 11 with 4N sodium hydroxide and then to pH = 7.3 with 1N hydrochloric acid. The solution was extracted three times with 200 ml portions of chloroform, the extract was dried over anhydrous sodium sulfate, filtered and concentrated to dryness. The resulting powder was recrystallized from ethanol-diethyl ether to afford 1.0 g of the title product. Yield: 55%. M.p. 240-245 °C. (dec). $[\alpha]_D^{22}$ = -77.8° (c = 0.2, 0.05N NaOH).

## Example 9

(S)-(-)-9-Fluoro-2,3-dihydro-3-methyl-10-(4-methyl-1-piperazinyl)-7-oxo-7H-pyrido[1,2,3-de][1,4]benzoxazine-6-carboxylic acid

1.2 G of N-methylpiperazine were added to 1.15 g of (S)-(-)-9,10-difluoro-2,3-dihydro-3-methyl-7-oxo-7H-pyrido[1,2,3-de][1,4]benzoxazine-6-carboxylic acid dissolved in 6 ml of pyridine. Next, the mixture was heated at 120 °C for 10 hours, while stirring.

After cooling the workup was carried out as described in example 8.

In this case diethyl ether was added to the residue, obtained by evaporating the chloroform extract. The crystalline material thus formed was filtered off, washed and dried to afford 1.3 g of crude material. Recrystallization from isopropanol afforded 1.1g of the title product. Yield: 74.3%. $[\alpha]_D^{22}$ = -78.1° (c = 0.2, 0.05N NaOH). M.p. 225-226 °C.

$^1$H NMR ($CDCl_3$, ppm) delta = 1.62 (d, 3H), 2.38 (s, 3H), 2.58 (br.s, 4H), 3.41 (m, 4H), 4.44 (ABq, 2H), 4.59 (m, 1H), 7.67 (d, 1H), 8.65 (s, 1H).

## Example 10

Ethyl (R)-(-)-9,10-difluoro-2,3-dihydro-2-methyl-7-oxo-7H-pyrido[1,2,3-de][1,4]benzoxazine-6-carboxylate

A mixture of 1.00 g of R-(+)-7,8-difluoro-3,4-dihydro-2-methyl-2H-[1,4]benzoxazine, as such produced in example 5, was allowed to react with 1.16 g of diethyl ethoxymethylenemalonate under the conditions as described in example 6 and the workup was as reported there.

1.3 G of the title product were isolated. Yield: 78%. M.p. 254-255 °C. $[\alpha]_D^{22}$ = -88.4. (c = 0.16, $CHCl_3$).

$^1$H NMR ($CF_3COOD$, ppm) delta = 1.16 (tr, 3H), 1.39 (d, 3H), 4.20 and 4.58 (2x dd, 2H), 4.33 (q, 2H), 4.42 (m, 1H), 7.69 (dd, 1H), 8.84 (s, 1H).

## Example 11

(R)-(-)-9,10-Difluoro-2,3-dihydro-2-methyl-7-oxo-7H-pyrido[1,2,3-de][1,4]benzoxazine-6-carboxylic acid

2.8 G of ethyl (R)-(-)-9,10-difluoro-2,3-dihydro-2-methyl-7-oxo-7H-pyrido[1,2,3-de][1,4]benzoxazine-6-carboxylate were treated with 28 ml of acetic acid and 7 ml of conc. hydrochloric acid as described in example 7.

After the usual workup 2.2 g of title product were obtained. Yield: 87%. M.p. 278-280 °C. $[\alpha]_D^{22}$ = -56.4° (c = 0.2, $CHCl_3$).

$^1$H NMR ($CF_3COOD$, ppm) delta = 1.46 (d, 3H), 4.28 and 4.67 (dd, d, 2H), 4.49 (m, 1H), 7.76 (m, 1H), 8.79 (s, 1H).

## Example 12

(R)-(-)-9-Fluoro-2,3-dihydro-2-methyl-10-(4-methyl-1-piperazinyl)-7-oxo-7H-pyrido[1,2,3-de][1,4]benzoxazine-6-carboxylic acid

A mixture of 2.0 g of (R)-(-)-9,10-difluoro-2,3-dihydro-2-methyl-7-oxo-7H-pyrido[1,2,3-de][1,4]-benzoxazine-6-carboxylic acid and 2.1 g of N-methylpiperazine in 10.4 ml of pyridine was heated at reflux temperature for 6 hours. After cooling the reaction mixture was evaporated to dryness, then water was added to the residue and the crystalline product so obtained was filtered and washed with water and ethanol.

Further purification was carried out by dissolving the crude product in diluted sodium hydroxide, and crystallization was induced by adding 1N hydrochloric acid up to pH=7.3. The crystals were filtered, washed and dried to yield 2.2 g of the title compound. Yield: 85%. M.p. 295°C (dec). $[\alpha]_D^{22}$ = -46.9° (c=0.29, 0.05N NaOH).

$^1$H NMR ($CDCl_3$, ppm) delta = 1.63 (d, 3H), 2.49 (s, 2H), 2.72 (br.s, 4H), 3.49 (br.s, 4H), 4.06 and 4.33 (2x dd, 2H), 4.48 (m, 1H), 7.72 (d, 1H), 8.58 (s, 1H).

## Example 13

(S)-3,4-Difluoro-2-(2-hydroxypropoxy)nitrobenzene and (S)-3,4-difluoro-2-(1-hydroxyisopropoxy)nitrobenzene

7.6 G of (S)-1,2-propanediol dissolved in 25 ml of tetrahydrofuran, were added to 1.2 g of sodium hydride suspended in ml of tetrahydrofuran and after metallation 8.85 g of 2,3,4-trifluoronitrobenzene in 25 ml of tetrahydrofuran were added.

Both reaction and workup conditions were similar to those described in example 1.

After purification by column chromatography 8 g of an oily product were obtained, consisting of a 3:2 mixture of the title isomers. The mixture had $[\alpha]_D^{22}$ = -29.3° (c= 2.09, $CHCl_3$). Both isomers proved to be optically pure as confirmed by NMR.

$^1$H NMR ($CDCl_3$, ppm) major compound: delta = 1.26 (d, 3H), 4.12 and 4.38 (2x m, 2H), 4.22 (m, 1H), 7.02 (m, 1H), 7.72 (m, 1H).

Minor compound: delta = 1.40 (d, 3H), 3.77 (m, 2H), 4.70 (m, 1H), 7.02 (m, 1H), 7.68 (m, 1H).

In addition, 1.3 g of the slightly impure (S)-1,2-bis(2,3-difluoro-6-nitrophenoxy)propane were isolated.

$^1$H NMR ($CDCl_3$, ppm) delta = 1.53 (d, 3H), 4.38 and 4.61 (2x m, 2H), 4.99 (m, 1H), 7.01 (m, 1H), 7.67 (m, 1H).

## Example 14

(S)-3,4-Difluoro-2-(2-mesyloxypropoxy)nitrobenzene and (S)-3,4-difluoro-2-(1-mesyloxyisopropoxy)-nitrobenzene

11.1 G of the mixture described in example 13 and 9.2 ml of triethylamine in 200 ml of diethyl ether were treated with 5.7 g of methanesulfonyl chloride diluted with 50 ml of diethyl ether as described in example 2.

After the usual workup 14.3 g of a 3:2 mixture of the title compounds were isolated. Yield: 100%. The mixture had $[\alpha]_D^{22}$ = +27.1° (c=1.64, $CHCl_3$) and was used as such in example 15.

$^1$H NMR ($CDCl_3$, ppm) major compound: delta = 1.53 (d, 3H), 3.07 (s, 3H), 4.38 (m, 2H), 5.13 (m, 1H), 7.10 (m, 1H), 7.74 (m, 1H). Minor compound: delta = 1.47 (d, 3H), 3.03 (s, 3H), 4.38 (m, 2H), 4.83 (m, 1H), 7.10 (m, 1H), 7.74 (m, 1H).

Example 15

(S)-3,4-Difluoro-2-(2-mesyloxypropoxy)aniline and (S)-3,4-difluoro-2-(1-mesyloxyisopropoxy)aniline

To 14.0 g of the mixture prepared in example 14 and dissolved in 200 ml of abs. ethanol were added 3 g of 10% Pd/C and the mixture was saturated with $H_2$ at 20-25 °C, while stirring vigorously.
After absorption of the required amount of $H_2$ the reaction mixture was handled according to example 3. 11.9 G of a mixture of the title compounds were isolated. Yield: 94%. The mixture was used as such in example 16.

Example 16

(R)-(+)-7,8-Difluoro-3,4-dihydro-3-methyl-2H-[1,4]benzoxazine

10.0 G of potassium tert-butoxide were added in small portions while stirring to 11.24 g of the mixture as prepared in example 15 and dissolved in 300 ml of anhydrous tetrahydrofuran.
After workup according to example 4, 1.5 g of title product were obtained in an optically pure form (less than 1% of the enantiomer was detected by NMR). Yield: 20%. $[\alpha]_D^{22}$ = +6.7° (c=1.79, $CHCl_3$).
The NMR spectrum was identical to that in example 4.

Example 17

(S)-(-)-7,8-Difluoro-3,4-dihydro-2-methyl-2H-[1,4]benzoxazine

The fractions containing the by-product of example 16 were collected and evaporated to afford 1.15 g of optically pure (S)-7,8-difluoro-3,4-dihydro-2-methyl-2H-[1,4]benzoxazine, as shown by NMR spectroscopy.
Yield: 15%. $[\alpha]_D^{22}$ = -38.3° (c=1.91, $CHCl_3$).
The NMR spectrum was identical to that in example 5.

Example 18

Ethyl (R)-(+)-9,10-difluoro-2,3-dihydro-3-methyl-7-oxo-7H-pyrido[1,2,3-de][1,4]benzoxazine-6-carboxylate

The reaction was carried out starting from 1.35 g of (R)-(+)-7,8-difluoro-3,4-dihydro-3-methyl-2H-[1,4]-benzoxazine and 1.58 g of diethyl ethoxymethylenemalonate for the condensation reaction and 10 g of ethyl polyphosphate for the ring closure reaction according to the procedure described in example 6.
1.38 G of title compound were obtained. Yield: 61%.
$[\alpha]_D^{22}$ = +67.8 °C (c=0.24, acetic acid).
$^1H$ NMR ($CF_3COOD$, ppm) delta =1.21 (tr, 3H), 1.47 (d, 3H), 4.41 (m, 4H), 4.88 (m, 1H), 7.78 (tr, 1H), 9.00 (s, 1H).

Example 19

(R)-(+)-9,10-Difluoro-2,3-dihydro-3-methyl-7-oxo-7H-pyrido[1,2,3-de][1,4]benzoxazine-6-carboxylic acid

A mixture of 1.3 g of (R)-(+)-9,10-difluoro-2,3-dihydro-3-methyl-7-oxo-7H-pyrido[1,2,3-de][1,4]-benzoxazine-6-carboxylate, 13 ml of acetic acid and 3.25 ml of conc. hydrochloric acid was heated at reflux temperature for 3 hours.
After cooling to 0 °C the crystals were collected by filtration and washed successively with water, ethanol and diethyl ether. After drying 1.05 g of title product were obtained. Yield: 89%. M.p. >300 °C. $[\alpha]_D^{22}$ = +64.0° (c=0.2, DMSO).
$^1$H NMR ($CF_3COOD$, ppm) delta = 1.53 (d, 3H), 4.46 (ABq, 2H), 4.74 (m, 1H), 7.84 (tr, 1H), 9.13 (s, 1H).

Example 20

(R)-(+)-9-Fluoro-2,3-dihydro-3-methyl-10-(4-methyl-1-piperazinyl)-7-oxo-7H-pyrido[1,2,3-de][1,4]-benzoxazine-6-carboxylic acid

A mixture of 0.95 g of (R)-(+)-9,10-difluoro-2,3-dihydro-3-methyl-7-oxo-7H-pyrido[1,2,3-de][1,4]-benzoxazine-6-carboxylic acid, 5 ml of pyridine and 1.0 g of N-methylpiperazine was stirred at reflux temperature for 8 hours.
After cooling the reaction mixture was handled as described in example 8.
Diethyl ether was added to the residue and the solid material thus obtained was filtered and washed. The crude product was recrystallized from isopropanol to afford 1.0 g of the title product. Yield: 82%. M.p. 224 °C. $[\alpha]_D^{22}$ = +78.3° (c=0.2, 0.05N NaOH).
$^1$H NMR ($CDCl_3$, ppm) delta = 1.63 (d, 3H), 2.39 (s, 3H), 2.58 (br.s, 4H), 3.43 (m, 4H), 4.44 (ABq, 2H), 4.62 (m, 1H), 7.64 (d, 1H), 8.67 (s, 1H).

Example 21

Ethyl (S)-(+)-9,10-difluoro-2,3-dihydro-2-methyl-7-oxo-7H-pyrido[1,2,3-de][1,4]benzoxazine-6-carboxylate

The procedure described in example 10 was followed starting from 1.0g of (S)-(+)-7,8-difluoro-2,3-dihydro-2-methyl-4H-[1,4]benzoxazine.
Addition of diethyl ether afforded 1.3 g of title compound. Yield: 78%. M.p. 252-253 °C. $[\alpha]_D^{22}$ = +89.2 (c=0.2, $CHCl_3$).
$^1$H NMR ($CF_3COOD$, ppm) delta =1.25 (tr, 3H), 1.48 (d, 3H), 4.30 and 4.67 (dd, d, 2H), 4.42 (q, 2H), 4.52 (m, 1H), 7.78 (tr, 1H), 8.93 (s, 1H).

Example 22

(S)-(+)-9,10-Difluoro-2,3-dihydro-2-methyl-7-oxo-7H-pyrido[1,2,3-de][1,4]benzoxazine-6-carboxylic acid

1.25 G of ethyl (S)-(+)-9,10-difluoro-2,3-dihydro-2-methyl-7-oxo-7H-pyrido[1,2,3-de][1,4]benzoxazine-6-carboxylate were hydrolyzed and handled according to example 11. 0.9 G of the title product was obtained. Yield: 82%. M.p. 288-289 °C. $[\alpha]_D^{22}$ = +65.3. (c=0.15, $CHCl_3$).
$^1$H NMR ($CF_3COOD$, ppm) delta =1.43 (d, 3H), 4.26 and 4.64 (dd,d, 2H), 4.47 (m, 1H), 7.74 (dd, 1H), 8.96 (s, 1H).

Example 23

(S)-(+)-9-Fluoro-2,3-dihydro-2-methyl-10-(4-methyl-1-piperazinyl)-7-oxo-7H-pyrido[1,2,3-de][1,4]-benzoxazine-6-carboxylic acid

A mixture of 0.84 g of (S)-(+)-9,10-difluoro-2,3-dihydro-2-methyl-7-oxo-7H-pyrido[1,2,3-de][1,4]-benzoxazine-6-carboxylic acid, 4.3 ml of pyridine and 0.85 g of N-methylpiperazine was heated at reflux temperature for 8 hours, while stirring.
After cooling the reaction mixture was handled as described in example 12.
0.9 G of title product was isolated. Yield: 83%. M.p. 293-295 °C. $[\alpha]_D^{22}$ = +46.4° (c=0.19, 0.05N NaOH).
$^1$H NMR ($D_2O$, 1 drop of 10N NaOD, ppm) delta = 1.30 (d, 3H), 2.09 (s, 3H), 2.35 (br.s, 4H), 3.00 (br.s, 4H), 3.64 (tr, 1H), 4.09 (m, 2H), 6.94 (d, 1H), 7.97 (s, 1H).

## Example 24

(S)-(-)-3,4-Difluoro-2-[2-(2-tetrahydropyranyloxy)propoxy]nitrobenzene

A solution of 15.69 g of diethyl azodicarboxylate in 50 ml of tetrahydrofuran was added slowly to a solution of 12.29 g of 2,3-difluoro-6-nitrophenol, 11.25 g of (S)-2-(2-tetrahydropyranyloxy)-1-propanol and 23.68 g of triphenylphosphine in 250 ml of tetrahydrofuran. The addition was completed after 1.75 hours, the temperature being maintained at 25 °C by cooling in a water bath. The reaction mixture was stirred overnight at ambient temperature. Next, the solvent was removed by distilling and the residue was treated with a 1:1 mixture of diethyl ether and petroleum ether (40-60). The solid was separated by filtration and the solution was washed four times with 0.1N sodium hydroxide, once with brine and water, dried over anhydrous sodium sulfate and concentrated to afford 34.93 g of crude product. This material was chromatographed on a silica gel column (eluent: toluene/acetone: 9/1). The fractions containing the title product were collected and evaporated to dryness. 15.64 G of title compound were obtained. Yield: 70%. $[\alpha]_D^{24}$ = -13.9° (c=1.2,$CHCl_3$). The product proved to be a 3:2 mixture of diastereomers.
$^1$H NMR ($CDCl_3$, ppm) major compound, delta = 1.33 (d, 3H), 1.5-1.9 (m, 6H), 3.49 and 3.88 (2x m, 2H), 4.1-4.4 (m, 3H), 4.79 (tr, 1H), 6.98 (m, 1H), 7.65 (m. 1H).
Minor compound, delta = 1.28 (d, 3H), 1.5-1.9 (m, 6H), 3.49 and 3.88 (2x m, 2H), 4.1-4.4 (m, 3H), 4.73 (tr, 1H), 6.98 (m, 1H), 7.65 (m, 1H).

## Example 25

(S)-(-)-3,4-Difluoro-2-(2-hydroxypropoxy)nitrobenzene

To a suspension of 3.3 g of Dowex 50W-X8-400 $H^+$ (RTM) ion-exchange resin in 60 ml of methanol, 3.3 g of (S)-(-)-3,4-difluoro-2-[2-(2-tetrahydropyranyloxy)propoxy]nitrobenzene were added, while stirring.
After reacting at room temperature for 40 minutes, the ion-exchange resin was filtered and the filtrate was evaporated to dryness. The residue was dissolved in diethyl ether and the solution was washed with water, dried over anhydrous sodium sulfate, filtered and concentrated under reduced pressure. The residue, 2.3 of an oily product (100% yield), was used as such in example 26. The product had $[\alpha]_D^{22}$ = -3.9° (c=2.3, $CHCl_3$).
$^1$H NMR ($CDCl_3$, ppm) delta = 1.26 (d, 3H), 2.73 (br. s, 1H), 4.12 and 4.39 (2x m, 2H), 4.23 (m, 1H), 7.03 (m, 1H), 7.74 (m, 1H).

## Example 26

(S)-(-)-3,4-Difluoro-2-(2-mesyloxypropoxy)nitrobenzene

A solution of 1.15 g of methanesulfonyl chloride in 10 ml of diethyl ether was added to a stirred solution of 2.2 g of (S)-3,4-difluoro-2-(2-hydroxypropoxy)nitrobenzene and 1.5 g of triethylamine in 50 ml of diethyl ether while keeping the temperature between 15-20° C.
After 30 minutes the reaction mixture was handled in the usual way (example 2).
3.4 G of the title product were obtained. Yield: 100%. It was used in example 27 without further purification.
The product had $[\alpha]_D^{22}$ = -2.5° (c=2.0, $CHCl_3$).
$^1H$ NMR ($CDCl_3$, ppm) delta = 1.55 (d, 3H), 3.08 (s, 3H), 4.37 (m, 2H), 5.13 (m, 1H), 7.09 (m, 1H), 7.73 (m, 1H).

## Example 27

(S)-(-)-3,4-Difluoro-2-(2-mesyloxypropoxy)aniline

A solution of 3.3 g of (S)-(-)-3,4-fluoro-2-(2-mesyloxypropoxy)nitrobenzene in 60 ml abs. ethanol was reduced with 0.2 g of 10% Pd/C and $H_2$ and the reaction mixture was handled according to example 3.
The yield was 2.6 g of an oil (100%). The product had $[\alpha]_D^{22}$ = -3.5° (c=2, $CHCl_3$) and was used as such in example $^1H$ NMR ($CDCl_3$, ppm) delta = 1.47 (d, 3H), 3.09 (s, 3H), 4.17 (m, 2H), 5.15 (m, 1H), 6.43 (m, 1H), 6.73 (m, 1H).

## Example 28

(R)-(+)-7,8-Difluoro-3,4-dihydro-3-methyl-2H-[1,4]benzoxazine

2.5 G of (S)-(-)-3,4-difluoro-2-(2-mesyloxypropoxy)aniline in 80 ml of anhydrous tetrahydrofuran were cyclized by adding 2.3 g of potassium tert-butoxide and handled as described in example 4.
The residue was purified by column chromatography on silica gel (eluent: petroleum ether (40-60)/diethyl ether: 3/2) to afford 0.6 g of title product. Yield: 37.5%. $[\alpha]_D^{22}$ = +8.7° (c=2.0, $CHCl_3$). The product proved to be optically pure, as confirmed by NMR, and was contaminated with traces of 2-allyloxy-3,4-difluoroaniline.
$^1H$ NMR ($CDCl_3$, ppm) delta = 1.20 (d, 3H), 3.51 (m, 1H), 3.78 and 4.27 (2x m, 2H), 6.26 (m, 1H), 6.56 (m, 1H).
The reaction described above was repeated starting from 3.0 g of (S)-3,4-difluoro-2-(2-mesyloxypropoxy)-aniline. The residue was purified by chromatography on silica gel (eluent: petroleum ether (40-60)/diethyl ether: 1/1) to afford 0.85 g of the title product. Yield: 43%.
In addition, 0.25 g of a by-product was isolated, which showed a positive ninhydrine test. The structure was elucidated by NMR as (3R)-7,8-difluoro-2,3-dihydro-3-methyl-4-[(1R)-2-(6-amino-2,3-difluorophenoxy)-1-methylethyl][1,4]benzoxazine.
$^1H$ NMR ($CDCl_3$, ppm) delta = 1.21 and 1.35 (2x d, 6H), 3.48 (br.s, 2H), 3.6-4.3 (m, 6H), 6.30 and 6.47 (2x m, 2H), 6.64 (m, 2H).

## Example 29

(R)-3,4-Difluoro-2-[2-(2-tetrahydropyranyloxy)propoxy]nitrobenzene

0.2 G of 60% sodium hydride, previously washed with pentane, was suspended in 10 ml of tetrahydrofuran. Next, a mixture of 3.2 g of (R)-2-(2-tetrahydropyranyloxy)-1-propanol and 10 ml of tetrahydrofuran was added to the stirred suspension and the reaction mixture was stirred for 0.5 hour.
The resulting clear solution was added slowly to a stirred solution of 0.89 g of 2,3,4-trifluoronitrobenzene in 10 ml of tetrahydrofuran while keeping the temperature at 15-20° C. The reaction mixture was stirred for

another 0.5 hour, then concentrated. Water and diethyl ether were added to the residue. The organic layer was separated, washed with water, dried over anhydrous sodium sulfate, filtered and evaporated under reduced pressure.

The crude residue was purified by chromatography on silica gel (eluent: hexane/diethyl ether/triethylamine: 3/2/0.1) to afford 1.25 g of title product as a mixture of diastereomers (ratio: 55/45). Yield: 78.4%.

$^1H$ NMR ($CDCl_3$, ppm) delta = 1.30 (d, 3H), 1.33 (d, 3H), 1.4-1.9 (m, 6H), 3.50 and 3.88 (2x m, 2H), 4.1-4.4 (m, 3H), 4.75 (tr, 1H), 4.79 (tr, 1H), 6.99 (m, 1H), 7.68 (m, 1H).

## Example 30

(R)-3,4-Difluoro-2-(2-hydroxypropoxy)nitrobenzene

0.75 G of Dowex 50W-X8-400 $H^+$ (RTM) ion-exchange resin was added to a stirred solution of 0.75 g of (R)-3,4-difluoro-2-[2-(2-tetrahydropyranyloxy)propoxy]nitrobenzene in 15 ml of methanol. After stirring for 0.5 hour the ion-exchange resin was filtered off and the filtrate was concentrated under reduced pressure to afford 0.5 g of the oily title product. Yield: 95.2%. The product had $[\alpha]_D^{22}$ = +3.3° (c = 2.4; $CHCl_3$).

$^1H$ NMR ($CDCl_3$; ppm) delta = 1.27 (d, 3H), 2.93 (s, 1H), 4.12 and 4.38 (dABq, 2H), 4.23 (m, 1H), 7.03 (m, 1H), 7.73 (m, 1H).

## Example 31

(R)-2-[2-(1-Ethoxyethoxy)propoxy]-3,4-difluoronitrobenzene

0.4 G of 60% sodium hydride, previously washed with pentane, was suspended in 20 ml of tetrahydrofuran. Next, a mixture of 2.96 of (R)-2-(1-ethoxyethoxy)-1-propanol and 10 ml of tetrahydrofuran was added to the stirred suspension. After reacting for 0.5 hour the clear solution was added slowly to 1.79 g of 2,3,4-trifluoronitrobenzene dissolved in 10 ml of tetrahydrofuran and the reaction mixture was stirred for another 0.5 hour.

The solvent was removed under reduced pressure, and diethyl ether and water were added to the residue. The organic layer was separated, washed with water, dried over anhydrous sodium sulfate, filtered and evaporated to dryness. The crude residue was purified by chromatography on silica gel (eluent: hexane/diethyl ether/triethylamine: 7/3/0.1).

Finally, 2.5 g of title product were isolated as a mixture of diastereomers (ratio: 2/1). Yield: 82%.

$^1H$ NMR ($CDCl_3$, ppm) delta = 1.17 (tr, 3H), 1.20 (tr, 3H), 1.29 (m, 6H), 3.57 (m, 2H), 4.0-4.4 (m, 3H), 4.82 (q, 1H), 4.89 (q, 1H), 7.01 (m, 1H), 7.66 (m, 1H).

## Example 32

(R)-3,4-Difluoro-2-(2-hydroxypropoxy)nitrobenzene

1.0 G of Dowex 50W-X8-400 $H^+$ (RTM) ion-exchange resin was added to a stirred solution of 2.0 g (R)-3,4-difluoro-2-[2-(1-ethoxyethoxy)propoxy]nitrobenzene in 50 ml of methanol. After stirring for 1 hour the ion-exchange resin was filtered off and the filtrate was concentrated to afford 1.6 g of the oily title product. Yield: 100%.

The product has $[\alpha]_D^{22}$ = +4.0° (c=2.0, $CHCl_3$).

The NMR spectrum was identical to that of the product obtained in example 30.

## Example 33

(R)-3-Chloro-4-fluoro-2-(2-hydroxypropoxy)nitrobenzene

5.2 G of 60% sodium hydride, previously washed with pentane, were suspended in 200 ml of dry tetrahydrofuran. Next, a mixture of 41.6 g of (R)-2-(2-tetrahydropyranyloxy)-1-propanol and 100 ml of tetrahydrofuran was added to the stirred suspension at 15-20° C. After reacting for 0.5 hour the clear solution was added slowly to 21.3 g of 3-chloro-2,4-difluoronitrobenzene dissolved in 100 ml of tetrahydrofuran and the reaction mixture was stirred for another 0.5 hour.
The reaction mixture was concentrated and the residue was dissolved in diethyl ether, washed with water, dried over anhydrous sodium sulfate, filtered and evaporated to afford 51.0 g of crude product, (R)-3-chloro-4-fluoro-2-[2-(2- tetrahydropyranyloxy)propoxy]nitrobenzene. This product was dissolved in 300 ml of methanol and treated with 5.0 g of Dowex 50W-X8-400 $H^+$ (RTM) ion-exchange resin while stirring overnight. The ion-exchange resin was filtered off and the filtrate was concentrated under reduced pressure. The residue was distilled, yielding two fractions of the title product: 16.1 g of b.p. 130-140° C/0.5 mm Hg and 3.3 g of b.p. 140-150° C/0.5 mm Hg, which were combined. Yield: 68.4% (corr.).
The product had $[\alpha]_D^{22}$ = -12.5° (c=2.0, $CHCl_3$) and contained 10 mol % of 1,2-propanediol.
$^1H$ NMR ($CDCl_3$, ppm) delta = 1.29 (d, 3H), 2.95 (s, 1H), 4.08 and 4.18 (dABq, 2H), 4.27 (m, 1H), 7.10 (m, 1H), 7.87 (m, 1H).
The title product was optically pure; less than 1% of the enantiomer was detected by NMR.

## Example 34

(R)-3-Chloro-4-fluoro-2-(2-mesyloxypropoxy)nitrobenzene

15.0 G of 3-chloro-4-fluoro-2-(2-hydroxypropoxy)nitrobenzene were dissolved in 200 ml of dry diethyl ether and 12.5 ml of triethylamine. The solution was stirred and a mixture of 7.56 g of methanesulfonyl chloride and 50 ml of diethyl ether were added while keeping the temperature at 15-20° C. After stirring for 0.5 hour water was added, the organic layer was separated, washed with water, dried over anhydrous sodium sulfate, filtered and evaporated to dryness. 21.58° C of title product were obtained. Yield: 91.2%.
$^1H$ NMR ($CDCl_3$, ppm) delta = 1.54 (d, 3H), 3.07 (s, 3H), 4.23 and 4.38 (dABq, 2H), 5.18 (m, 1H), 7.13 (m, 1H), 7.87 (m, 1H).

## Example 35

(R)-3-Chloro-4-fluoro-2-(2-mesyloxypropoxy)aniline

To 6.55 g of (R)-3-chloro-4-fluoro-2-(2-mesyloxypropoxy)nitrobenzene, dissolved in 150 ml of toluene, was added 1.0 g of 10% palladium on charcoal. The mixture was saturated with $H_2$ at 20-25° C while stirring vigorously. After the required amount of $H_2$ had been absorbed some ml of ethanol were added to dissolve the amine. The reaction mixture was filtered over Hyflo (RTM) and the filtrate was concentrated under reduced pressure to afford 6.0 g of the oily title product (100% yield), which was used as such in cyclization.

## Example 36

(S)-8-Chloro-7-fluoro-3,4-dihydro-3-methyl-2H-[1,4]benzoxazine

1.49 G of (R)-3-chloro-4-fluoro-2-(2-mesyloxypropoxy)nitrobenzene in 20 ml of toluene were treated with 1.12 g of powdered potassium hydroxide, 0.7 g of powdered potassium carbonate and 85 mg of tetrabutylammonium hydrogen sulfate while stirring overnight. Water was added to the reaction mixture and the organic layer was separated, washed with water, dried over anhydrous sodium sulfate, filtered and evaporated to dryness. The residue was purified by chromatography on silica gel (eluent: diethyl ether/hexane: 1/1). 0.75 G of title product was obtained. Yield: 75%. The product had $[\alpha]_D^{22}$ = -17.9° (c = 2.0, $CHCl_3$).
$^1H$ NMR ($CDCl_3$, ppm) delta = 1.17 (d, 3H), 3.47 (m, 1H), 3.63 (s, 1H), 3.80 and 4.30 (dABq, 2H), 6.41 (m, 1H), 6.57 (m, 1H).

## Example 37

### (R)-2-(1-Benzyloxyisopropoxy)-3,4-difluoronitrobenzene

A solution of 5.31 g of 2,3,4-trifluoronitrobenzene and 5.48 g of (R)-1-benzyloxy-2-propanol in 30 ml of toluene was added to a stirred suspension of 6.72 g of powdered potassium hydroxide and 4.14 g of powdered potassium carbonate in 80 ml of toluene kept at -5 to -10° C. The reaction mixture was stirred for 30 minutes at -5° C. Water was added and the organic layer was separated. The aqueous layer was washed with toluene and the combined organic layers were washed with water to neutral. The toluene was evaporated and the residue was purified by chromatography on silica gel (eluent: toluene). 8.8 G of title compound were obtained. Yield: 91%. The product had $[\alpha]_D^{25}$ = -62.0° (C = 2.0, $CHCl_3$).
$^1H$ NMR ($CDCl_3$, ppm) delta = 1.40 (d, 3H), 3.55 and 3.65-3.73 (dd, m, 2H), 4.39 and 4.45 (2x d, 2H), 4.65-4.72 (m, 1H), 6.84-6.92 (m, 1H), 7.10-7.32 (m, 5H), 7.55-7.62 (m, 1H).

## Example 38

### (R)-3,4-Difluoro-2-(1-hydroxyisopropoxy)aniline

1.0 G of 10% Pd/C (palladium on charcoal) was added to a solution of 8.2 g of (R)-2-(1-benzyloxyisopropoxy)-3,4-difluoronitrobenzene in 80 ml of abs. ethanol and 60 ml of toluene and the mixture was saturated with $H_2$ at 20-25° C while stirring vigorously.
After the required amount of $H_2$ had been absorbed the reaction mixture was filtered over Hyflo (RTM) and the filtrate concentrated. The residue was purified by column chromatography on silica gel (eluent: diethyl ether).
4.0 G of the title compound were isolated as an oil, which solidified on standing. Yield: 77%. M.p. 58-59° C. The product had $[\alpha]_D^{20}$ = -50.2° (c = 1.8, $CHCl_3$).
$^1H$ NMR ($CDCl_3$, ppm) delta = 1.33 (d, 3H), 3.59-3.71 (m, 2H), 4.27 (m, 1H), 6.40 (m, 1H), 6.71 (m, 1H).

## Example 39

### (R)-7,8-Difluoro-3,4-dihydro-2-methyl-2H-[1,4]benzoxazine

A solution of 1.94 g of methanesulfonyl chloride in 20 ml of dry diethyl ether was added dropwise to a stirred solution of 3.05 g of (R)-3,4-difluoro-2-(1-hydroxyisopropoxy)aniline and 2.53 g of triethylamine in 70 ml of dry diethyl ether kept at -10 to -15° C. After stirring at 0° C for 3 hours water was added and the organic layer was separated, washed with water, dried over anhydrous sodium sulfate, filtered and evaporated to dryness. The residue was dissolved in 70 ml of toluene, then 3.36 g of powdered potassium hydroxide, 2.07 g of powdered potassium carbonate and 100 mg of tetrabutylammonium hydrogen sulfate were added and the mixture was stirred at 20° C for 8 hours. Water was added, the organic layer was

separated, washed with water, dried over anhydrous sodium sulfate, filtered and evaporated to dryness. The residue was purified by chromatography on silica gel (eluent: diethyl ether/hexane: 1/1). The fractions with product were combined and evaporated to dryness. The residue was treated with petroleum ether (40/60) and filtered. 0.2 G of solid (R)-7,8-difluoro-2,3-dihydro-4-mesyl-2-methyl-[1,4]benzoxazine was obtained.
$^1$H NMR ($CDCl_3$, ppm) delta = 1.48 (d, 3H), 2.96 (s, 3H), 3.16 and 4.24, (dd,d, 2H) 4.29 (m, 1H), 6.66-6.77 (m, 1H), 7.35-7.43 (m, 1H).
The filtrate was evaporated under reduced pressure to afford 2.0 g of the title compound. Yield: 72%. This product had $[\alpha]_D^{20}$ = +38.8 (c=2.0, $CHCl_3$). The optical purity was higher than 99%, as shown by NMR spectroscopy. The NMR spectrum was identical to that of the title compound in example 5.

## Example 40

### (R)-3,4-Difluoro-2-(2-hydroxypropylthio)nitrobenzene

To a stirred solution of 11.46 g of 3,4-difluoro-2-mercaptonitrobenzene in 250 ml of abs. ethanol were added 11.5 ml of ethyldiisopropylamine while maintaining the temperature at 20°C. After stirring for 10 minutes 4.94 g of (R)-1,2-propylene oxide were added and stirring was continued for 3 hours. The reaction mixture was concentrated under reduced pressure and water and diethyl ether were added to the residue. The ethereal layer was separated, washed successively with water, 2N sodium hydroxide and water. Next, the organic layer was dried over anhydrous sodium sulfate, filtered and evaporated to dryness to afford 15.0 g of crude product, which was purified by chromatography on silica gel (eluent: diethyl ether/hexane: 1/1). 13.3 G of oily title product were obtained. Yield: 89%. The product had $[\alpha]_D^{20}$ = -36.3° (c=2.0, $CHCl_3$).
$^1$H NMR ($CDCl_3$, ppm) delta = 1.257 (d, 3H), 2.534 (d, 1H), 2.983 and 3.179 (2x dd, 2H), 3.871 (m, 1H), 7.256 (m, 1H), 7.662 (m, 1H).

## Example 41

### (R)-3,4-Difluoro-2-(2-hydroxypropylthio)aniline

60 G of stannous chloride dihydrate were added to a stirred solution of 13.0 g of (R)-3,4-difluoro-2-(2-hydroxypropylthio)nitrobenzene in 100 ml of abs. ethanol. The reaction mixture was brought to the boiling temperature, while maintaining reflux. The reaction mixture was stirred for 0.5 hour, then allowed to cool. It was poured into ice water and 15% sodium hydroxide solution was added to adjust to pH=10. The aqueous layer was extracted three times with diethyl ether and the combined organic layers were washed with water, dried over anhydrous sodium sulfate, filtered and evaporated to dryness. The residue was purified by chromatography on silica gel (eluent: diethyl ether) to afford 11.1 g of the oily title compound, which had $[\alpha]_D^{22}$ = -103.7° (c=1.9, $CHCl_3$). Yield: 97%.
$^1$H NMR ($CDCl_3$, ppm) delta = 1.211 (d, 3H), 2.626 and 2.889 (2x dd, 2H), 3.147 (br.s, 1H), 3.703 (m, 1H), 4.474 (br.s, 2H), 6.451 (m, 1H), 6.961 (m, 1H).

## Example 42

### (S)-7,8-Difluoro-2,3-dihydro-4-mesyl-3-methyl-[1.4]benzothiazine

A solution of 11.5 g of methanesulfonyl chloride in 50 ml of toluene was added slowly to a stirred solution of 10.8 g of (R)-3,4-difluoro-2-(2-hydroxypropylthio)aniline and 17.3 ml of triethylamine in 190 ml of toluene while maintaining the temperature at 5°C. The reaction mixture was stirred for 0.5 hour. Next, 25.0 g of powdered potassium hydroxide and 0.625 g of tetrabutylammonium hydrogen sulfate were added. After stirring for 0.5 hour the temperature was raised to 35°C. Stirring was continued for 3 hours, after which

water was added and the organic layer was separated, washed with water, dried over anhydrous sodium sulfate, filtered and evaporated to dryness. The residue was purified by chromatography on silica gel (eluent: diethyl ether/hexane: 1/1). 7.68 G of title product were obtained. M.p.: 99-101 °C. This product had $[\alpha]_D^{20}$ = +55.5° (c=1.1, $CHCl_3$).
$^1H$ NMR ($CDCl_3$, ppm) delta = 1.25 (d, 3H), 2.85 (s, 3H), 2.82-2.88 and 3.37 (m, dd, 2H), 4.85 (m, 1H), 6.96 (m, 1H), 7.36 (m, 1H).

## Example 43

### (S)-7,8-Difluoro-3,4-dihydro-3-methyl-2H-[1,4]benzothiazine

A mixture of 6.9 g of (S)-7,8-difluoro-2,3-dihydro-4-mesyl-3-methyl-[1,4]benzothiazine, 8.3 g of phenol and 85 ml of 47% hydrobromic acid was heated under reflux for 16 hours. The reaction mixture was poured onto 800 g of crushed ice. The solution was made alkaline by adding diluted sodium hydroxide and then extracted three times with diethyl ether. The combined ethereal layers were washed with water, dried over anhydrous sodium sulfate, filtered and evaporated to dryness. 5.5 G of crude title product were obtained, which was purified by chromatography on silica gel (eluent: diethyl ether/hexane: 1/1) to yield 2.8 g of pure title product and 1.8 g of slightly impure material. The product had $[\alpha]_D^{20}$ = -64.0° (c=2.5, $CHCl_3$).
$^1H$ NMR ($CDCl_3$, ppm) delta = 1.29 (d, 3H), 2.73 and 2.89 (2x dd, 2H), 3.59 (m, 1H), 3.82 (br.s, 1H), 6.14 (m, 1H), 6.67 (m, 1H).

## Example 44

### Ethyl (S)-9,10-difluoro-2,3-dihydro-3-methyl-7-oxo-7H-pyrido[1,2,3-de][1,4]benzothiazine-6-carboxylate

A mixture of 2.01 g of (S)-7,8-difluoro-3,4-dihydro-3-methyl-2H-[1,4]benzothiazine and 2.16 g of diethyl ethoxymethylenemalonate was stirred under a nitrogen atmosphere and heated at 140-145 °C for 3 hours. After cooling, 15 g of ethyl polyphosphate were added and the reaction mixture was heated at 140-145 °C for another 2 hours. Next the mixture was cooled, poured into ice water and extracted three times with chloroform. The combined chloroform layers were washed with 5% sodium carbonate solution and water, dried over anhydrous sodium sulfate, filtered and evaporated to dryness. Diethyl ether was added to the residue and the crystalline material was filtered, washed and dried to afford 2.3 g of title product. Yield: 70.6%.
This compound had $[\alpha]_D^{22}$ = -41.8° (c=0.2, $CHCl_3$).
$^1H$ NMR ($CF_3COOD$, ppm) delta = 1.23 (tr, 3H), 1.50 (d, 3H), 3.10 and 3.39 (2x d, 2H), 4.39 (m, 2H), 5.14 (m, 1H), 7.97 (dd, 1H), 9.03 (s, 1H).

## Example 45

### (S)-9,10-Difluoro-2,3-dihydro-3-methyl-7-oxo-7H-pyrido[1,2,3-de][1,4]benzothiazine-6-carboxylic acid

A mixture of 2.0 g of ethyl (S)-9,10-difluoro-2,3-dihydro-3-methyl-7-oxo-7H-pyrido[1,2,3-de][1,4]-benzothiazine-6-carboxylate, 20 ml of acetic acid and 5 ml of conc. hydrochloric acid was heated at reflux temperature for 3 hours. After cooling to 5 °C the crystals were collected by filtration and washed successively with water, ethanol and diethyl ether. After drying 1.65 g of title product were obtained. Yield: 91.8%. The product had $[\alpha]_D^{22}$ = -14.1° (c=0.25, $CHCl_3$). M.p.: > 300 °C.
$^1H$ NMR ($CF_3COOD$, ppm) delta = 1.52 (d, 3H), 3.10 and 3.40 (2x d, 2H), 5.16 (m, 1H), 7.98 (dd, 1H), 9.12 (s, 1H).

## Example 46

(S)-9-Fluoro-2,3-dihydro-3-methyl-10-(4-methyl-1-piperazinyl)-7-oxo-7H-pyrido[1,2,3-de][1,4]benzothiazine-6-carboxylic acid

1.49 G of (S)-9,10-difluoro-2,3-dihydro-3-methyl-7-oxo-7H-pyrido[1,2,3-de][1,4]benzothiazine-6-carboxylic acid were transformed into the corresponding chelate and next treated with N-methylpiperazine for 24 hours as described in example 8.
The reaction residue was heated to reflux temperature in 40 ml of methanol and 1.42 ml of triethylamine for 5 hours. The mixture was cooled to room temperature and filtered to isolate 0.32 g of starting material. The filtrate was concentrated under reduced pressure and 40 ml of 4N hydrochloric acid were added. The residue dissolved partly and filtration afforded another 0.35 g of starting material. The filtrate was washed three times with chloroform and the aqueous layer was adjusted to pH = 11 with 4N sodium hydroxide solution and then to pH = 7.3 with 1N hydrochloric acid. The solution was extracted three times with chloroform, the combined organic layers were dried over anhydrous sodium sulfate, filtered and concentrated to dryness. The residue was recrystallized from isopropanol to afford 0.4 g of title compound. Yield: 21.3%. M.p.: 260-262 °C (dec.). The product had $[\alpha]_D^{20}$ = -77.9° (c=0.2, $CHCl_3$).
$^1H$ NMR ($CDCl_3$, ppm) delta = 1.59 (d, 3H), 2.40 (s, 3H), 3.06 and 3.44 (2x d, 2H), 2.73-3.53 (br., 8H), 4.84 (m, 1H), 7.89 (d, 1H), 8.72 (s, 1H).

## Example 47

(S)-3,4-Difluoro-2-(2-hydroxypropylthio)nitrobenzene

11.46 G of 3,4-difluoro-2-mercaptonitrobenzene were treated with 4.94 g of (S)-1,2-propylene oxide in the presence of 8.51 g of N-ethyldiisopropylamine as described in example 40. Also the workup was similar. 13.2 G of title product were obtained. Yield: 84.4%. The product had $[\alpha]_D^{22}$ = +35.8 (c=2.0, $CHCl_3$).
$^1H$ NMR ($CDCl_3$, ppm) delta = 1.26 (d, 3H), 2.64 (br., 1H), 2.99 and 3.18 (2x dd, 2H), 3.87 (m, 1H), 7.26 (m, 1H), 7.66 (m, 1H).

## Example 48

(S)-3,4-Difluoro-2-(2-hydroxypropylthio)aniline

13.0 G of (S)-3,4-difluoro-2-(2-hydroxypropylthio)nitrobenzene were reduced with 60 g of stannous chloride dihydrate as described in example 41.
After a similar workup 9.8 g of oily title compound were obtained. Yield: 86%. The product had $[\alpha]_D^{25}$ = +101.8 (c=1.2, $CHCl_3$).
$^1H$ NMR ($CDCl_3$, ppm) delta = 1.21 (d, 3H), 2.63 and 2.89 (2x dd, 2H), 3.71 (m, 1H), 4.18 (br., $NH_2$ + OH), 6.45 (m, 1H), 6.96 (m, 1H).

## Example 49

(R)-7,8-Difluoro-2,3-dihydro-4-mesyl-3-methyl-[1,4]benzothiazine

Both mesylation and ring closure of 9.5 g of (S)-3,4-difluoro-2-(2-hydroxypropylthio)aniline were carried out according to the procedures described in example 42. Also the workup was similar.

7.0 G of solid title product were obtained. Yield: 57.6%. M.p. 105-107° C. The product had $[\alpha]_D^{20}$ = -68.4° (c=0.9, $CHCl_3$).
$^1H$ NMR ($CDCl_3$, ppm) delta = 1.25 (d, 3H), 2.85 (s, 3H), 2.85 and 3.36 (2x dd, 2H), 4.86 (m, 1H), 6.96 (m, 1H), 7.36 (m, 1H).

Example 50

(R)-7,8-Difluoro-3,4-dihydro-3-methyl-2H-[1,4]benzothiazine

Demesylation of 6.9 g of (R)-7,8-difluoro-2,3-dihydro-4-mesyl-3-methyl-[1,4]benzothiazine with phenol and hydrobromic acid was carried out as described in example 43. Also the workup was similar.
5.0 G of the oily title product were obtained. Yield: 100%. The product had $[\alpha]_D^{20}$ = +65.4° (c=2.0, $CHCl_3$).
The compound proved to be optically pure, as demonstrated by NMR.
$^1H$ NMR ($CDCl_3$, ppm) delta = 1.31 (d, 3H), 2.75 and 2.91 (dABq, 2H), 3.61 (m, 1H), 3.78 (br.s, 1H), 6.15 (m, 1H), 6.69 (m, 1H).

Example 51

Ethyl (R)-9,10-difluoro-2,3-dihydro-3-methyl-7-oxo-7H-pyrido[1,2,3-de][1,4]benzothiazine-6-carboxylate

A mixture of 4.7 g of (R)-7,8-difluoro-3,4-dihydro-3-methyl-2H-[1,4]benzothiazine and 5.4 g of diethyl ethoxymethylenemalonate was stirred under a nitrogen atmosphere and heated at 140-150° C for 8 hours. After cooling 37.5 g of ethyl polyphosphate were added and the reaction mixture was heated at 140-145° C for 4 hours. The workup was identical to that described in example 44. In this way, 5.1 g of title product were obtained with $[\alpha]_D^{22}$ = +44.8° (c=0.2, $CHCl_3$). Yield: 67.1%.
$^1H$ NMR ($CF_3COOD$, ppm) delta = 1.29 (tr, 3H), 1.55 (d, 3H), 3.15 and 3.45 (dABq, 2H), 4.47 (m, 2H), 5.20 (m, 1H), 8.06 (tr, 1H), 9.09 (s, 1H).

Example 52

(R)-9,10-Difluoro-2,3-dihydro-3-methyl-7-oxo-7H-pyrido[1,2,3-de][1,4]benzothiazine-6-carboxylic acid

5.0 GC of ethyl (R)-9,10-difluoro-2,3-dihydro-3-methyl-7-oxo-7H-pyrido[1,2,3-de][1,4]benzothiazine-6-carboxylate were treated with 50 ml of acetic acid and 12.5 ml of conc. hydrochloric acid as described in example 45. Also an identical workup was carried out to afford 3.95 g of solid title product. Yield: 86.4%. M.p. 278° C (dec).
The product had $[\alpha]_D^{22}$ = +14.6° (c=0.27, $CHCl_3$).
$^1H$ NMR ($CF_3COOD$, ppm) delta = 1.54 (d, 3H), 3.11 and 3.42 (dABq, 2H), 5.17 (m, 1H), 8.00 (tr, 1H), 9.14 (s, 1H).

Example 53

(R)-9-Fluoro-2,3-dihydro-3-methyl-10-(4-methyl-1-piperazinyl)-7-oxo-7H-pyrido[1,2,3-de][1,4]benzothiazine-6-carboxylic acid

3.78 G of (R)-9,10-difluor-2,3-dihydro-3-methyl-7-oxo-7H-pyrido[1,2,3-de][1,4]benzothiazine-6-carboxylic acid were transformed into the corresponding chelate and then treated with N-methylpiperazine for 48 hours, as described in examples 8 and 46. The reaction residue was dissolved in 100 ml of 95% methanol and 6.5 ml of triethylamine and refluxed for 3 hours. The mixture was cooled to room temperature and 100 ml of 4N hydrochloric acid and 100 ml of chloroform were added. The undissolved material was filtered off and recrystallized from ethanol to afford 1.2 g of the title product. M.p. 257-259 °C. The product had $[\alpha]_D^{20}$ = +83.7 (c=0.2, $CHCl_3$).

The aqueous layer was separated from the filtrate, washed twice with chloroform and adjusted to pH=11 with 4N sodium hydroxide solution, then to pH=7.3 with 1N hydrochloric acid. The aqueous fraction was extracted three times with 100 ml portions of chloroform and the combined organic layers were dried over anhydrous sodium sulfate, filtered and evaporated to dryness. The residue was recrystallized from ethanol to afford 1.6 g of title compound, which had $[\alpha]_D^{20}$ = +83.3° (c=0.2, $CHCl_3$). M.p. 262-265 °C.

Both fractions of title compound were almost pure as confirmed by NMR. Total yield: 56.0%.

$^1H$ NMR ($CDCl_3$, some drops of DMSO ($D_6$), ppm) delta = 1.58 (d, 3H), 2.42 (s, 3H), 2.42, 2.88, 3.09 and 3.44 (4x br.s, 8H), 3.07 and 3.44 (dABq, 2H), 4.91 (m, 1H), 7.88 (d, 1H), 8.79 (s, 1H).

## Example 54

(S)-3-Chloro-4-fluoro-2-(2-hydroxypropylthio)nitrobenzene

12.0 G of 2-chloro-3-fluoro-6-nitrothiofenol were dissolved in 250 ml of abs. ethanol. After adding 11.2 ml of ethyldiisopropylamine, the solution was stirred and the temperature was kept at 20 °C. Next, 3.82 g of (S)-propylene oxide were added. The crystalline thiophenolate, formed immediately after addition of the base, dissolved within some hours. The reaction mixture was stirred overnight and then concentrated under reduced pressure. The residue was dissolved in diethyl ether, washed with water, 0.1N sodium hydroxide solution, again with water and saturated sodium chloride solution, dried over anhydrous sodium sulfate, filtered and evaporated to dryness to afford 14.71 g of crude material. The product was purified by chromatography on silica gel (eluent: petroleum ether (40-60)/diethyl ether: 1/1).

13.71 G of the title product were obtained. Yield: 89%. The compound had $[\alpha]_D^{20}$ = +67.8° (c=7.7, $CHCl_3$).

$^1H$ NMR ($CDCl_3$, ppm) delta = 1.24 (d, 3H), 2.93 and 3.14 (2x dd, 2H), 3.86 (m, 1H), 7.29 (dd, 1H), 7.59 (dd, 1H).

## Example 55

(S)-3-Chloro-4-fluoro-2-(2-hydroxypropylthio)aniline

28.2 G of stannous chloride were added to a stirred solution of 6.94 g of (S)-3-chloro-4-fluoro-2-(2-hydroxypropylthio)nitrobenzene in 50 ml of abs. ethanol. Shortly after that the temperature rose to 50 °C. The reaction mixture was stirred for 0.5 hour, another 1.5 g of stannous chloride were added and the mixture was heated under reflux for 1 hour. The reaction mixture was cooled, poured onto 250 g of crushed ice and adjusted to pH=10 by adding 2N of potassium hydroxide solution. The aqueous layer was extracted three times with diethyl ether. The combined ethereal layers were washed with water and saturated sodium chloride solution, dried over anhydrous sodium sulfate, filtered and evaporated to dryness to afford 5.76 g of title product. Yield: 93%. The product had $[\alpha]_D^{20}$ = -53.6° (c=1.0, $CHCl_3$).

1H NMR ($CDCl_3$, ppm) delta = 1.21 (d, 3H), 2.63 and 2.96 (2x dd, 2H), 3.20 (br.s, 1H), 3.68 (m, 1H), 4.51 (br.s, 2H), 6.62 (dd, 1H), 6.96 (dd, 1H).

## Example 56

(R)-8-Chloro-7-fluoro-2,3-dihydro-4-mesyl-3-methyl-[1,4]benzothiazine

A solution of 4.7 g of (S)-3-chloro-4-fluoro-2-(2-hydroxypropylthio)nitrobenzene and 6.9 ml of triethylamine in 75 ml of toluene was stirred and cooled to 5° C. A solution of 4.6 g of methanesulfonyl chloride in 20 ml of toluene was added slowly and the mixture was stirred for 0.5 hour. An additional amount of mesylation reagent did not influence the product composition. Next, 10 g of powdered potassium hydroxide and 0.25 g of tetrabutylammonium hydrogen sulfate were added and the reaction mixture was stirred overnight. 100 Ml of toluene and 50 ml of water were added and the organic layer was separated, washed with water and evaporated to dryness to afford 6.32 g of crude product, which was purified by chromatography on silica gel (eluent: petroleum ether (40-60)/diethyl ether: 1/1). 5.12 G of title product were isolated. Yield: 86%. The product had $[\alpha]_D^{20}$ = -53.6° (c=1.0, $CHCl_3$).
$^1H$ NMR ($CDCl_3$, ppm) delta = 1.23 (d, 3H), 2.84 (s, 3H), 2.90 and 3.43 (2x dd, 2H), 4.84 (m, 1H), 6.96 (dd, 1H), 7.49 (dd, 1H).

## Example 57

(R)-8-Chloro-7-fluoro-3,4-dihydro-3-methyl-2H-[1,4]benzothiazine

4.47 G of (R)-8-chloro-7-fluoro-2,3-dihydro-4-mesyl-3-methyl-[1,4]benzothiazine and 5.0 g of phenol were dissolved in 50 ml of 48% hydrobromic acid. The mixture was heated under reflux for 170 minutes. Next, the reaction mixture was cooled and poured onto 500 g of crushed ice. A solution of 20 g of sodium hydroxide in 100 ml of water was added and the aqueous layer was extracted three times with diethyl ether. The combined organic layers were washed with 2N sodium hydroxide solution, saturated sodium chloride solution, dried over anhydrous sodium sulfate, filtered and evaporated to dryness to afford 3.67 g of crude product. It was purified by chromatography on silica gel (eluent: petroleum ether (40-60)/diethyl ether: 1/1). 3.13 G of title product were obtained, which had $[\alpha]_D^{20}$ = +61.2° (c=2.1, $CHCl_3$). Yield: 95%.
On NMR analysis the optical purity proved to be more than 99%.
$^1H$ NMR ($CDCl_3$, ppm) delta = 1.28 (d, 3H), 2.78 and 2.93 (2x dd, 2H), 3.56 (m, 1H), 3.79 (br.s, 1H), 6.29 (dd, 1H), 6.68 (dd, 1H).

## Example 58

Ethyl (R)-10-chloro-9-fluoro-2,3-dihydro-3-methyl-7-oxo-7H-pyrido[1,2,3-de][1,4]benzothiazine-6-carboxylate

A mixture of 4.35 g of (R)-8-chloro-7-fluoro-3,4-dihydro-3-methyl-2H-[1,4]benzothiazine and 4.42 g of diethyl ethoxymethylenemalonate was stirred under a nitrogen atmosphere and heated at 140-145° C for 7 hours.
After the mixture had been allowed to cool overnight, 32 g of ethyl polyphosphate were added and the reaction mixture was heated at 140-145° C for 6 hours.
The workup was identical to that described in example 44, resulting in 5.98 g of title compound. Yield: 88%. The compound had $[\alpha]_D^{20}$ = +53.0° (c=0.17, $CHCl_3$).
$^1H$ NMR ($CDCl_3$, acetone (D6), ppm) delta = 1.29 (tr, 3H), 1.56 (d, 3H), 3.24 and 3.55 (dABq, 2H), 4.47 (m, 2H), 5.25 (m, 1H), 7.99 (d, 1H), 9.17 (s, 1H).

## Example 59

(R)-10-Chloro-9-fluoro-2,3-dihydro-3-methyl-7-oxo-7H-pyrido[1,2,3-de][1,4]benzothiazine-6-carboxylic acid

5.86 G of ethyl (R)-10-chloro-9-fluoro-2,3-dihydro-3-methyl-7-oxo-7H-pyrido[1,2,3-de][1,4]benzothiazine-6-carboxylate were treated with 55 ml of acetic acid and 15 ml of conc. hydrochloric acid as described in example 45. Also an identical workup was carried out to afford 4.62 g of title compound, which had $[\alpha]_D^{23}$ = +25.3° (c=0.2, $CHCl_3$).
$^1H$ NMR ($CF_3COOD$, ppm) delta = 1.49 (d, 3H), 3.13 and 3.46 (2x dd, 2H), 5.15 (m, 1H), 7.89 (d, 1H), 9.11 (s, 1H).

## Example 60

(R)-10-Chloro-9-fluoro-2,3-dihydro-3-methyl-7-oxo-7H-pyrido[1,2,3-de][1,4]benzothiazine-6-carboxylic acid 1-oxide

1.50 G of (R)-10-chloro-9-fluoro-2,3-dihydro-3-methyl-7-oxo-7H-pyrido[1,2,3-de][1,4]benzothiazine-6-carboxylic acid were dissolved in 150 ml of acetic acid and heated to 100° C, while stirring. Then, 6 ml of 30% hydrogen peroxide in 30 ml of acetic acid were added slowly. The temperature rose to 110° C and stirring was continued for 3 hours. The reaction mass was cooled to 15° C and the precipitated solid was filtered off, washed with diethyl ether and dried to afford 0.31 g of a by-product. IR analysis indicated oxidation to the sulfon (KBr: 1331, 1142 $cm^{-1}$). Yield: 19%.
The filtrate was concentrated under reduced pressure. The residue was treated with diethyl ether, filtered off and washed with water, ethanol and diethyl ether and dried under reduced pressure to afford 1.08 g of title product. Yield: 69% (uncorr.).
NMR analysis provided much evidence for the structure of the title product, the purity of which, however, left much to be desired.
$^1H$ NMR ($CF_3COOD$, acetone (D6), ppm) delta = 1.7 (m, 3H), 3.2 and 3.5 (2x m, 2H), 5.0 (m, 1H), 8.1 (d, 1H), 9.0 (s, 1H).

## Example 61

(R)-9-Fluoro-2,3-dihydro-3-methyl-10-(4-methyl-1-piperazinyl)-7-oxo-7H-pyrido[1,2,3-de][1,4]benzothiazine-6-carboxylic acid 1-oxide

0.85 G of crude (R)-10-chloro-9-fluoro-2,3-dihydro-3-methyl-7-oxo-7H-pyrido[1,2,3-de][1,4]benzothiazine-6-carboxylic acid 1-oxide was suspended in 35 ml of dimethyl sulfoxide while stirring and 2.0 g of N-methylpiperazine were added. The reaction mass was heated at 140° C for 1 hour. Then, the reaction mixture was cooled and the solvent was distilled off. The residue was mixed with methanol, filtered and the filtrate was concentrated under reduced pressure to afford 2.15 g of crude material. Crystallization of the mass from ethanol failed, and the solution was concentrated under vacuum. Next, the residue was stirred with 25 ml of ethanol and filtered to yield 0.3 g of solid material, consisting of two products as shown by TLC analysis on silica gel (eluent: n-butyl alcohol/acetic acid/water: 2/1/1).
The mixture was used as such in example 62.

## Example 62

(R)-9-Fluoro-2,3-dihydro-3-methyl-10-(4-methyl-1-piperazinyl)-7-oxo-7H-pyrido[1,2,3-de][1,4]benzothiazine-6-carboxylic acid

0.3 G of crude material, prepared as described in example 61, was dissolved in 30 ml of dry dimethylformamide and 0.35 ml of phosphorus tribromide was added at 0-5° C. The reaction mixture was stirred for 0.5 hour at low temperature. Water was added to the reaction mass, which was evaporated to dryness. Toluene was added to the residue, and distilled off. The procedure was repeated once, then the

residue was dissolved in 20 ml of water. The aqueous layer was extracted with chloroform and adjusted to pH=7 with 2N sodium hydroxide solution, then extracted twice with chloroform. The combined organic layers were concentrated under reduced pressure to afford 0.15 g of solid product, which, on TLC analysis on silica gel (eluent: n-butyl alcohol/acetic acid/water: 2/1/1), was found to have the same $R_f$ value as the product prepared in example 53. M.p. 255-257 °C. The compound had $[\alpha]_D^{20}$ = +78.7° (c=0.5, $CHCl_3$).
$^1H$ NMR ($CDCl_3$, ppm) delta = 1.60 (d, 3H), 2.40 (s, 3H), 2.40, 2.87, 3.06 and 3.43 (4x br.s, 8H), 3.06 and 3.46 (2x dd, 2H), 4.87 (m, 1H), 7.87 (d, 1H), 8.74 (s, 1H).

## Example 63

(R)-3,4-Difluoro-2-(1-hydroxyisopropylthio)nitrobenzene

4.17 G of (R)-2-mercapto-1-propanol and 7.49 g of 2,3,4-trifluoronitrobenzene were dissolved in 75 ml of dry toluene and added slowly to a stirred suspension of 4.39 g of powdered potassium hydroxide in 75 ml of toluene, cooled at -10 °C. The mixture was stirred at this low temperature for 1 hour. TLC analysis (silica gel, diethyl ether/hexane: 1/1) showed half of the starting material to have been converted into two products. After the reaction mixture had been left standing at room temperature for a weekend, water was added and the toluene layer was separated, washed with water and evaporated under reduced pressure to afford 7.65 g of crude mass, which was purified by chromatography on silica gel (eluent: diethyl ether/hexane: 1/1) to provide 2.37 g of title product, which was impure. Yield: 21% (uncorr.).
In addition, 3.80 g of (R)-1-(2,3-difluoro-6-nitrophenoxy)-2-(2,3-difluoro-6-nitrophenylthio)propane were isolated. Yield: 44.3%, based on 2,3,4-trifluoronitrobenzene.
$^1H$ NMR ($CDCl_3$, ppm) delta = 1.34 (d, 3H), 3.61 (m, 3H), 7.28 (m, 1H), 7.62 (m, 1H).

## Example 64

(S)-3,4-Difluoro-2-(1-hydroxyisopropylthio)nitrobenzene

4.88 G of (S)-2-mercapto-1-propanol were dissolved and 3.51 g of powdered potassium hydroxide were suspended in 75 ml of toluene, while stirring. The mixture was cooled to -15 °C and 8.85 g of 2,3,4-trifluoronitrobenzene, dissolved in 75 ml of toluene, were added slowly. The reaction mixture was stirred at -15 °C for 2 hours. Then, the cooling bath was removed and the reaction mixture was extracted twice with water. The organic layer was dried over anhydrous sodium sulfate, filtered and evaporated to dryness to afford 11.55 g of crude residue. It was purified by chromatography on silica gel (eluent: petroleum ether (40-60)/diethyl ether: 1/1). 5.06 G of starting material were recovered. Yield: 57%. In addition, 3.43 g of the title product were obtained. Yield: 64.5% (corr.). The product had $[\alpha]_D^{20}$ = +39.2° (C=1.0, $CHCl_3$).
$^1H$ NMR ($CDCl_3$, ppm) delta = 1.33 (d, 3H), 3.63 (m, 3H), 7.28 (m, 1H), 7.61 (m, 1H).

## Example 65

(S)-3,4-Difluoro-2-(1-hydroxyisopropylthio)aniline

15 G of stannous chloride dihydrate were added to a stirred solution of 3.0 g of (S)-3,4-difluoro-2-(1-hydroxyisopropylthio)nitrobenzene in 20 ml of abs. ethanol. The reaction mixture was stirred for 1 hour, during which period its temperature rose to 62 °C. The workup was identical to that described in example 55.
1.93 G of oily title product were obtained, which had $[\alpha]_D^{20}$ = +11.2° (c=0.86, $CHCl_3$).
$^1H$ NMR ($CDCl_3$, ppm) delta = 1.65 (d, 3H), 3.2 (m, 1H), 3.5 (m, 2H), 6.48 (m, 1H), 6.99 (m, 1H).

Example 66

(S)-7,8-Difluoro-2,3-dihydro-4-mesyl-2-methyl-[1,4]benzothiazine

Both mesylation and ring closure of 1.8 g of (S)-3,4-difluoro-2-(1-hydroxyisopropylthio)aniline were carried out according to the procedures described in example 42. Also the workup was identical. 1.5 G of solid title compound were obtained. Yield: 65.1%. The compound had $[\alpha]_D^{20}$ = -114.3° (c=1.01, $CHCl_3$).
$^1H$ NMR ($CDCl_3$, ppm) delta = 1.42 (d, 3H), 2.90 (s, 3H), 3.00 and 4.48 (2x dd, 2H), 3.40 (m, 1H), 6.91 (m, 1H), 7.37 (m, 1H).

Example 67

(S)-7,8-Difluoro-3,4-dihydro-2-methyl-2H-[1,4]benzothiazine

A mixture of 1.5 g of (S)-7,8-difluoro-2,3-dihydro-4-mesyl-2-methyl-[1,4]benzothiazine, 1.8 g of phenol and 18.5 ml of 48% hydrobromic acid was heated under reflux for 8 hours. The reaction mixture was cooled and poured into 150 ml of water. The solution was made alkaline with diluted sodium hydroxide solution. and was extracted three times with diethyl ether. The combined ethereal layers were washed with water, dried over anhydrous sodium sulfate, filtered and evaporated under reduced pressure to afford 1.4 g of title compound, which had $[\alpha]_D^{20}$ = -55.2° (c=1.9, $CHCl_3$). Yield: 93%.
$^1H$ NMR ($CDCl_3$, ppm) delta = 1.38 (d, 3H), 3.18 and 3.55 (2x dd, 2H), 3.32 (m, 1H), 6.18 (m, 1H), 6.69 (m, 1H).

Example 68

(R)-3,4-Difluoro-2-(2,3-isopropylidenedioxypropoxy)nitrobenzene

A solution of 7.0 g of (R)-2,3-isopropylidenedioxy-1-propanol and 8.85 g of 2,3,4-trifluoronitrobenzene in 75 ml of toluene was added slowly to a stirred suspension of 4.3 g of powdered potassium hydroxide in 75 ml of toluene, while maintaining the temperature at -10° C. Next, the reaction mixture was stirred at this low temperature for 0.5 hour. Water was added to the reaction mixture and the organic layer was extracted, separated, washed with water, dried over anhydrous sodium sulfate, filtered and evaporated to dryness. 16.0 G of an oily residue were obtained, containing 10 mol% of toluene. Yield: 100%.
$^1H$ NMR ($CDCl_3$, ppm) delta = 1.37 (s, 3H), 1.42 (s, 3H), 3.96 and 4.16 (2x dd, 2H), 4.23 and 4.34 (2x dd, 2H), 4.50 (m, 1H), 7.02 (m, 1H), 7.68 (m, 1H).
The compound was dried carefully under reduced pressure, after which it had $[\alpha]_D^{20}$ = +8.54° (c=2.6, $CHCl_3$).

Example 69

(S)-2-(2,3-Dihydroxypropoxy)-3,4-difluoronitrobenzene

3.5 G of Dowex 50W-X8-400 $H^+$ (RTM) ion-exchange resin were suspended in a stirred solution of 15.0 g of (R)-3,4-difluoro-2-(2,3-isopropylidenedioxypropoxy)nitrobenzene, of 90% quality, in 150 ml of methanol. The mixture was stirred overnight, then filtered to remove the ion-exchange resin. The filtrate was concentrated under reduced pressure. The crude residue was purified by chromatography on silica gel (eluent: diethyl ether) to afford 10.2 g of oily title compound, containing traces of diethyl ether. Yield: 88%.

The product had $[\alpha]_D^{20}$ = -15.0° (c=0.7, $CHCl_3$).
$^1H$ NMR ($CDCl_3$, ppm) delta = 3.00 (br.s, 1H), 3.57 (br.s, 1H), 3.73 and 3.83 (2x dd, 2H), 4.13 (m, 1H), 4.34 and 4.43 (2x m, 2H), 7.02 (m, 1H), 7.71 (m, 1H).

Example 70

(R)-3,4-Difluoro-2-(2,3-isopropylidenedioxypropoxy)aniline

10.0 G of (R)-3,4-difluoro-2-(2,3-isopropylidenedioxypropoxy)nitrobenzene were dissolved in a mixture of 80 ml of abs. ethanol and 1.0 g of triethylamine. Next, 1.0 g of 10% Pd/C was added, the mixture was stirred vigorously and saturated with $H_2$ at 20-25°C. After absorption of the required amount of $H_2$, the mixture was filtered over Hyflo (RTM) and the filtrate was concentrated under reduced pressure. The residue was dissolved in diethyl ether and the ethereal layer was washed with water, dried over anhydrous sodium sulfate, filtered and evaporated to dryness to yield 6.65 g of the oily title compound, which was contaminated with 35 mol% of toluene.
$^1H$ NMR ($CDCl_3$, ppm) delta = 1.40 and 1.47 (2x s, 6H), 3.8-4.1 (m, 4H), 3.73 (br.s, 2H), 4.43 (m, 1H), 6.36 (m, 1H), 6.70 (m, 1H).
The synthesis was repeated according to the process described above. The title compound so obtained had $[\alpha]_D^{20}$ = +11.9° (c=2.4, $CHCl_3$).

Example 71

(R)-3,4-Difluoro-2-(2,3-isopropylidenedioxypropoxy)-N-tosylaniline

To a stirred solution of 2.43 g of (R)-3,4-difluoro-2-(2,3-isopropylidenedioxypropoxy)aniline in 10 ml of pyridine was added dropwise a solution of 1.9 of p-toluenesulfonyl chloride in 10 ml of pyridine. The reaction mixture was stirred overnight, then evaporated under reduced pressure and the residue was dissolved in toluene. In order to remove pyridine completely the solution was evaporated again. The residue was dissolved in diethyl ether. The ethereal solution was washed with water, dried over anhydrous sodium sulfate, filtered and evaporated to dryness to afford 4.0 g of crude product, which was purified by chromatography on silica gel (eluent: diethyl ether/hexane: 3/2). 3.6 G of title compound were obtained. Yield: 88%. It had $[\alpha]_D^{20}$ = +8.3° (c=0.8, $CHCl_3$).
$^1H$ NMR ($CDCl_3$, ppm) delta = 1.48 and 1.56 (2x s, 6H), 2.37 (s, 3H), 3.7-4.1 (m, 4H), 4.34 (m, 1H), 6.85 (m, 1H), 7.21 and 7.67 (ABq, 4H), 7.34 (m, 1H), 8.07 (s, 1H).

Example 72

(S)-3,4-Difluoro-2-(2,3-dihydroxypropoxy)-N-tosylaniline

0.6 G of Dowex 50W-X8-400 $H^+$ (RTM) ion-exchange resin was added to a stirred solution of 3.1 g of (R)-3,4-difluoro-2-(2,3-isopropylidenedioxypropoxy)-N-tosylaniline in 30 ml of methanol. The suspension was stirred for 18 hours. TLC analysis on silica gel (eluent: diethyl ether) showed some remaining starting material, but the addition of 0.6 g of fresh Dowex resin had no effect. The reaction mixture was filtered and the filtrate was evaporated to dryness. The residue was purified by chromatography on silica gel (eluent: diethyl ether) to afford 2.7 g of oily title compound. Yield: 96%. It had $[\alpha]_D^{20}$ = +142.8° (c=0.8, $CHCl_3$).
$^1H$ NMR ($CDCl_3$, ppm) delta = 2.33 (s, 3H), 3.7-4.0 (m, 4H), 4.05 (m, 1H), 6.77 (m, 1H), 7.21 (m, 1H), 7.20 and 7.68 (ABq, 4H).

Example 73

(R)-7,8-Difluoro-2,3-dihydro-3-hydroxymethyl-4-tosyl-[1,4]benzoxazine

A solution of 0.87 g of diethyl azodicarboxylate in 10 ml of tetrahydrofuran was added dropwise at room temperature to a stirred solution of 1.6 g of (S)-3,4-difluoro-2-(2,3-dihydroxypropoxy)-N-tosylaniline and 1.31 g of triphenylphosphine in 25 ml of dry tetrahydrofuran. After stirring for 2 hours TLC analysis on silica gel (eluent: diethyl ether) showed complete conversion of starting material. The reaction mixture was concentrated, diethyl ether was added to the residue and the solid triphenylphosphine oxide was filtered off. The filtrate was evaporated under reduced pressure and the residue was purified by chromatography on silica gel (eluent: diethyl ether/hexane: 9/1) to afford 1.4 g of title product, which proved to be optically pure (≧ 99%). The compound was contaminated with 16 mol % of diethyl ether and 14 mol % of diethyl azodicarboxylate.
$^1$H NMR ($CDCl_3$, ppm) delta = 2.40 (s, 3H), 2.66 (br.s, 1H), 3.15 and 4.28 (dd,d, 2H), 3.4-3.7 (m, 2H), 4.42 (m, 1H), 6.78 (m, 1H), 7.26 and 7.38 (ABq, 4H), 7.66 (m, 1H).

Example 74

(S)-7,8-Difluoro-3-fluoromethyl-2,3-dihydro-4-tosyl-[1,4]benzoxazine

1.8 G of (R)-7,8-difluoro-2,3-dihydro-3-hydroxymethyl-4-tosyl-[1,4]benzoxazine were dissolved in 10 ml of dichloromethane and the solution was added slowly to a stirred mixture of 1 ml of diethylaminosulfur trifluoride and 10 ml of dichloromethane, which had been cooled at -70° C. The mixture was stirred at the low temperature for 2 hours and at room temperature overnight. Ethanol was added and the mixture was stirred for 10 minutes to destroy the excess of diethylaminosulfur trifluoride. Water was added and the organic layer was separated, washed with water and evaporated under reduced pressure. The residue was purified by chromatography on silica gel (eluent: hexane/diethyl ether: 1/1) to afford 0.6 g of title product, which solidified on standing. It was slightly impure.
The title product had been prepared previously in the same solvent as described above, but first at room temperature and finally at 40° C. Only 0.2 g of impure title product was obtained from 2.0 g of starting material. The structure was confirmed by NMR.
$^1$H NMR ($CDCl_3$, ppm) delta = 2.34 (s, 3H), 3.23 (m, 1H), 4.3-4.6 (m, 3H), 4.63 (m, 1H), 6.81 (m, 1H), 7.30-7.50 (ABq, 4H), 7.68 (m, 1H).

Example 75

(S)-7,8-Difluoro-3-fluoromethyl-3,4-dihydro-2H-[1,4]benzoxazine

1.1 G of (S)-7,8-difluoro-3-fluoromethyl-2,3-dihydro-4-tosyl-[1,4]benzoxazine, 1.4 g of phenol and 15 ml of 48% hydrobromic acid were heated at 100° C for 3 hours. TLC analysis on silica gel (hexane/diethyl ether: 1/1) showed the formation of more than one product and the absence of starting material. The reaction mass was poured onto ice, and the solution was made alkaline by adding 4N sodium hydroxide solution. The aqueous layer was extracted three times with diethyl ether. The combined ethereal layers were dried over anhydrous sodium sulfate, filtered and evaporated to dryness. The residue was purified by chromatography on silica gel (eluent: diethyl ether/hexane: 1/1) to afford 0.15 g of oily title compound, which had $[\alpha]_D^{20}$ = +29.4° (c=1.1, $CHCl_3$).
$^1$H NMR ($CDCl_3$, ppm) delta = 3.76 (m, 1H), 4.03 (br.s, 1H), 4.21 (m, 2H), 4.46 (m, 2H), 6.31 (m, 1H), 6.59 (m, 1H).

Example 76

(R)-2-(3-Benzyloxy-2-hydroxypropoxy)-3,4-difluoronitrobenzene

3.5 G of 2,3-difluoro-6-nitrophenol, 3.88 g of ethyldiisopropylamine and 6.56 g of (R)-benzyl glycidyl ether were dissolved in 30 ml of ethanol, transferred to a Carius tube and heated at 120 °C for 4 hours. The reaction mixture was cooled and evaporated under reduced pressure. The residue was distributed between water and diethyl ether. The ethereal layer was separated, washed with water, diluted sodium hydroxide solution, saturated sodium chloride solution, dried over anhydrous sodium sulfate, filtered and evaporated to dryness. The residue, 9.82 g of a dark oil, was purified by chromatography on silica gel (eluent: hexane/diethyl ether: 1/1) to afford 4.07 g of a 4:1 mixture of the title compound and its regioisomer, the 3-benzyloxy-1-hydroxyisopropoxy derivative. Yield: 60%. The mixture was used as such in example 77.
$^1$H NMR ($CDCl_3$, ppm) delta = 3.64 (d, 2H), 4.18 (m, 1H), 4.33-4.43 (m, 2H), 4.56 (s, 2H), 6.96 (m, 1H), 7.32 (m, 5H), 7.67 (m, 1H).

Example 77

(R)-2-(3-Benzyloxy-2-hydroxypropoxy)-3,4-difluoroaniline

3.73 G of the mixture prepared in example 76 were dissolved under stirring in 25 ml of abs. ethanol. 12.41 G of stannous chloride dihydrate were added, thus increasing the reaction temperature to 50 °C. After stirring for 0.5 hour conversion was not completed. Another 2.5 g of stannous chloride dihydrate were added and the mixture was stirred at 40 °C for 1 hour. The reaction mass was poured onto 100 g of crushed ice. The precipitate was dissolved by adding 2N sodium hydroxide solution and the aqueous layer was extracted three times with diethyl ether. The combined ethereal layers were washed with water and saturated sodium chloride solution and dried over anhydrous sodium sulfate, filtered and evaporated to dryness to afford 3.3 g of crude material. It was purified by chromatography on silica gel (eluent: diethyl ether) to afford 1.37 g of title product, 0.28 g of its regioisomer (3-benzoyl-1-hydroxyisopropoxy-derivative) and 0.87 g of a mixed fraction, indicating that no full separation had occurred. Total yield: 2.57 g, 72%.
$^1$H NMR ($CDCl_3$, ppm) delta = 3.58 (m, 2H), 4.01-4.11 (m, 2H), 4.06 (m, 1H), 4.52 (s, 2H), 6.30 (m, 1H), 6.68 (m, 1H), 7.29 (m, 5H).
Regioisomer, delta = 3.8 (m, 4H), 4.20 (m, 1H), 4.56 (s, 2H), 6.34 (m, 1H), 6.70 (m, 1H), 7.31 (m, 5H).

Example 78

(R)-2-(3-Benzyloxy-2-tosyloxypropoxy)-3,4-difluoro-N-tosylaniline

0.84 G of the mixed fraction prepared in example 77 was dissolved in 15 ml of dry pyridine, while stirring. 1.08 G of p-toluenesulfonyl chloride in 10 ml of dry pyridine were added slowly at room temperature. The reaction mixture was stirred for 24 hours, then 0.28 g of p-toluenesulfonyl chloride and 0.07 g of N-methylimidazole were added and stirring was continued for another 26 hours. Finally, 0.36 g of p-toluenesulfonyl chloride was added. The reaction mixture was stirred for 22 hours, then pyridine was distilled off. The remainder was distilled off azeotropically by using toluene and heptane. The residue was mixed with water and extracted twice with diethyl ether. The ethereal layer was washed twice with diluted hydrochloric acid, once with water and once with saturated sodium chloride solution, dried over anhydrous sodium sulfate, filtered and evaporated to dryness. The residue (1.8 g) was purified by chromatography on silica gel (eluent: petroleum ether (40-60)/diethyl ether: 1/1) to afford 0.90 g of title product. Yield: 63%.

In a previous experiment 1.14 g of (R)-2-(3-benzyloxy-2-hydroxypropoxy)-3,4-difluoroaniline in 15 ml of dry pyridine were mixed with 1.47 g of p-toluenesulfonyl chloride dissolved in 10 ml of dry pyridine. The reaction mixture was stirred at room temperature for 1 hour. The workup including the chromatographic

purification was carried out as described above. 0.23 G of title compound was obtained.
$^1$H NMR ($CDCl_3$, ppm) delta = 2.32 (s, 3H), 2.44 (s, 3H), 3.55 (d, 2H), 3.9-4.1 (m, 2H), 4.51 (q, 2H), 4.88 (m, 1H), 6.81 (m, 1H), 7.1-7.8 (m, 14H).
In addition, 0.90 g of (R)-2-(3-benzyloxy-2-hydroxypropoxy)-3,4-difluoro-N-tosylaniline was isolated.
$^1$H NMR ($CDCl_3$, ppm) delta = 2.33 (s, 3H), 3.5 (m, 3H), 3.83-3.98 (m, 2H), 4.05 (m, 1H), 4.58 (s, 2H), 6.83 (m, 1H), 7.14 and 7.66 (ABq, 4H), 7.3 (m, 6H).

0.80 G of (R)-2-(3-benzyloxy-2-hydroxypropoxy)-3,4-difluoro-N-tosylaniline was tosylated according to the first procedure described above. 0.78 G of title product, as confirmed by NMR, was obtained, containing an unknown byproduct. In addition, 0.15 g of unreacted starting material was recovered.

## Example 79

(S)-3-Benzyloxymethyl-7,8-difluoro-2,3-dihydro-4-tosyl-[1,4]benzoxazine

1.45 G of slightly impure (R)-2-(3-benzyloxy-2-tosyloxypropoxy)-3,4-difluoro-N-tosylaniline were dissolved with stirring in 40 ml of dry toluene. To the solution were added successively 0.32 g of powdered potassium carbonate, 0.6 g of powdered potassium hydroxide and 0.04 g of tetrabutylammonium hydrogen sulfate. The suspension was stirred at room temperature for 2.5 hours. Then, water was added, the organic layer was separated, washed with water and evaporated to dryness to afford 1.33 g of crude material, which was purified by chromatography on silica gel (eluent: petroleum ether (40-60)/diethyl ether: 1/1) to give 0.51 g of pure title product and 0.64 g of a mixture, which was crystallized from hexane/tetrahydrofuran to provide 0.33 g of title compound. Total yield: 0.84 g (80%).
$^1$H NMR ($CDCl_3$, ppm) delta = 2.38 (s, 3H), 3.12 and 4.33 (2x dd, 2H), 3.35-3.55 (m, 2H), 4.50 (m, 3H), 6.74 (m, 1H), 7.26 (m, 5H), 7.29-7.46 (ABq, 4H), 7.61 (m, 1H).

## Example 80

(S)-7,8-Difluoro-2,3-dihydro-3-hydroxymethyl-4-tosyl-[1,4]-benzoxazine

0.61 G of (S)-3-benzyloxymethyl-7,8-difluoro-2,3-dihydro-4-tosyl-[1,4]benzoxazine was dissolved in 50 ml of abs. ethanol and 20 ml of toluene. 0.1 G of 10% Pd/C was added, the suspension was stirred vigorously, and then saturated with $H_2$. After the required amount of $H_2$ had been absorbed, the catalyst was filtered off over Hyflo (RTM) and the filtrate was evaporated under reduced pressure to afford 0.52 g of product, which on TLC analysis proved to be a 1:1 mixture of starting material and reduced product.
The reduction procedure was repeated, but no higher degree of conversion into reduced product was observed. The residue was then purified by chromatography on silica gel (eluent: diethyl ether/petroleum ether (40-60): 5/1) to afford 0.29 g of starting material, as evidenced by NMR. In addition, 0.23 g of title compound was obtained. Yield: 98.7% (corr.). The compound had $[\alpha]_D^{23}$ = -130.5° (c = 1.0, $CHCl_3$).
$^1$H NMR ($CDCl_3$, ppm) delta = 2.40 (s, 3H), 3.15 and 4.26 (2x dd, 2H), 3.51-3.66 (m, 2H), 4.42 (m, 1H), 6.78 (m, 1H), 7.27-7.48 (ABq, 4H), 7.67 (m, 1H).
The compound was identified as a 95:5 mixture of the title product and its optical isomer.

## Example 81

(S)-2-[1-(4,4′-Dimethoxytrityloxy)isopropoxy]-3,4-difluoronitrobenzene

A solution of 7.0 g of (S)-1-(4,4′-dimethoxytrityloxy)-2-propanol and 3.54 g of 2,3,4-trifluoronitrobenzene in 30 ml of dry toluene was added slowly to a stirred suspension of 2.8 g of powdered potassium hydroxide in 30 ml of toluene, cooled at -10° C. The cooling bath was removed and the mixture was stirred at room

temperature for 1 hour. TLC on silica gel indicated that no reaction had occurred. Next, 0.1 g of tetrabutylammonium hydrogen sulfate was added and the mixture was stirred for 19 hours. Water was added and the organic layer was separated, washed with water, and concentrated under reduced pressure. The crude residue was purified by chromatography on silica gel (eluent: diethyl ether/hexane: 2/3) to afford 5.7 g of pure, viscous title compound and an additional amount of 1.0 g, which was slightly impure.
$^1H$ NMR ($CDCl_3$, ppm) delta = 1.32 (d, 3H), 3.27 and 3.39 (dABq, 2H), 3.78 (s, 6H), 4.61 (m, 1H), 6.7-7.3 (m, 13H), 6.92 (m, 1H), 7.66 (m, 1H).

## Example 82

(S)-3,4-Difluoro-2-(1-hydroxyisopropoxy)nitrobenzene

5.5 G of (S)-2-[1-(4,4′-dimethoxytrityloxy)isopropoxy]-3,4-difluoronitrobenzene were dissolved in 100 ml of methanol while stirring. 3.5 G of Dowex 50W-X8-400 $H^+$ (RTM) ion-exchange resin were added and the suspension was stirred for 2 hours. The reaction mixture was filtered, and the filtrate was concentrated under reduced pressure. The residue was purified by chromatography on silica gel (eluent: diethyl ether/hexane: 1/1) to afford 2.0 g of title compound, which had $[\alpha]_D^{23}$ = -71.4° (c = 1.4, $CHCl_3$). Yield: 86%.
$^1H$ NMR ($CDCl_3$, ppm) delta = 1.40 (d, 3H), 2.79 (m, 2H), 4.71 (m, 1H), 6.99 (m, 1H), 7.67 (m, 1H).

## Example 83

(S)-3,4-Difluoro-2-(1-mesyloxyisopropoxy)nitrobenzene

A solution of 1.15 g methanesulfonyl chloride in 10 ml of dry diethyl ether was added slowly to a stirred mixture of 2.1 g of (S)-3,4-difluoro-2-(1-hydroxyisopropoxy)nitrobenzene and 1.5 g of triethylamine in 50 ml of dry diethyl ether, while maintaining the temperature at 15-20° C. The mixture was stirred for another 0.5 hour and water was added. The organic layer was separated, washed with water, dried over anhydrous sodium sulfate, filtered and concentrated under reduced pressure to afford 2.8 g of oily title compound, which had $[\alpha]_D^{23}$ = +57.1° (c = 1.3, $CHCl_3$). Yield: 100%.
$^1H$ NMR ($CDCl_3$, ppm) delta = 1.46 (d, 3H), 3.03 (s, 3H), 4.38 (m, 2H), 4.82 (m, 1H), 7.05 (m, 1H), 7.71 (m, 1H).

## Example 84

(S)-3,4-Difluoro-2-(1-mesyloxyisopropoxy)aniline

To 2.6 g of (S)-3,4-difluoro-2-(1-mesyloxyisopropoxy)nitrobenzene dissolved in 70 ml of abs. ethanol was added 0.3 g of 10% Pd/C. The mixture was stirred vigorously and then saturated with $H_2$ at 20-25° C. After the required amount of $H_2$ had been absorbed the reaction mixture was handled according to example 3. 1.8 G of oily title compound were obtained, which had $[\alpha]_D^{23}$ = +13.7° (c = 0.65, $CHCl_3$). Yield: 76.6%.
$^1H$ NMR ($CDCl_3$, ppm) delta = 1.39 (d, 3H), 3.06 (s, 3H), 4.36 (dABq, 2H), 4.56 (m, 1H), 6.39 (m, 1H), 6.73 (m, 1H).

## Example 85

(S)-7,8-Difluoro-3,4-dihydro-2-methyl-2H-[1,4]benzoxazine

1.8 G of (S)-3,4-difluoro-2-(1-mesyloxyisopropoxy)aniline, dissolved in 10 ml of toluene, were added to a stirred mixture of 1.7 g of powdered potassium hydroxide, 1.0 g of powdered potassium carbonate and 0.2 g of tetrabutylammonium hydrogen sulfate in 30 ml of toluene. The temperature rose to not more than 30 °C. The mixture was stirred overnight at room temperature, then water was added, the organic layer was separated, washed with water, dried over anhydrous sodium sulfate, filtered and evaporated to dryness. The crude residue was purified by chromatography on silica gel (eluent: toluene) to afford 0.2 g of oily title product, which had $[\alpha]_D^{23}$ = -40.0° (c=0.45, $CHCl_3$). Yield: 16.9%.

$^1H$ NMR ($CDCl_3$, ppm) delta = 1.42 (d, 3H), 3.08 and 3.35 (dABq, 2H), 3.7 (br.s, 1H), 4.26 (m, 1H), 6.26 (m, 1H), 6.53 (m, 1H).

## Example 86

### (R)-3-Chloro-4-fluoro-2-(1-hydroxyisopropoxy)nitrobenzene

A solution of 2.6 g of diethyl azodicarboxylate in 10 ml of dry tetrahydrofuran was added slowly to a stirred solution of 2.5 g of 2-chloro-3-fluoro-6-nitrophenol, 4.91 g of (S)-1-(4,4′-dimethoxytrityloxy)-2-propanol and 3.95 g of triphenylphosphine in 50 ml of dry tetrahydrofuran. Then, the mixture was stirred at room temperature for 18 hours. Next, it was concentrated under reduced pressure and the residue was treated with a 1:1 mixture of diethyl ether and hexane, and filtered. The filtrate was washed with 2N sodium hydroxide solution and with water, dried over anhydrous sodium sulfate, filtered and evaporated to dryness to afford 9.0 g of crude material. It was dissolved in 100 ml of methanol and stirred with 5 g of Dowex 50W-X8-400 $H^+$ (RTM) ion-exchange resin at room temperature for 2 hours. Next, the ion-exchange resin was filtered off, and the filtrate was evaporated under reduced pressure. The residue was purified by chromatography on silica gel (diethyl ether/hexane: 1/1) to afford 1.9 g of pure title product and 1.3 g of a slightly impure fraction. The pure product had $[\alpha]_D^{23}$ = +10.3° (c=1.0, $CHCl_3$).

$^1H$ NMR ($CDCl_3$, ppm) delta = 1.33 (d, 3H), 3.80 (m, 2H), 4.66 (m, 1H), 7.04 (dd, 1H), 7.81 (dd, 1H).

## Example 87

### (R)-3-Chloro-4-fluoro-2-(1-mesyloxyisopropoxy)nitrobenzene

3.0 G of (R)-3-chloro-4-fluoro-2-(1-hydroxyisopropoxy)nitro benzene were mesylated with 1.72 g of methanesulfonyl chloride and 2.8 ml of triethylamine in 70 ml of dry diethyl ether as described in example 83, and also the workup was identical. The residue was purified by chromatography on silica gel (eluent: diethyl ether/hexane: 1/1) to afford 2.6 g of the oily title product, which had $[\alpha]_D^{22}$ = -60.9° (c=1.0, $CHCl_3$). Yield: 66.0%.

$^1H$ NMR ($CDCl_3$, ppm) delta = 1.43 (d, 3H), 3.01 (s, 3H), 4.39 (m, 2H), 4.80 (m, 1H), 7.09 (dd, 1H), 7.86 (dd, 1H).

## Example 88

### (R)-3-Chloro-4-fluoro-2-(1-mesyloxyisopropoxy)aniline

2.5 G of (R)-3-chloro-4-fluoro-2-(1-mesyloxyisopropoxy)nitrobenzene were reduced with $H_2$ and 0.3 g of 10% Pd/C in 70 ml abs. ethanol as described in example 84, and also the workup was identical. 2.1 G of oily title product were isolated, which had $[\alpha]_D^{23}$ = +3.0° (c=1.0, $CHCl_3$). Yield: 91%.

$^1H$ NMR ($CDCl_3$, ppm) delta = 1.40 (d, 3H), 3.06 (s, 3H), 4.36 (m, 2H), 4.70 (m, 1H), 6.57 (dd, 1H), 6.78 (dd, 1H).

## Example 89

(R)-8-Chloro-7-fluoro-3,4-dihydro-2-methyl-2H-[1,4]benzoxazine

1.78 G of (R)-3-chloro-4-fluoro-2-(1-mesyloxyisopropoxy)aniline, dissolved in 10 ml of toluene, were added to a stirred mixture of 1.4 g of powdered potassium hydroxide, 0.8 g of powdered potassium carbonate and 0.1 g of ammonium hydrogen sulfate in 20 ml of toluene and cooled at 0 °C. The mixture was stirred at the low temperature for 4 hours and was left standing overnight at room temperature. Water was added, the organic layer was separated, washed with water and evaporated under reduced pressure. The residue was purified by chromatography on silica gel (eluent: diethyl ether/hexane: 3/2) to afford 1.25 g of the oily title product, which was slightly impure and had $[\alpha]_D^{23}$ = +38.4° (c=2.0, $CHCl_3$). Yield: 100%.
$^1H$ NMR ($CDCl_3$, ppm) delta = 1.42 (d, 3H), 3.06 and 3.34 (2x dd, 2H), 4.30 (m, 1H), 6.43 (m, 1H), 6.56 (m, 1H).

## Example 90

(S)-3-Chloro-4-fluoro-2-(2,3-isopropylidenedioxypropylthio)nitrobenzene

1.04 G of 2-chloro-3-fluoro-6-nitrothiophenol were mixed with 1.43 g of (S)-2,3-isopropylidenedioxy-1-tosyloxypropane and 3.0 g of powdered potassium carbonate in 25 ml of acetone and heated under reflux for 8 hours. The reaction mixture was concentrated under reduced pressure and the residue was distributed between diethyl ether and water. The ethereal layer was separated, washed with 2N sodium hydroxide solution and water, dried over anhydrous sodium sulfate, filtered and evaporated to dryness. The residue was purified by chromatography on silica gel (eluent: diethyl ether/hexane: 1/1) to afford 0.4 g of the oily title compound. Yield: 24.8%.
$^1H$ NMR ($CDCl_3$, ppm) delta = 1.30 (s, 3H), 1.36 (s, 3H), 2.96 and 3.23 (2x m, 2H), 3.72 and 4.08 (2x m, 2H), 4.20 (m, 1H), 7.28 (m, 1H), 7.59 (m, 1H).

## Example 91

(R)-3-Chloro-4-fluoro-2-(1-hydroxyisopropoxy)nitrobenzene and (S)-3-chloro-4-fluoro-2-(2-hydroxypropoxy)-nitrobenzene

A solution of 3.83 g of diethyl azodicarboxylate in 10 ml of tetrahydrofuran was added slowly to a stirred solution of 3.83 g of 2-chloro-3-fluoro-6-nitrophenol, 1.52 g of (S)-1,2-propanediol and 5.76 g of triphenylphosphine in 60 ml of tetrahydrofuran, while maintaining the temperature at 20 °C. The mixture was stirred overnight at room temperature. Then, the reaction mixture was concentrated under reduced pressure, and the residue was distributed between diethyl ether and water. The solid material was filtered off and the ethereal layer was separated, and washed with water, dried over anhydrous sodium sulfate, filtered and evaporated to dryness. The residue was purified by chromatography on silica gel (eluent: diethyl ether/hexane: 2/3) to afford 3.4 g of an oily product, consisting of a 3:1 mixture of the title compounds. The mixture had $[\alpha]_D^{25}$ = +7.8° (c=1.3, $CHCl_3$).
$^1H$ NMR ($CDCl_3$, ppm) minor compound, delta = 1.28 (d, 3H), 4.07 and 4.20 (dABq, 2H), 4.28 (m, 1H), 7.12 (m, 1H), 7.87 (m, 1H). Major compound, delta = 1.33 (d, 3H), 3.80 (m, 2H), 4.66 (m, 1H), 7.07 (m, 1H), 7.82 (m, 1H).

## Example 92

(R)-2-[2-(1-Ethoxyethoxy)propoxyl-3,4-difluoroaniline

0.94 G of (R)-2-[2-(1-ethoxyethoxy)propoxy]-3,4-difluoronitrobenzene was dissolved in 10 ml of methanol. 0.3 G of 10% Pd/C and 1.5 g of ammonium formate were added and the mixture was stirred vigorously. The temperature rose to not more than 35° C. The mixture was stirred for 0.5 hour, then filtered over Hyflo (RTM). The filtrate was evaporated to dryness and the residue was distributed between water and diethyl ether. The ethereal layer was separated, washed once with water, dried over anhydrous sodium sulfate, and evaporated under reduced pressure. The crude residue was purified by chromatography on silica gel (eluent: diethyl ether/hexane: 3/2) to afford 0.8 g of title compound. Yield: 94.0%.
$^1$H NMR ($CDCl_3$, ppm) delta = 1.22 (m, 3H), 1.30 (d, 3H), 1.35 (d, 3H), 3.53-3.66 (m, 2H), 3.96-4.13 (m, 3H), 4.86 (m, 1H), 6.36 (m, 1H), 6.69 (m, 1H).

## Example 93

(R)-2-[2-(1-Ethoxyethoxy)propoxy]-3,4-difluoronitrobenzene

A stirred suspension of 6.72 g of powdered potassium hydroxide and 4.14 g of powdered potassium carbonate in 80 ml of toluene was cooled to -5° C, after which a solution of 5.31 g of 2,3,4-trifluoronitrobenzene and 5.18 g of (R)-2-(1-ethoxyethoxy)-1-propanol in 45 ml of toluene was added slowly, while maintaining the temperature at 0° C. The mixture was stirred for 0.5 hour. Next, water was added and the organic phase was separated. The aqueous layer was extracted twice with toluene and the combined organic layers were washed with water several times till pH=8. The organic phase was evaporated to dryness and the residue was purified by chromatography on silica gel (eluent: hexane/diethyl ether/triethylamine: 2/1/0.01) to afford 8.0 g of title compound, as a 1:1 mixture of diastereomers. Yield: 87%.
$^1$H NMR ($CDCl_3$, ppm) delta = 1.18 (tr, 3H), 1.21 (tr, 3H), 1.30 (m, 6H), 3.58 (m, 2H), 4.1-4.4 (m, 3H), 4.82 (q, 1H), 4.86 (q, 1H), 7.00 (m, 1H), 7.66 (m, 1H).
In addition, 0.7 g of a more polar by-product was obtained, which was identified as (R,R)-2,4-bis[2-(1-ethoxyethoxy)propoxy]-3-fluoronitrobenzene. Yield: 6.7%.
$^1$H NMR ($CDCl_3$, ppm) delta = 1.20 (m, 6H), 1.33 (m, 12H), 3.52 and 3.68 (2x m, 4H), 4.01-4.16 (m, 6H), 4.86 (m, 2H), 6.76 (m, 1H), 7.71 (m, 1H).

## Example 94

(S)-3,4-Difluoro-2-(2-hydroxypropoxy)nitrobenzene and (S)-3,4-difluoro-2-(1-hydroxyisopropoxy)nitrobenzene

A mixture of 0.84 g of (S)-1,2-propanediol and 0.25 g of lithium amide in 10 ml of dry tetrahydrofuran was stirred at room temperature for 1 hour. 1.77 G of 2,3,4-trifluoronitrobenzene dissolved in 10 ml of tetrahydrofuran were added slowly and next the reaction mixture was stirred for 1 hour. The mixture was concentrated under reduced pressure and the residue was distributed between diethyl ether and water. The ethereal layer was separated, washed with water, dried over anhydrous sodium sulfate, filtered and evaporated to dryness. The crude residue was purified by chromatography on silica gel (eluent: hexane/diethyl ether: 3/2) to afford 1.2 g of a 63:37 mixture of the title compounds. Yield: 51.5%. The mixture had $[\alpha]_D^{23}$ = -30.4° (c=2.3, $CHCl_3$).
$^1$H NMR ($CDCl_3$, ppm) delta = 1.26 (d, 3H), 4.11 and 4.36 (2x m, 2H), 4.26 (m, 1H), 7.01 (m, 1H), 7.71 (m, 1H).
Minor compound: delta = 1.40 (d, 3H), 3.78 (m, 2H), 4.70 (m, 1H), 7.01 (m, 1H), 7.71 (m, 1H).
In addition, 0.15 g of a by-product was isolated. Its structure was elucidated as (S)-1,2-bis(2,3-difluoro-6-nitrophenoxy)propane. Yield: 8.0%.
$^1$H NMR ($CDCl_3$, ppm) delta = 1.53 (d, 3H), 4.36 and 4.60 (2x m, 2H), 5.00 (m, 1H), 6.99 (m, 2H), 7.66 (m, 2H).

Example 95

(S)-3-Chloro-4-fluoro-2-(2-hydroxypropoxy)nitrobenzene and (S)-3-chloro-4-fluoro-2-(1-hydroxyisopropoxy)-nitrobenzene

0.5 G of 60% sodium hydride was washed with pentane and then suspended in 12.5 ml of dry tetrahydrofuran. The suspension was stirred and cooled to 10°C and 3.8 g of (S)-1,2-propanediol in 9 ml of dry tetrahydrofuran were added. The mixture was stirred for 0.5 hour. 10 Ml of the solution were added slowly to a stirred solution of 0.97 g of 3-chloro-2,4-difluoronitrobenzene in 10 ml of tetrahydrofuran, kept at 10°C. Next, the reaction mixture was stirred for 0.5 hour and concentrated under reduced pressure. The residue was treated with water and diethyl ether. The ethereal layer was separated, washed with water, dried over anhydrous sodium sulfate, filtered and evaporated to dryness. The crude product was purified by chromatography on silica gel (eluent: diethyl ether/hexane: 1/1) to afford 0.8 g of a 65:35 mixture of the title compounds. Yield: 65%.

$^1H$ NMR ($CDCl_3$, ppm) major compound, delta = 1.28 (d, 3H), 4.07 and 4.20 (2x dd, 1H), 4.27 (m, 1H), 7.08 (m, 1H), 7.86 (m, 1H).

Regioisomer, delta = 1.34 (d, 3H), 3.79 (m, 2H), 4.66 (m, 1H), 7.08 (m, 1H), 7.86 (m, 1H).

The same ratio of regioisomers was obtained by performing the reaction in dimethyl sulfoxide.

Example 96

(S)-1,2-Bis(2,3-difluoro-6-nitrophenoxy)propane

A solution of 1.77 g of 2,3,4-trifluoronitrobenzene and 0.76 g of (S)-1,2-propanediol in 10 ml of diethyl ether was added dropwise to a stirred suspension of 2.24 g of powdered potassium hydroxide and 1.38 g of powdered potassium carbonate in 30 ml of toluene, while keeping the temperature at -10°C. Next, the mixture was stirred at the low temperature for 0.5 hour. Water was added to the mixture, the organic layer was separated, washed with water, dried over anhydrous sodium sulfate, filtered and concentrated under reduced pressure. The residue was purified by chromatography on silica gel (eluent: diethyl ether/hexane: 1/1) to afford 1.6 g of title compound, which had $[\alpha]_D^{22}$ = +61.8° (c=1.5, $CHCl_3$). Yield: 82.0%.

$^1H$ NMR ($CDCl_3$, ppm) delta = 1.53 (d, 3H), 4.38 and 4.60 (dABq, 2H), 4.99 (m, 1H), 7.02 (m, 2H), 7.67 (m, 2H).

In addition, 0,3 g of a mixture of (S)-3,4-difluoro-2-(2-hydroxypropoxy)nitrobenzene and (S)-3,4-difluoro-2-(1-hydroxyisopropoxy)nitrobenzene was obtained. Yield: 13.3%.

The reaction was repeated, but this time 0.76 g of (S)-1,2-propanediol in 10 ml of tetrahydrofuran was added to a stirred suspension of 2.24 g of powdered potassium hydroxide, 1.38 g of powdered potassium carbonate and 1.77 g of 2,3,4-trifluoronitrobenzene in 30 ml of tetrahydrofuran, while maintaining the temperature at -10°C. After that, the suspension was stirred for 2 hours at -10°C and then worked up as described above. 1.65 G of crude material were purified and afforded 1.2 g of title product, as evidenced by NMR analysis. Yield: 61.5%. In addition, 0.1 g of a 55:45 mixture of the regioisomers referred to above was obtained.

$^1H$ NMR ($CDCl_3$, ppm) delta = 1.26 (d, 3H), 4.13 and 4.37 (dABq, 2H), 4.26 (m, 1H), 7.02 (m, 1H), 7.70 (m, 1H).

Regioisomer, delta = 1.40 (d, 3H), 3.78 (m, 2H), 4.70 (m, 1H), 7.02 (m, 1H), 7.70 (m, 1H).

Example 97

(S)-1,2-Bis(2,3-difluoro-6-nitrophenoxy)propane

A solution of 2.09 g of diethyl azodicarboxylate in 10 ml of tetrahydrofuran was added slowly to a stirred

solution of 2.33 g of (R)-3,4-difluoro-2-(2-hydroxypropoxy)nitrobenzene, which was not entirely pure, 1.75 g of 2,3-difluoro-6-nitrophenol and 3.14 g of triphenylphosphine in 40 ml of tetrahydrofuran, while maintaining the temperature at 20°C. The mixture was stirred at room temperature for 18 hours, then worked up according to the procedure described in example 24.

The silica gel column was eluted with diethyl ether/hexane: 1/1 to provide 3.3 g of the oily title compound. Yield: 85%. The product had $[\alpha]_D^{23}$ = +57.5° (c=1.9, $CHCl_3$).

$^1H$ NMR ($CDCl_3$, ppm) delta = 1.53 (d, 3H), 4.38 and 4.60 (dABq, 2H), 4.98 (m, 1H), 7.02 (m, 2H), 7.67 (m, 2H).

## Example 98

(S)-3-Chloro-4-fluoro-2-[2-(2-tetrahydropyranyloxy)propoxy]nitrobenzene

A solution of 10.4 g of diethyl azodicarboxylate in 50 ml of tetrahydrofuran was added slowly to a stirred solution of 9.6 g of 2-chloro-3-fluoro-6-nitrophenol, 8.0 g of (S)-2-(2-tetrahydropyranyloxy)-1-propanol and 15.7 g of triphenylphosphine in 250 ml of tetrahydrofuran. The reaction mixture was stirred overnight, then worked up as described in example 24. Chromatographic purification on silica gel (eluent: hexane/diethyl ether/triethylamine: 3/2/0.025) provided 16.0 g of title compound as a 2:1 mixture of both diastereomers. The mixture had $[\alpha]_D^{22}$ = -29.7° (c=2.2, $CHCl_3$). Yield: 96%.

$^1H$ NMR ($CDCl_3$, ppm) delta = 1.38 (d, 3H), 1.5-1.9 (m, 6H), 3.52 and 3.91 (2x m, 2H), 4.1-4.3 (m, 3H), 4.86 (tr, 1H), 7.07 (m, 1H), 7.84 (m, 1H).

Minor compound, delta = 1.33 (d, 3H), 1.5-1.9 (m, 6H), 3.52 and 3.91 (2x m, 2H), 4.1-4.3 (m, 3H), 4.78 (tr, 1H), 7.07 (m, 1H), 7.84 (m, 1H).

## Example 99

(S)-3-Chloro-4-fluoro-2-[2-(2-tetrahydropyranyloxy)propoxy]nitrobenzene

A solution of 5.8 g of 3-chloro-2,4-difluoronitrobenzene in 20 ml of toluene was added slowly to a stirred suspension of 5.6 g of powdered potassium hydroxide, 4.55 g of powdered potassium carbonate and 5.3 g of (S)-2-(2-tetrahydropyranyloxy)-1-propanol in 100 ml of toluene, while maintaining the temperature at 15°C. The mixture was stirred at room temperature for 0.5 hour. Water was added and the organic layer was separated. The aqueous layer was extracted once with toluene. The combined organic layers were dried over anhydrous sodium sulfate, filtered and evaporated under reduced pressure to afford 12 g of crude product. It was purified by chromatography on silica gel (eluent: diethyl ether/hexane: 1/1) to give 6.8 g of title compound. On TLC analysis it was identical to the product obtained in example 33. Yield: 68%.

In addition, 2.7 g of one and 0.5 g of a second by-product were obtained which on NMR analysis were identified as diastereomeric (S,S)-3-chloro-2,4-bis[2-(2-tetrahydropyranyloxy)propoxy]nitrobenzenes. The separation of the diastereomers was not complete. Yield: 29.1 %.

$^1H$ NMR ($CDCl_3$, ppm) delta = 1.31 and 1.38 (2x d, 6H), 1.4-1.9 (m, 12H), 3.52 (m, 2H), 3.9-4.3 (m, 8H)), 4.81 and 4.90 (2x m, 2H), 6.84 (d, 1H), 7.89 (d, 1H).

Diastereomer, delta = 1.36 and 1.38 (2x d, 6H), 1.4-1.9 (m, 6H), 3.53 (m, 2H), 3.9-4.4 (m, 8H), 4.90 and 4.95 (2x m, 2H), 6.78 (d, 1H), 7.88 (d, 1H).

In a previous experiment a catalytic amount of tetrabutylammonium hydrogen sulfate was used. A 65% yield of the title compound was obtained, and nearly the same amount of by-products as formed above.

In a later experiment a mixture of 2,3,4-trifluoronitrobenzene and (S)-2-(2-tetrahydropyranyloxy)-1-propanol in toluene was added to a mixture of powdered potassium hydroxide and powdered potassium carbonate in toluene. After purification a 84% yield of title compound was obtained. There was an obviously smaller amount of by-products.

## Example 100

(R)-3-Chloro-4-fluoro-2-[2-(2-tetrahydropyranyloxy)propoxy]nitrobenzene

A mixture of 0.96 g of 2-chloro-3-fluoro-6-nitrophenol, 1.05 ml of ethyldiisopropylamine and 1.48 g of (R)-1-iodo-2-(2-tetrahydropyranyloxy)propane in 5 ml of dry dimethylformamide was heated under stirring at 80-100 °C for 30 hours. The reaction mixture was cooled and evaporated under reduced pressure. The residue was diluted with toluene and the solvent was distilled off under vacuum. The residue was distributed between diethyl ether and water. The ethereal layer was separated, washed with water, dried over anhydrous sodium sulfate, filtered and concentrated to dryness. The crude mass was purified by chromatography on silica gel (eluent: diethyl ether/hexane: 1/4 and 0.5% of triethylamine) to afford 0.4 g of title compound, a 5:2 mixture of diastereomers. The NMR spectra were identical to those reported in example 98.

In a previous experiment 2.2 g of potassium 2-chloro-3- fluoro-6-nitrophenoxide and 2.7 g of (R)-1-iodo-2-(2-tetrahydropyranyloxy)propane in 8 ml of dimethylformamide were heated at 80-100 °C for 36 hours. The workup was identical to that described above. After purification 0.9 g of an oil was obtained, 40% of which consisted of title compound, a mixture of diastereomers, as evidenced by NMR analysis.

## Example 101

(S)-3-Chloro-4-fluoro-2-(2-hydroxypropoxy)nitrobenzene and (S)-3-chloro-4-fluoro-2-(1-hydroxyisopropoxy)-nitrobenzene

0.53 G of powdered potassium hydroxide was added to a solution of 1.9 g of 2-chloro-3-fluoro-6-nitrophenol in 7.0 ml of dimethylformamide and the mixture was stirred for 1 hour. 1.4 G of (S)-1-bromo-2-propanol were added and the mixture was heated at 80-100 °C for 16 hours, while stirring. Then, the reaction mixture was concentrated under reduced pressure and water and diethyl ether were added to the residue. The ethereal layer was separated, washed with 0.1N sodium hydroxide solution and water, dried over anhydrous sodium sulfate, filtered and concentrated under reduced pressure to afford 1.2 g of product, which was purified by chromatography on silica gel (eluent: hexane/diethyl ether: 1/1). 0.9 G of a 2:1 mixture of the title compounds was obtained. Yield: 37. 5%. The mixture had $[\alpha]_D^{20}$ = +6.9° (c=2.13, $CHCl_3$). The major compound consisted of a 94:6 mixture of the optical isomers.

$^1$H NMR ($CDCl_3$, ppm) delta = 1.28 (d, 3H), 4.08 and 4.21 (dABq, 2H), 4.28 (m, 1H), 7.09 (m, 1H), 7.87 (m, 1H).

Minor Compound, delta = 1.35 (d, 3H), 3.80 (m, 2H), 4.66 (m, 1H), 7.06 (m, 1H), 7.82 (m, 1H).

## Example 102

(S)-3-Chloro-4-fluoro-2-(2-hydroxypropoxy)nitrobenzene and (S)-3-chloro-4-fluoro-2-(1-hydroxyisopropoxy)-nitrobenzene

15.0 G of (S)-3-chloro-4-fluoro-2-[2-(2-tetrahydropyranyloxy)propoxy]nitrobenzene were dissolved in 200 ml of methanol. 15 Ml of 4N hydrochloric acid were added. After stirring at room temperature for 0.5 hour, 90% of the starting material was hydrolyzed as shown by TLC analysis (hexane/diethyl ether: 1/1). Another 5 ml of 4N hydrochloric acid were added and stirring was continued for 0.5 hour. The reaction mixture was neutralized by adding 2N sodium hydroxide solution, after which the solution was evaporated to dryness. The residue was distributed between diethyl ether and water. The organic layer was separated and washed with water, dried over anhydrous sodium sulfate, filtered and evaporated to dryness. 11.9 G of crude material were obtained, which was purified by chromatography on silica gel (eluent: diethyl ether/hexane: 1/1) to afford 9.8 g of oily product, consisting of a 2:1 mixture of the title compounds. Yield: 87.5%.

$^1$H NMR ($CDCl_3$, ppm) major compound, delta = 1.27 (d, 3H), 4.13 (dABq, 2H), 4.26 (m, 1H), 7.11 (dd, 1H),

7.87 (dd, 1H).
Regioisomer delta = 1.34 (d, 3H), 3.78 (d, 2H), 4.63 (m, 1H), 7.06 (dd, 1H , 7.82 (dd, 1H).

## Example 103

(R)-3,4-Difluoro-2-(2-hydroxypropoxy)nitrobenzene and (R)-3,4-difluoro-2-(1-hydroxyisopropoxy)nitrobenzene

To a stirred solution of 1.0 g of (R)-3,4-difluoro-2-(2-hydroxypropoxy)nitrobenzene, with $[\alpha]_D^{20}$ = +4.1° (c=2.0, $CHCl_3$), in 15 ml of methanol was added 1.0 ml of 4N hydro chloric acid, after which the mixture was stirred for 0.5 hour. The mixture was neutralized by adding 2N sodium hydroxide solution and concentrated under reduced pressure. The residue was dissolved in diethyl ether, the ethereal layer was washed with water, dried over anhydrous sodium sulfate, filtered and evaporated to dryness.
On NMR analysis the product proved to be a 2:1 mixture of the title isomers. The mixture had $[\alpha]_D^{20}$ = +27.1. (c=2.0, $CHCl_3$), which value confirmed the (R)-configuration of both isomers.
$^1H$ NMR ($CDCl_3$, ppm) delta = 1.26 (d, 3H), 4.11 and 4.39 (dABq, 2H), 4.23 (m, 1H), 7.00 (m, 1H), 7.73 (m, 1H).
Regioisomer, delta = 1.40 (d, 3H), 3.79 (m, 2H), 4.70 (m, 1H), 7.00 (m, 1H), 7.67 (m, 1H).

## Example 104

(S)-3-Chloro-4-fluoro-2-(2-mesyloxypropoxy)nitrobenzene and (S)-3-chloro-4-fluoro-2-(1-mesyloxyisopropoxy)nitrobenzene

4.5 G of the mixture obtained in example 102 and 3.7 ml of triethylamine were dissolved in 80 ml of dry tetrahydrofuran. The mixture was stirred and a solution of 2.29 g of methanesulfonyl chloride in 25 ml of dry tetrahydrofuran was added, while keeping the temperature at 10°C. TLC analysis on silica gel (eluent: petroleum ether (40-60)/diethyl ether: 1/1) showed complete conversion just after finishing the addition. The reaction mixture was evaporated to dryness and the residue was distributed between diethyl ether and water. The organic layer was separated, washed with water, dried over anhydrous sodium sulfate and concentrated under reduced pressure to give 5.6 g of a 2:1 mixture of the title compounds. The mixture was used as such in example 105. Yield: 94.8%.

## Example 105

(R)-2-(2-Bromopropoxy)-3-chloro-4-fluoronitrobenzene and (S)-2-(1-bromoisopropoxy)-3-chloro-4-fluoronitrobenzene

5.6 G of the mixture produced in example 104 were dissolved in 15 ml of dry acetone. The solution was added slowly to a stirred mixture of 7.4 g of lithium bromide and 100 mg of cupric bromide in 100 ml of acetone. The reaction mixture was heated under reflux for 24 hours, and evaporated to dryness. The residue was distributed between water and diethyl ether. The layers were separated and the ethereal phase was washed with water, dried over anhydrous sodium sulfate, filtered and concentrated under reduced pressure. The residue was purified by chromatography on silica gel (eluent: diethyl ether/petroleum ether (40-60): 2/3) to afford 4.7 g of a 2:1 mixture of the title compounds. Yield: 88.0%. The mixture was used as such in example 106.
$^1H$ NMR ($CDCl_3$, ppm) major compound, delta = 1.86 (d, 3H), 4.35 (dABq, 2H), 4.42 (m, 1H), 7.10 (dd, 1H), 7.86 (dd, 1H).
Minor compound, delta = 1.50 (d, 3H), 3.59 (dABq, 2H), 4.71 (m, 1H), 7.10 (dd, 1H), 7.86 (dd, 1H).

Example 106

(R)-2-(2-Bromopropoxy)-3-chloro-4-fluoroaniline and (S)-2-(1-bromoisopropoxy)-3-chloro-4-fluoroaniline

4.7 G of the mixture prepared in example 105 were dissolved in 50 ml of abs. ethanol and 0.5 g of 10% Pd/C was added. The suspension was stirred vigorously and saturated with $H_2$. After the required amount of $H_2$ had been absorbed, the catalyst was filtered off over Hyflo (RTM) and the filtrate was evaporated to dryness. The residue was dissolved in diethyl ether, washed with water, dried over anhydrous sodium sulfate, filtered and evaporated under reduced pressure. 4.2 G of a 2:1 mixture of the title compounds were obtained. Yield: 98.9%. The mixture was used as such in example 107.

Example 107

(S)-8-Chloro-7-fluoro-3,4-dihydro-3-methyl-2H-[1,4]benzoxazine and (S)-8-chloro-7-fluoro-3,4-dihydro-2-methyl-2H-[1,4]benzoxazine

4.0 G of the mixture described in example 106 were dissolved under stirring in 125 ml of dry tetrahydrofuran. To this solution 3.8 g of potassium tert-butoxide were added in small portions at 15°C over a period of 20 minutes. The reaction mixture was stirred for 1 hour, then concentrated at low temperature. The residue was distributed between diethyl ether and water. The ethereal layer was separated, washed with water to neutral pH, dried over anhydrous sodium sulfate, filtered and concentrated under reduced pressure to afford 3 g of crude product. It was purified by chromatography on silica gel (eluent: hexane/diethyl ether: 2/3). The title compounds were obtained in yields of 1.3 g and 0.6 g respectively (66.6%). The latter had $[\alpha]_D^{20}$ = -38.9° (c=1.3, $CHCl_3$).
On TLC analysis (silica gel; hexane/diethyl ether: 1/1) both compounds were identical to authentic samples.

Example 108

(S)-3,4-Difluoro-2-(2,3-isopropylidenedioxypropylthio)nitrobenzene

A solution of 10.4 g of diethyl azodicarboxylate in 50 ml of tetrahydrofuran was added slowly to a stirred mixture of 9.55 g of 2,3-difluoro-6-nitrothiophenol, (R)-2,3-isopropylidenedioxy-1-propanol and 15.7 g of triphenylphosphine in 250 ml of tetrahydrofuran, while keeping the temperature at 20°C. The reaction mixture was stirred at room temperature for 18 hours and then worked up as described in example 24. A 3/1 mixture of hexane/diethyl ether was used as eluent for chromatographic purification. The product fractions were combined and evaporated to dryness. The residue was treated with hexane, and the remaining unsoluble triphenylphosphine oxide was removed by filtration. The filtrate was concentrated under reduced pressure to afford 8.35 g of the oily title compound, which had $[\alpha]_D^{23}$ = +34.7° (c=1.5, $CHCl_3$). Yield: 54%.
1H NMR ($CDCl_3$, ppm) delta = 1.30 (s, 3H), 1.35 (s, 3H), 3.05 and 3.30 (dABq, 2H), 3.73 and 4.06 (dABq, 2H), 4.21 (m, 1H), 7.24 (m, 1H), 7.69 (m, 1H).

Example 109

(S)-2-(2,3-Dihydroxypropylthio)-3,4-difluoronitrobenzene

5.4 G of (S)-3,4-difluoro-2-(2,3-isopropylidenedioxypropylthio)nitrobenzene were dissolved under stirring

in 400 ml of methanol. 1.0 G of Dowex 50W-8X-400 $H^+$ (RTM) ion-exchange resin was added and the mixture was stirred at room temperature for 18 hours. The resin was filtered off and the filtrate was concentrated under reduced pressure. The solid residue was recrystallized from diethyl ether/pentane to give 3.7 g of title compound, which had $[\alpha]_D^{23}$ = +24.0° (c = 1.0, $CHCl_3$). M.p. 70-71 °C. Yield: 78%.
$^1H$ NMR ($CDCl_3$ + DMSO (D6), ppm) delta = 3.54 (m, 2H), 3.63 and 3.76 (2x m, 2H), 3.96 (m, 1H), 7.24 (m, 1H), 7.69 (m, 1H).

## Example 110

(R)-3,4-Difluoro-2-(2-mesyloxypropoxy)aniline

2.5 G of ammonium formate were added to a vigorously stirred mixture of 1.56 g of (R)-3,4-difluoro-2-(2-mesyloxypropoxy)nitrobenzene and 0.5 g of 10% Pd/C in 5 ml of methanol. The reaction mixture was stirred at room temperature for 2 hours and filtered over Hyflo (RTM). The filtrate was concentrated under reduced pressure. The residue was distributed between water and diethyl ether. The ethereal layer was separated and the aqueous layer was extracted twice with diethyl ether. The combined ethereal fractions were washed with water, dried over anhydrous sodium sulfate, filtered and evaporated under reduced pressure to provide 1.45 g of oily title compound, contaminated with 10 mol % of solvents.
$^1H$ NMR ($CDCl_3$, ppm) delta = 1.48 (d, 3H), 3.10 (s, 3H), 4.14 (m, 2H), 5.13 (m, 1H), 6.39 (m, 1H), 6.71 (q, 1H).

## Example 111

(R)-3,4-Difluoro-2-(1-tosyloxyisopropoxy)nitrobenzene

0.4 G of 60% sodium hydride, previously washed with pentane, was mixed with 20 ml of diethyl ether and a solution of 2.33 g of (R)-3,4-difluoro-2-(2-hydroxypropoxy)nitrobenzene in 10 ml of diethyl ether was added, while stirring. The mixture was stirred for 0.5 hour, then 1.9 g of p-toluenesulfonyl- chloride in 10 ml of diethyl ether were added slowly and stirring was continued at room temperature for 18 hours, and at reflux temperature for 6 hours. The reaction mixture was cooled, water was added and the ethereal layer was separated, washed with water, dried over anhydrous sodium sulfate, filtered and evaporated to dryness. The residue was purified by chromatography on silica gel (eluent: hexane/diethyl ether: 1/1) to afford 2.0 g of a 5:1 mixture of the title compound and its regioisomer. Yield: 51.7%. The mixture solidified on standing and had $[\alpha]_D^{23}$ = -16.5° (c = 0.75, $CHCl_3$). The mixture was recrystallized from diethyl ether/pentane to give $[\alpha]_D^{23}$ = -23.3° (c = 0.3, $CHCl_3$) and m.p. 62-63 °C.
$^1H$ NMR ($CDCl_3$, ppm) delta = 1.40 (d, 3H), 2.45 (s, 3H), 4.20 (m, 2H), 4.75 (m, 1H), 6.99 (m, 1H), 7.33 and 7.73 (ABq, 4H), 7.66 (m, 1H).
Regioisomer, delta = 1.44 (d, 3H), 2.41 (s, 3H), 4.20 (m, 2H), 4.93 (m, 1H), 6.99 (m, 1H), 7.29 and 7.80 (ABq, 4H), 7.66 (m, 1H).
The mixture was used as such in example 112.

## Example 112

(R)-3,4-Difluoro-2-(1-tosyloxyisopropoxy)aniline

To 1.7 g of the mixture prepared in example 111, dissolved in 40 ml of abs. ethanol, was added 0.4 g of 10% Pd/C. The mixture was stirred vigorously and saturated with $H_2$. After the required amount of $H_2$ had been absorbed, the catalyst was filtered over Hyflo (RTM) and the filtrate was concentrated to dryness. The residue was dissolved in diethyl ether and the ethereal layer was washed with water, dried over anhydrous

sodium sulfate, filtered and evaporated to dryness to provide 1.4 g of a 5.25:1 mixture of the title compound and its regioisomer. Yield: 100%. The mixture had $[\alpha]_D^{23}$ = -12.4° (c=1.0, $CHCl_3$).
$^1H$ NMR ($CDCl_3$, ppm) delta = 1.31 (d, 3H), 2.43 (s, 3H), 3.66 (br.s, 2H), 4.14 (m, 2H), 4.43 (m, 1H), 6.36 (m, 1H), 6.69 (m, 1H), 7.33 and 7.78 (ABq, 4H).
Regioisomer delta = 1.35 (d, 3H), 2.43 (s, 3H), 3.66 (br.s, 2H), 4.14 (m, 2H), 4.95 (m, 1H), 6.36 (m, 1H), 6.69 (m, 1H), 7.33 and 7.78 (ABq, 4H).
The mixture was used as such in example 113.

## Example 113

(R)-7,8-Difluoro-3,4-dihydro-2-methyl-2H-[1,4]benzoxazine

1.2 G of the mixture prepared in example 112 were dissolved in 5 ml of toluene and the solution obtained was added slowly to a stirred suspension of 0.7 g of powdered potassium hydroxide, 0.4 g of powdered potassium carbonate, 50 mg of tetrabutylammonium hydrogen sulfate in 15 ml of toluene, while maintaining the temperature at 5° C. The reaction mixture was stirred at 5° C for 1 hour, at ambient temperature for 1 hour and at 40° C for 4 hours. Finally, the reaction mixture was left standing overnight. Water was added and the organic layer was separated, then washed with water, and evaporated under reduced pressure. The residue was purified by chromatography on silica gel (eluent: hexane/diethyl ether: 1/1) to give 0.5 g of the title compound, which on TLC and IR was identical to an authentic sample. The compound had $[\alpha]_D^{23}$ = +35.6° (c=1.5, $CHCl_3$). Yield: 80.0%.
$^1H$ NMR ($CDCl_3$, ppm) delta = 1.41 (d, 3H), 3.08 and 3.34 (dABq, 2H), 4.27 (m, 1H), 6.26 (m, 1H), 6.54 (m, 1H).

## Example 114

(R)-3,4-Difluoro-2-(2-hydroxypropoxy)nitrobenzene and (R)-3,4-difluoro-2-(1-hydroxyisopropoxy)nitrobenzene

0.4 G of 60% sodium hydride, previously washed with pentane, was suspended in 20 ml of dry diethyl ether under stirring and 2.33 g of (R)-3,4-difluoro-2-(2-hydroxypropoxy)nitrobenzene were added. The mixture was heated at reflux temperature for 2 hours. The reaction mixture was cooled, water was added and the organic layer was separated, dried over anhydrous sodium sulfate, filtered and evaporated to dryness to afford 2.2 g of a mixture of the title compounds, which had $[\alpha]_D^{23}$ = +38.1° (c=2.0, $CHCl_3$).

Isomerization was continued by heating the mixture of the two regioisomers in diethyl ether under reflux for 4 hours. After the workup according to the procedure described above, a mixture with $[\alpha]_D^{23}$ = +35.7° (c=2.0, $CHCl_3$) was obtained.

## Example 115

(R)-7,8-Difluoro-3,4-dihydro-3-hydroxymethyl-2H-[1,4]benzoxazine

A stirred mixture of 3.55 g of (R)-7,8-difluoro-2,3-dihydro-3-hydroxymethyl-4-tosyl-[1,4]benzoxazine, 34 ml of 48% hydrobromic acid and 3.4 g of phenol was heated at 130° C for 2 hours. The reaction mixture was made alkaline by adding 4N sodium hydroxide solution and then extracted with diethyl ether. The ethereal phase was washed with brine, dried over anhydrous sodium sulfate, filtered and evaporated to dryness. The residue was purified by chromatography on silica gel (eluent: diethyl ether/hexane: 9/1) to afford 1.5 g of title compound, which had $[\alpha]_D^{23}$ = +16.3° (c=2.0, $CHCl_3$). Yield: 75%.
$^1H$ NMR ($CDCl_3$, ppm) delta = 3.52 (m, 1H), 3.62 and 3.72 (dABq, 2H), 4.09 and 4.24 (dABq, 2H), 6.29 (m,

1H), 6.56 (m, 1H).

## Claims

1. (R)- or (S)-7-Fluoro-8-X-3,4-dihydro-2H-[1,4]benzoxazines and -benzothiazines of the general formula III

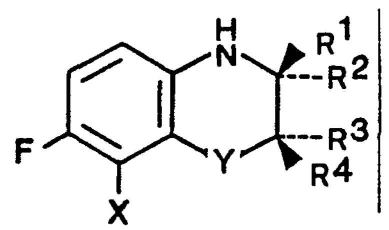

III

where one of the substituents $R^1$, $R^2$, $R^3$ and $R^4$ is $CH_2OH$ or $CH_2Z$, the remaining being hydrogen, Z is hydrogen, fluoro or protected hydroxy, Y is oxygen or sulfur and X is fluoro, chloro, methyl or hydrogen, with the proviso that for benzoxazines where $R^3$ and $R^4$ both are hydrogen, X is methyl or hydrogen.

2. (R)- or (S)-Pyridobenzothiazines of the general formula XVIII,

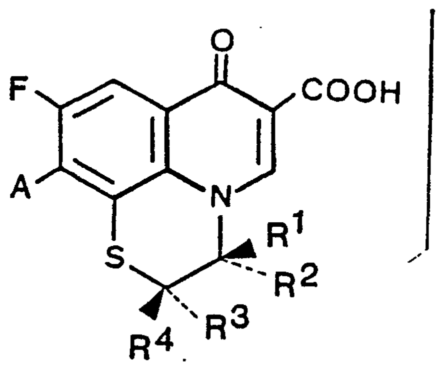

XVIII

where A is X or $NQ^1Q^2$, where $Q^1$ and $Q_2$ are the same or different and are either hydrogen, or a (1-6C)alkyl group, with the proviso that $Q^1$ and $Q^2$ are not both hydrogen, or together form a (3-7C)heterocyclic ring which contains one or two nitrogen ring atoms and/or an oxygen ring atom and which ring may be substituted by a (1-4C)alkyl group and where $R^1$, $R^2$, $R^3$, $R^4$ and X are as defined in claim 1.

3. Process for preparing 7-fluoro-8-X-3,4-dihydro-2H-[1,4]benzoxazines and -benzothiazines of the general formula III,

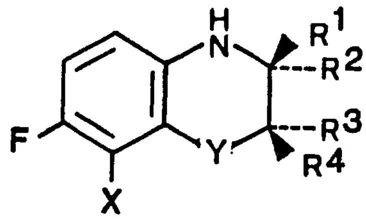

III

where X is fluoro, chloro, methyl or hydrogen and Y is oxygen or sulfur, where
a. $R^1$ = $CH_2Z$ or $CH_2OH$ when in the starting compound IX
$R^6$ = hydroxy and $R^5$ = hydrogen or
b. $R^2$ = $CH_2Z$ or $CH_2OH$, when in the starting compound IX
$R^6$ = hydrogen and $R^5$ = hydroxy or
c. $R^3$ = $CH_2Z$ or $CH_2OH$, when in the starting compound X
$R^7$ = hydrogen and $R^8$ = hydroxymethyl or
d. $R^4$ = $CH_2Z$ or $CH_2OH$, when in the starting compound X
$R^7$ = hydroxymethyl and $R^8$ = hydrogen
in each case the remaining R's are hydrogen and where Z is as defined in claim 1, in a stereochemically pure form or as a racemic mixture, and which comprises the following steps

a. reacting 4-fluoro-3-halo-2-(hydroxy(3C)alkyl-Y)nitrobenzenes of formula IX,

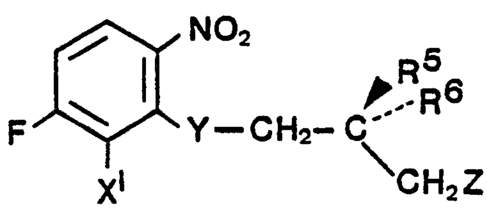

IX

where either
$R^5$ = hydroxy and $R^6$ = hydrogen or
$R^5$ = hydrogen and $R^6$ = hydroxy,
$X'$ is fluoro or chloro, Y is oxygen or sulfur and Z is as defined in claim 1 in a stereochemically pure form or as a racemic mixture, and/or of formula X,

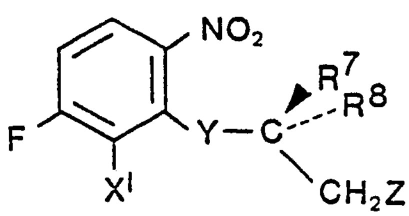

X

where either
$R^7$ = hydrogen and $R^8$ = hydroxymethyl or
$R^7$ = hydroxymethyl and $R^8$ = hydrogen
and $X'$, Y and Z are as defined above, in a stereochemically pure form or as a racemic mixture, with an hydroxyl group activating agent, affording the corresponding compound of formula IXa or Xa where the hydroxyl group is replaced by a proper leaving group and the other substituents are as defined before,

b. which step is preceded or followed by converting the nitro group in IX, IXa, X or Xa into an amino group with a reducing agent according to methods known in the art to give an aniline derivative,

c. treating the resulting aniline derivative with a base effecting ring closure of the aniline derivative,

d. optionally substituting the 8-fluoro atom of the resulting ring closed aniline derivative by a methyl group or a hydrogen atom by methods known in the art,

e. optionally removing the hydroxyl protecting group on $CH_2Z$ of the resulting ring closed aniline derivative by methods known in the art.

4. Process according to claim 3 characterised in that the leaving group is chosen from the groups comprising a methylsulfonyloxy group, a p-toluenesulfonyloxy group, a bromo atom or a group of the formula $(phenyl)_3P^+$-O-.

5. Process according to claim 3 or 4 characterised in that the reducing agent is chosen from the group comprising Raney nickel, stannous chloride, tin metal and hydrogen or ammonium formate with a noble metal catalyst.

6. Process according to any one of claims 3 - 5 characterised in that the base which effects ring closure is potassium tert-butoxide, potassium hydroxide or, preferably, a mixture of potassium hydroxide and potassium carbonate.

7. Process according to any one of claims 3 - 6, characterised in that the starting compound 4-fluoro-3-halo-2-(hydroxy(3C)alkyl-Y)nitrobenzene of formula IX or X, or a mixture of IX and X, is prepared by reacting under basic conditions a 2,4-difluoro-3-halonitrobenzene with a compound of the group comprising 3-Z-1,2-propanediol, 1-hydroxy-3-Z-2-propanethiol and 2-hydroxy-3-Z-1-propanethiol, where Y and Z are as defined in claim 3 and halo is fluoro or chloro.

8. Process according to any one of claims 3 - 6, characterised in that the starting compound 4-fluoro-3-halo-2-(hydroxy(3C)alkyl-Y)nitrobenzene according to formula IX or X, or a mixture of IX and X is prepared by reacting under basic conditions a 3-fluoro-2-halo-6-nitrophenol or the corresponding thiophenol with a 2-$CH_2Z$-oxirane of formula XI,

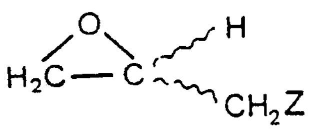

XI

where halo is fluoro or chloro and Y and Z are as defined in claim 3.

9. Process according to any one of claims 3 -6, characterised in that the starting compound 4-fluoro-3-halo-2-(hydroxy(3C)alkyl-Y)nitrobenzene of formula IX or X, or a mixture of IX and X, is prepared by reacting a 3-fluoro-2-halo-6-nitrophenol or the corresponding thiophenol with 1-L-3-Z-2-propanol in the presence of a base,
where L is an easily leaving atom or group, Y and Z are as defined in claim 3 and halo is fluoro or chloro.

10. Process according to any one of claims 3 -6, characterised in that the starting compound 4-fluoro-3-halo-2-(hydroxy(3C)alkyl-Y)nitrobenzene of formula IX or X, or a mixture of IX and X is prepared by reacting a mixture of 3-fluoro-2-halo-6-nitrophenol or the corresponding thiophenol with 3-Z-1,2-propanediol in the presence of triphenylphosphine and di(1-6C)alkyl azodicarboxylate, where Y and Z are as defined in claim 3 and halo is fluoro or chloro.

11. Process according to any one of claims 3 -6, characterised in that the starting compound 4-fluoro-3-halo-2-(hydroxy(3C)alkyl-Y)nitrobenzene of formula IX or X respectively is prepared by reacting a 3-fluoro-2-halo-6-nitrophenol or the corresponding thiophenol with the 2-OR″-3-Z-propanol or 1-OR″-3-Z-2-propanol respectively in the presence of triphenylphosphine and di(1-6C)alkyl azodicarboxylate, where R″ is a hydroxyl protecting group, halo is fluoro or chloro and Y and Z are as defined in claim 3 and removing the R″-group from the obtained product.

12. Process according to any one of claims 3 -6, characterised in that the starting compound 4-fluoro-3-halo-2-(hydroxy(3C)alkyl-Y)nitrobenzene of formula IX or X respectively is prepared by reacting a 3-fluoro-2-halo-6-nitrophenol or the corresponding thiophenol with 1-L-2-OR″-3-Z-propane or 2-L-1-OR″-3-Z-propane respectively in the presence of a base, where L is an easily leaving group, R″ is a hydroxyl protecting group, halo is fluoro or chloro and Y and Z are as defined in claim 3 and removing the R″-group from the obtained product.

13. Process according to any one of claims 3 -6, characterised in that the starting compound 4-fluoro-3-halo-2-(hydroxy(3C)alkyl-Y)nitrobenzene of formula IX or X respectively is prepared by reacting under basic conditions a 2,4-difluoro-3-halo-nitrobenzene with 2-OR″-3-Z-propanol or 1-OR″-3-Z-2-propanol respectively or a corresponding thiol, where Y and Z are as defined in claim 3, halo is fluoro or chloro and R″ is a hydroxy protecting group, and removing the hydroxyl protecting group from the obtained product.

14. Process according to claims 3-13, characterised in that the asymmetrical starting compound is used in a stereochemically pure form.

15. Process according to claim 14 followed by conversion of the stereochemically pure benzoxazine or benzothiazine of formula III into the corresponding stereochemically pure pyridobenzoxazine or pyridobenzothiazine of the general formula XII,

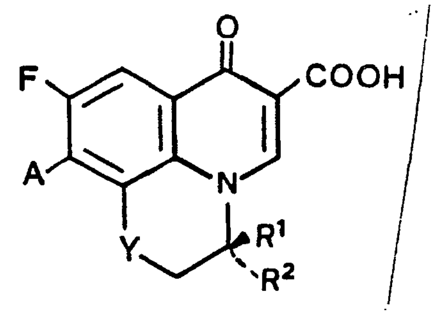

XII

where A is as defined in claim 2, one of the substituents $R^1$ and $R^2$ is $CH_2OH$ or $CH_2Z$, the other being hydrogen and where X, Y and Z are as defined in claim 3, by methods known in the art.

16. Process according to claim 15, characterised by the following steps

a. the benzoxazine or benzothiazine is reacted with di(1-6C)alkyl ethoxymethylenemalonate,

b. an acid is added in order to close the pyridine ring,

c. the obtained pyridobenzoxazine or pyridobenzothiazine of formula XIV,

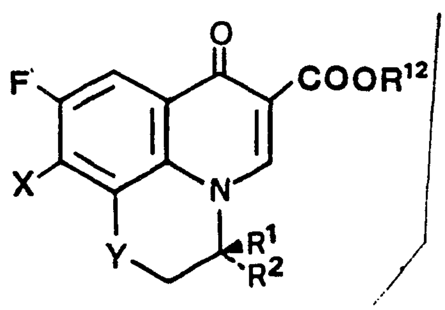

XIV

where $R^{12}$ is (1-6C)alkyl and X, Y, $R^1$ and $R^2$ are as defined in claim 15,
is hydrolysed affording the corresponding carboxylic acid,

d. as an option one of the following reactions is subsequently carried out

d1. the 10-fluoro atom is substituted with an amine

d2. a 10-chloro-pyridobenzothiazine is oxidized to the corresponding sulfoxide or sulfon, and the 10-chloro atom is substituted with an amine $NHQ^1Q^2$,
and then the sulfoxide or sulfon is reduced to the sulfide,
where $Q^1$ and $Q^2$ are as defined in claim 15.

17. process according to claim 15 or 16 characterised in that the compound of formula IV

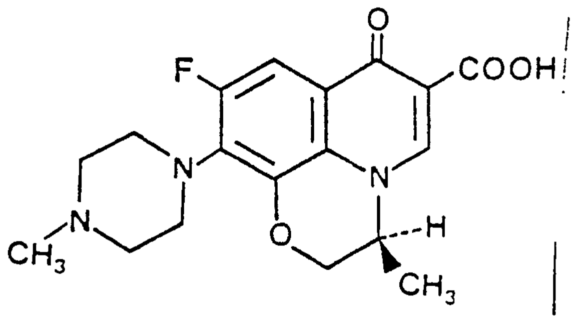

IV

((S)-ofloxacin) is prepared starting from the stereochemically pure benzoxazine of formula XIII

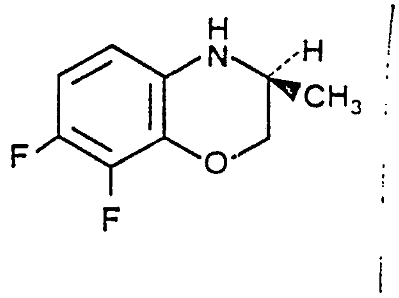

XIII

18. Process according to claim 14 followed by conversion of the stereochemically pure benzoxazine or benzothiazine of formula III into the corresponding stereochemically pure pyridobenzoxazine or pyrodobenzothiazine of the general formula VIII,

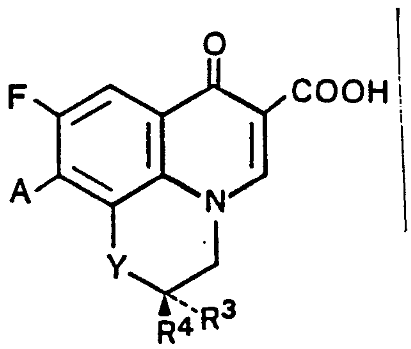

VIII

where A is as defined in claim 2, one of the substituents $R^3$ and $R^4$ is $CH_2OH$ or $CH_2Z$, the other being hydrogen and where X, Y and Z are as defined in claim 3, by methods known in the art.

19. Process according to claim 18, characterised by the following steps

a. the benzoxazine or benzothiazine is reacted with di(1-6C)alkyl ethoxymethylenemalonate,

b. an acid is added in order to close the pyridine ring,

c. the obtained pyridobenzoxazine or pyridobenzothiazine of formula XV,

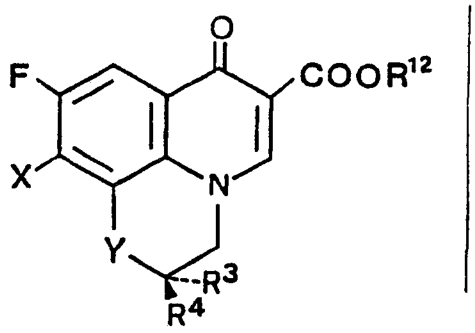

XV

where $R^{12}$ is (1-6C) alkyl and X, Y, $R^3$ and $R^4$ are as defined in claim 18, is hydrolysed affording the corresponding carboxylic acid,

d. as an option one of the following reactions is subsequently carried out

d1. the 10-fluoro atom is substituted with an amine $NHQ^1Q^2$

$d^2$. a 10-chloro-pyridobenzothiazine is oxidized to the corresponding sulfoxide or sulfon, and the 10-chloro atom is substituted with an amine $NHQ^1Q^2$, and then the sulfoxide or sulfon is reduced to the sulfide, where $Q^1$ and $Q^2$ are as defined in claim 18.

20. A pharmaceutical composition containing at least one therapeutically active pyridobenzoxazine of general formula V,

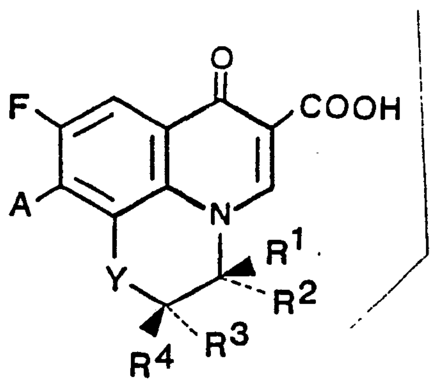

V

where A, $R^1$, $R^2$, $R^3$ or $R^4$ are as defined in claims 15 and 18 and the compound being in a stereochemically pure form, and its pharmaceutically acceptable hydrates, salts or esters, characterised in that this compound is prepared according to any one of claims 15-19.

21. A pharmaceutical composition containing an active substance and one or more adjuvants or diluents, characterised in that the active substance is a therapeutically active pyridobenzothiazine of general formula XVIII,

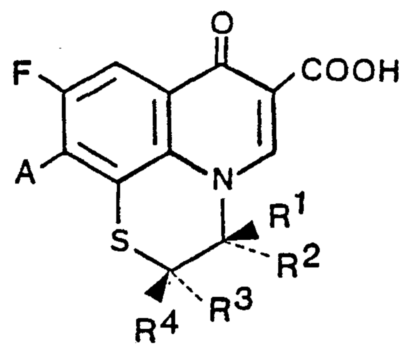

XVIII

as defined in claim 19 and its pharmaceutically acceptable hydrates, salts or esters.

22. A pharmaceutical composition comprising (S)-ofloxacin in stereochemically pure form and one or more adjuvants or diluents, characterised in that (S)-ofloxacin is prepared according to claim 15, 16 or 17.

23. A pharmaceutical antibacterial composition containing one or more adjuvants or diluents and (S)-9-fluoro-3-methyl-10-(4-methyl-1-piperazinyl)-2,3-dihydro-7-oxo-7H-pyrido[1,2,3-de][1,4]benzothiazine-6-carboxylic acid or a pharmaceutically acceptable hydrate, salt or ester.